# EUROPEAN PATENT SPECIFICATION

(11) **EP 4 137 794 B1**
(45) Date of publication and mention of the grant of the patent: **19.11.2025**
(21) Application number: 22191279.3
(22) Date of filing: 19.08.2022
(51) Int. Cl.: C09B 23/04, C09B 23/02, G01N 1/30, C09B 67/00, C09B 67/44

(54) **CYANINE DYES AND THEIR USAGE FOR IN VIVO STAINING OF MICROORGANISMS AND OTHER LIVING CELLS**
CYANINFARBSTOFFE UND DEREN VERWENDUNG ZUR IN-VIVO-FÄRBUNG VON MIKROORGANISMEN UND ANDEREN LEBENDEN ZELLEN
COLORANTS DE CYANINE ET LEUR UTILISATION POUR LA COLORATION IN VIVO DE MICRO-ORGANISMES ET D'AUTRES CELLULES VIVANTES

(30) Priority: 20.08.2021 EP 21192451
(43) Date of publication of application: 22.02.2023
(73) Proprietor: Philipps-Universität Marburg, 35037 Marburg (DE)
(72) Inventor: Vazquez, Olalla, 35037 Marburg (DE); Schulte, Leon, 35037 Marburg (DE); Linden, Greta, 80807 München (DE); Plocher, Benedikt, 61118 Bad Vilbel (DE)
(74) Representative: Stumpf, Peter

(56) References cited:
- EP-A1- 3 572 468
- LEON N SCHULTE ET AL: "A Far-Red Fluorescent DNA Binder for Interaction Studies of Live Multidrug-Resistant Pathogens and Host Cells", ANGEWANDTE CHEMIE, WILEY - V C H VERLAG GMBH & CO. KGAA, DE, vol. 130, no. 36, 6 August 2018 (2018-08-06), pages 11738 - 11742, XP071374875, ISSN: 0044-8249, DOI: 10.1002/ANGE.201804090

## Description

The invention relates to cyanine dyes, the production of cyanine dyes and their use for in vivo-staining of microorganisms, especially pathogens, and other living cells.

### Field of the invention

Several important microorganisms are still difficult to modify for GFP expression for the purpose of investigating them. This is particularly true for the growing number of multi-drug resistant bacteria, posing an emerging threat to patients in clinics worldwide. Specifically, these strains may acquire resistances to the antibiotics used to select transformants carrying the GFP transgene. Thus, the investigation of host cell interplay with drug-resistant bacteria by means of fluorescent labelling using GFP is not applicable generally. The expression "in vivo"-staining" as is used herein has the meaning of staining living cells without major negative interference on their viability so that their live behaviour, e.g. their interactions with other cells may be observed by methods known to the person skilled in the art after being stained, e.g. via flow cytometry or fluorescence microscopy etc. The surprising effect that the in vivo-staining is not negatively interferring with the viability of the stained cells is experimentally proven by comparative growth studies of stained cells at distinct dye-concentration in comparison with reference compounds, e.g. DMSO (preferred method with respect to microbial cells) and/or by application of resazurin-based assay to the cells several hours after being stained (preferred method for non-microbial cells). These comparative viability studies resp. resazurin-based assays are performed either with the cells one wants to investigate by use of in vivo staining or with HeLa cells or other standard cell lines known to the person skilled in the art (or, for example, *E. Coli* as standard microbial model organism with respect to microbial cells).

### Background of the technology

In vivo staining of cells with cyanine dyes as is defined herein (especially multiple-colour staining of one or more samples of cells), exerting the surprising effect that the staining with cyanine dyes does not negatively influence the viability of the stained cells, is unknown heretofore. Interference of staining with the viability of the stained cells is mostly not addressed at all, as can be exemplarily seen within the documents referenced below.

US 2016/340717 A1 reveals a dye resp. probe for detecting bacterial or viral endonucleases which is composed of an oligonucleotide, a fluorophore connected to the oligonucleotide and a fluorescence quencher, also being connected to the oligonucleotide. Fluorescence is emitted only upon cleavage of the dye/probe. US 2016/340717 A1 nowhere reveals that it is intended to keep cells living after being stained by the dye/probe. So, US 2016/340717 A1 is not addressing the problem to be solved by the invention revealed herein.

Jieqiong Qiu et al reveal combination probes with intercalating anchors and proximal fluorophores for DNA and RNA detection (cf. Nucleic Acis Research, 2016, Vol. 44, No. 17 - doi: 10.1093/narlgkw579)*.* This article describes the usage of an anchor-dye and a reporter dye, i. e. two dyes intercalating into DNA/RNA for achieving the desired staining. Nowhere is revealed that the dyes applied do not negatively interfere with the viability of the stained cells. The technical teaching of this document is completely different from the invention revealed herein below.

US 2011/0262904 A1 describes dyes having a good solubility in water and a good live cell permeability. But nowhere within US 2011/0262904 A1 it is stated or claimed that the dyes do not negatively interfere with the viability of the stained cells. Staining cells without negatively affecting their viability is not addressed at all in US 2011/0262904 A1, indicating that this problem is not concerned about. In US 2011/0262904 A1 it is only important that the dyes can permeate the membranes of live cells for staining, but the destiny of the cells, their viability upon being stained is of no importance. The technical teaching of this document is also completely different from the invention revealed herein below.

Similarly, WO 2013/189043 A1 and US 2015/0185182 A1 only describe live cells being stained, but the effect of the staining upon viability of the stained cells is not addressed at all. The technical teaching of this document is also completely different from the invention revealed herein below.

US 2010/0151509 A1 is revealing staining and counting leucocytes, for which purpose it is not necessary that stained cells stay alive after having been stained. Consequently interference of staining with the viability of the stained cells (leucocytes) is not addressed at all in US 2010/0151509 A1. Leucocytes by nature cannot multiply (grow) like other cells or microorganisms, so that this document is dealing with a totally different subject for which the parameter "viability" is not applicable at all. Thus, the technical teaching of this document is also completely different from the invention revealed herein below.

Tinghan Zhao et al. describe a very special dye being composed of a tetramethylrhodamin moiety being chemically bound to a specific MPP (mitochondria penetrating protein), rFrFrF.

Rhodamine dyes are known mitochondria markers by themselves, e.g. tetramethylrhodamine ethyl ester (TMRE), which is commercially available for the purpose of staining mitochondria in living cells. It is structural very close to TAMRA. The known applicability of TMRE for staining of living cells is misleading to the application of dyes of similar structure - like TAMRA. Also, TMRE is not interacting with the DNA and it is not a light-up dye, so background is always higher than with cyanine dyes. Thus, application of cyanine dyes for staining according to the invention will improve the background-to-signal ratio.

The article (cf. Bioconjugate Chem. 2019, 30, 2312 - 2316*)* provides only one specific dye-component (TAMRA), belonging to a class of dyes which is known to be usable for marking/staining mitochondria, and no hint at all that conjugates for staining mitochondria may be possible to be composed of cyanine dyes and MPP's. Cyanine dyes were unknown heretofore to be applicable for marking/staining mitochondria in live cells without negatively interfering with the viability of the stained cells.

Patent application EP 3 572 468 A1 reveals cyanine dyes binding to DNA and exerting fluorescence upon excitation with light of suitable wavelength, e.g. cyanine dye 3, exerting red fluorescence. Heretofore these compounds were only applied solely.

Now, it is found and revealed herein that it is possible to combine multiple DNA binding cyanine dyes exerting different fluorescence colours within one experiment without negatively interfering with the viability of the stained cells, thus providing the possibility of multichannel observation (red, green and yellow fluorescence) of interactions of different cells with each other. Also, new cyanine dyes of different structure, also binding to DNA, have been found, synthesized and investigated, which are also exerting red, yellow resp. green fluorescence upon excitation with light of suitable wavelength, e.g. cyanine dyes **1,** exerting green fluorescence, **2,** exerting yellow fluorescence, **15,** exerting green fluorescence, **16,** exerting yellow fluorescence, **17,** exerting red fluorescence, **18,** exerting green fluorescence, **19,** exerting yellow fluorescence and **20,** exerting red fluorescence.

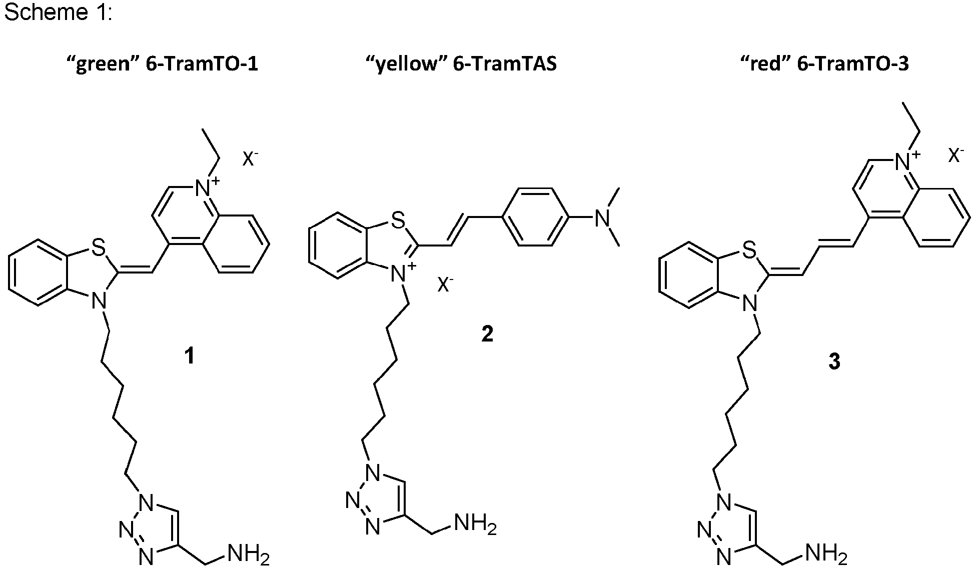

### Content of the invention

An objective of the present invention is to provide a method for staining living cells in such a way that different sample populations of cells are stained with different (at least two) cyanine dyes (most preferred cyanine dyes which are binding to nucleic acid) which are not reducing the viability of the stained cells, so that interaction of the stained population of cells can be observed via multichannel fluorescence microscopy.

A second objective of the present invention is to provide substances (cyanine dyes) that can be used for in vivo-staining of cells, especially microorganisms, without significantly reducing the viability of the stained cells (e. g. microbes, yeasts) according to the inventive method for staining living cells. The term "microorganism" (and all of its grammatical forms) as used with regard to the present invention comprehends not only all types of bacteria, fungi etc., but also pathogenic structures of all types. The terms "microorganism", "pathogen", "bacteria" etc. are therefore synonymously used throughout this whole revelation of the current invention. The term "cell" (and all of its grammatical forms) as used with regard to the present invention comprehends not only cells of microorganisms and its synonyms but also cells of vertebrates and plants.

In order to achieve the first objection a method for staining living cells is revealed herein applying at least two nucleic acid binding cyanine dyes with different fluorescence emission wavelengths which are not reducing the viability of the stained cells substantially.

A cyanine dye (most preferredly a cyanine dye which is binding to nucleic acid) is not reducing the viability of cells substantially according to the invention if: a) microbial (e. g. E. Coli) growth after 10h under identical breeding conditions at dye concentration of 2.5 µM is at least 90% compared to DMSO-reference and at dye concentration of 5 µM is at least 70% compared to DMSO-reference and/or b) growth of HeLa cells under identical breeding conditions at dye concentration of 10 µM is at least 50% of cell viability compared to reference (for example: concentration of 10 µM TriTO-1 leads to viability of about 62%, concentration of 10 µM 6-TramTO-1 **(1)** leads to viability of about 97%, concentration of 10 µM 6-TramTO-3 (3) leads to viability of about 97%).

It is well known to the person of ordinary skill in the art how to perform experimental measurements of microbial growth or cell viability as are described in the proceeding paragraph, for example regarding choosing the suitable microorganism or cell type, breeding conditions (growth medium, breeding temperature, surrounding atmosphere etc.), choice and applied amount of reference compounds if necessary (e. g. DMSO, MeCN), defining further reference conditions etc.

In order to achieve the second objective, the present invention provides new types of cyanine dyes of formula (I), 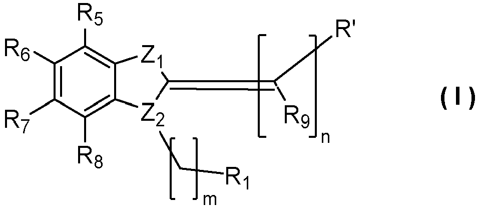 allowing in vivo staining of pathogens without negatively interfering with the viability of the pathogens.

The above objectives are achieved through the independent claims 1, 4 and 9 through 12. The dependent claims reveal additional advantageous embodiments of the invention.

The cyanine dyes from previous research exhibited mainly red fluorescence, enabling them to be detected in the far-red fluorescence channels of fluorescence microscopes or other fluorescence detection apparatuses only. The work revealed herein has led to new compounds (e. g. cyanine dyes **1, 2)** exerting excellent characteristics for live-staining (in vivo-staining) of cells, i.e. the new compounds exert distinctively less interference with the viability of cells upon staining in that they can also be used for staining cells of vertebrates, e.g. HeLa cells. The new compounds exert red, green and yellow fluorescence, thus providing the possibility of staining live cells with several cyanine dyes exerting different fluorescence colours at the same time and thus allowing a multichannel fluorescence observation of the behaviour of, for example, living microbial cells, to a much greater extent than previously known. Alternatively different species of microbial cells may be stained with different cyanine dyes, exerting different fluorescence, and being simultaneously observed within the same sample at the same time. This process of staining cells, basically without reducing the viability of the cells, is herein referred to as "in vivo staining".

Scheme 2 shows cyanine dyes according to the state-of-the-art exerting green, yellow and red fluorescence upon excitation with light of suitable wavelength, which are used as control samples within the experiments. Scheme 3 shows further examples of cyanine dyes according to the state of the art, being modified regarding substituent **X,** whereat general formula **Ia** represents examples of red fluorescent cyanine dyes and general formula **IIa** represents examples of green fluorescent cyanine dyes.

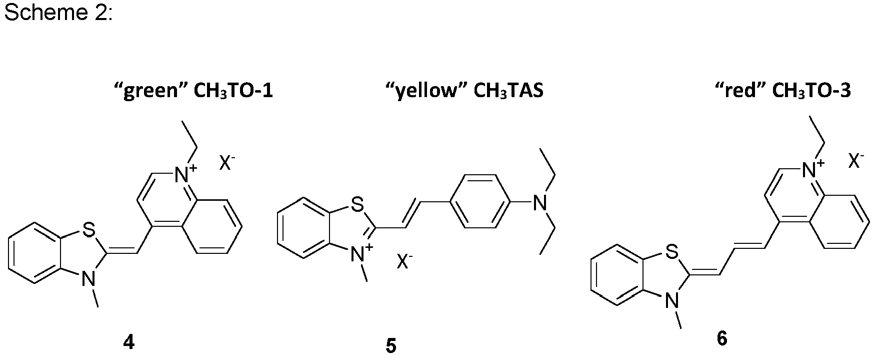

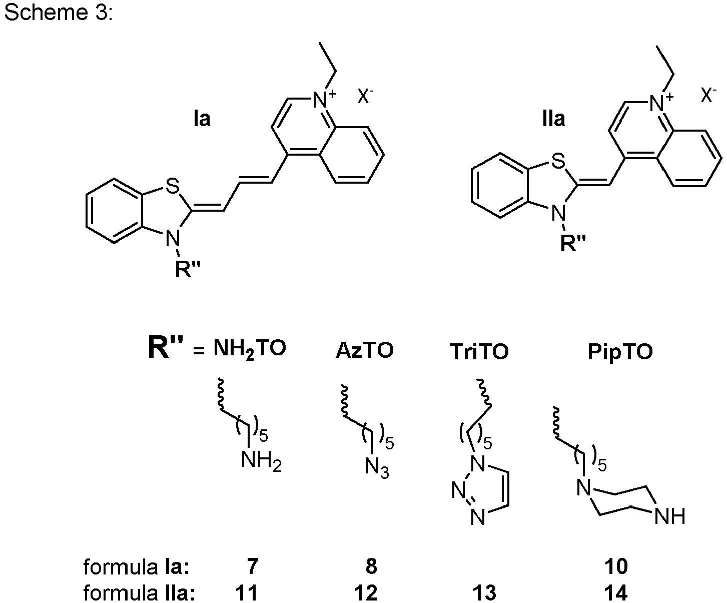

The scope of the invention is comprising compounds where substituent **R"** is (for example): 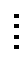 -(CH₂)₅-PPh₃ (e. g. **15** (green fluorescence), **16** (yellow fluorescence), **17** (red fluorescence)). The substituent -PPh₃ at the end of the side chain attached to the N-atom of the thiazole ring is introducing the possibility of staining organelles, especially e.g. mitochondria. This effect was heretofore unknown to be working in combination with cyanine dyes, thus this development, revealed herein, provides the possibility of staining organelles with cyanine dyes. Moreover, the scope of the invention even expands to cyanine dyes having peptide side chains as are exemplarily shown in Scheme 4 (substituent R₁ according to formula I being for example MPP i. e. mitochondria penetrating peptide, e.g. the peptide H₂N-Cys(R_{b})-Cha-dArg-Cha-dArg-COOH (SEQ ID 1)), whereat the substituent R_{b} is denoting the chemical bonding site to the core structure of the cyanine dyes, i. e. here the MPP is exemplarily connected via the thioether-bridge from cysteine to the core structure of the cyanine dye (cf. compounds **18, 19** and **20**)). Other examples of MPP's being comprised by the scope of the invention are: H₂N-Cha-dArg-Cha-dArg-COOH (SEQ ID 2), H₂N-dArg-Cha-dArg-Cha-dArg-Cha-COOH (SEQ ID 3), H₂N-dArg-Phe-dArg-Phe-dArg-Phe-COOH (SEQ ID 4). In order to connect them to the core of the cyanine dye according to the invention, first cysteine is attached via SPPS (solid phase peptide synthesis) accordingly and then the cystein-modified MPP connected to the prepared cyanine dye core via substitution reaction, as is known to the person skilled in the art. As can be seen exemplarily from scheme 4, any MPP to be applied may be acetylated at the N-terminus, whereat the examples are not meant to be confining the scope of the invention to the use of acetylated or otherwise at the N-terminus substituted MPP's.

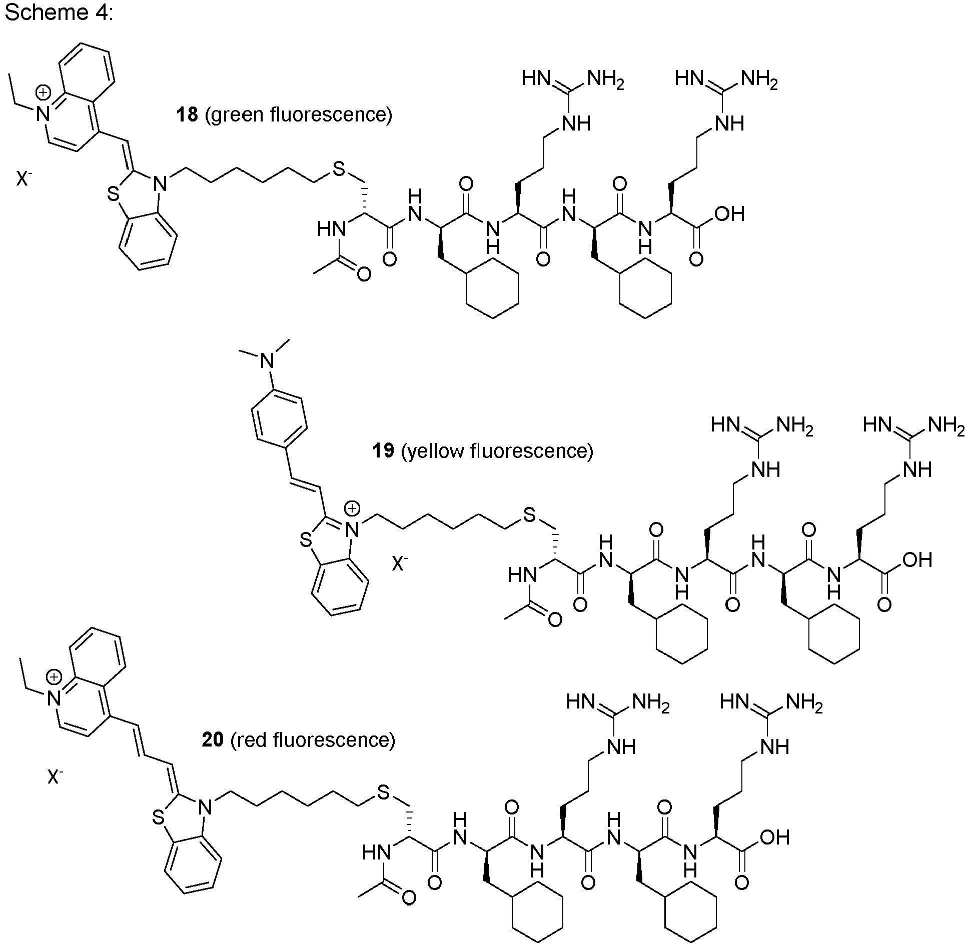

Following table 1 shows some of the molecular extinction coefficients that are exemplarily measured.

**Table 1: Molar extinction coefficients**

| **Compound** | **solvent** | **Molar extinction coefficient** |
|---|---|---|
| 6-TramTO-3 **(3)** | H₂O | 59403 M⁻¹cm⁻¹ |
| CH₃TO-3 **(6)** | DMSO | 37153 M⁻¹cm⁻¹ |
| 6-TramTAS **(2)** | MeCN | 70463 M⁻¹cm⁻¹ |
| CH₃TAS **(5)** | MeCN | lit. 82200 M⁻¹cm⁻¹ |
| 6-TramTO-1 **(1)** | H₂O | 61813 M⁻¹cm⁻¹ |
| CH₃TO-1 **(4)** | DMSO | lit. 63000 M⁻¹cm⁻¹ |

Therefore, the subject matter of the invention may be summarised as follows.

The subject matter of the invention is a method for staining living cells characterized in that the method comprises the following steps:
i) at least one cyanine dye is applied to at least one sample of cells by adding a solution of the at least one cyanine dye to the at least one sample of cells, and
ii) incubating the at least one sample of cells according to step i) for at least 15 minutes, so that stained living cells are existent. The method of the present invention is defined in claim 6.

The cells to be stained for investigation (= "sample of cells") and those for determining the non-interference of viability (= "eukariotic cells, for example HeLa cells") may be of the same type or of different type.

Also disclosed is a method for staining living cells as described before, characterized in that the method comprises the following steps i) and ii) and the additional step iii):
i) at least two cyanine dyes are applied to at least two separate
   samples of cells whereat each sample is stained with one of the at least two cyanine dyes by adding a solution of the cyanine dye to the sample of cells, whereat
      - each cyanine dye exerts a fluorescence emission wavelength different from the fluorescence emission wavelengths of all other cyanine dyes being applied and
      - each cyanine dye is not reducing the viability of the stained cells substantially, whereat the non-reduction of the viability of the living cells through the cyanine dye is defined for each cyanine dye by
         - comparable microbial growth measurement against DMSO under identical breeding conditions whereat the cell-count in the sample with the cyanine dye after 10 h breeding under identical conditions at dye concentration of 2.5 µM is at least 94% compared to DMSO-reference and/or at dye concentration of 5 µM is at least 80% compared to DMSO-reference and/or
         - comparable viability measurements of eukariotic cells, for example HeLa cells, under identical breeding
            conditions at dye concentration of 5 µM is at least 85% compared to reference, whereat the reference is either viability of the eukariotic cells, for example HeLa cells, in
            presence of acetonitrile or viability of the pure cells (eukariotic cells, for example HeLa cells) or viability of the cells (eukariotic cells, for example HeLa cells), grown in water,
   thus providing at least two separate samples of cells, each one being stained with at least one cyanine dye;
ii) each sample of cells according to modified step i) is incubated for at least 15 minutes, so that stained living cells are existent;
iii) the at least two separate samples of cells provided according to step ii),
   each one being stained with at least one cyanine dye, are combined within one sample vessel with each other thus forming one combined sample.

The cells to be stained for investigation (= "samples of cells") and those for determining the non-interference of viability (= "eukariotic cells, for example HeLa cells") may be of the same type or of different type.

In general the cyanine dyes of the invention bind to nucleic acid.

The subject matter of the invention also comprises any one method as described before, characterized in that the method comprises the following additional preparation step before step i):
a) obtaining and isolation of at least one sample of cells to be introduced into step i),
whereat this obtaining and isolation-step may include one or more steps for concentrating and/or breeding of the cells.

The subject matter of the invention also comprises a cyanine dye for usage in any one method as is described before, the chemical structure of the cyanine dye being according to general formula (I), 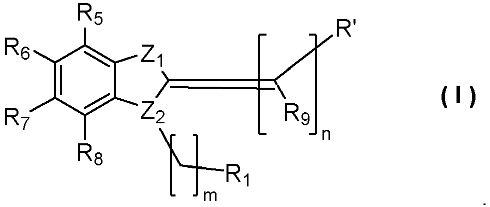 wherein
- R' is a moiety according to formula **(II)** or a moiety according to formula **(III),** 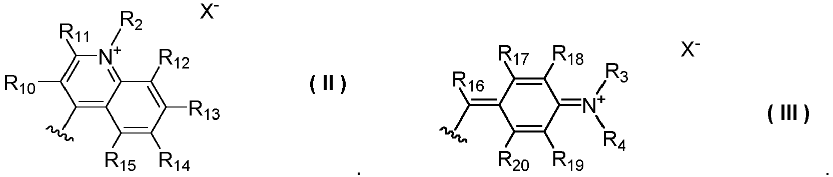
- R₂ is selected from the list comprising H, methyl, ethyl, propyl, iso-propyl,
   butyl, iso-butyl, tert-butyl, alkyl, alkinyl, alkylidene insofar as substituent R₂ is present according to the choice of R',
- R₃ and R₄ are independently from each other selected from the list comprising H, methyl, ethyl, propyl, iso-propyl, butyl, iso-butyl, tert-butyl, alkyl, alkinyl, alkylidene insofar as substituents R₃, R₄ are present according to the choice of R',
- R₅ to R₂₀ are independently from each other selected from the list comprising the substituents H, halogen, methyl, ethyl, propyl, isopropyl, methoxy, ethoxy, propoxy, amino, methylamino, ethylamino, monoalkylamino, dialkylamino, a nitro group, a sulfo group insofar as any one of substituents R₅ to R₂₀ is present according to the choice of R',
- Z₁ and Z₂ are each independently O, S, or N,
- X⁻ is selected from the list comprising halogenate, sulfate, triflate, trifluoro acetate, difluoro acetate or fluoro acetate,
- n is 1 to 5,
- m is 1 to 10,
characterized by
- R₁ is independently chosen from the list comprising the substituents triphenylphosphine, substituted triphenylphosphine, 1,2,3-triazol-1-yl, 1,2,3-triazol-2-yl, 1,2,3. triazol-4-(aminomethyl)-1-yl
   mitochondria penetrating peptide - whereat the mitochondria penetrating peptide is chemically bound to the alkyl-chain of length m via peptide-bond or via sulfide-bond or via ester-bond or via thioester-bond -
   if R' is chosen to be a moiety according to formula **(II);**
   or
- R₁ is independently chosen from the list comprising the substituents OH, SH, N₃, NH₂, NH₃⁺, NHR₃, NH₂R₃⁺, NR₂₁R₂₂, NR₂₁R₂₂H⁺ - whereat R₂₁ and R₂₂ are independently from each other chosen from the list comprising methyl, ethyl or propyl -, substituted or non-substituted heterocyclic structure, substituted or non-substituted cyclic structure, triphenylphosphine,
   substituted triphenylphosphine, 1,2,3-triazol-1-yl, 1,2,3-triazol-2-yl,
   substituted 1,2,3-triazol-1-yl, substituted 1,2,3-triazol-2-yl,
   mitochondria penetrating peptide - whereat the mitochondria penetrating peptide is chemically bound to the alkyl-chain of length m via peptide-bond or via sulfide-bond or via ester-bond or via thioester-bond - if R' is chosen to be a moiety according to formula **(III).**

The cyanine dye of the present invention is defined in claim 1.

Typically the cyanine dye of the invention is characterized in that the mitochondria penetrating peptide has the amino acid sequence H₂N-Cys-Cha-dArg-Cha-dArg-COOH according to SEQ ID 1 or the amino acid sequence according to SEQ ID 2 or the amino acid sequence according to SEQ ID 3 or the amino acid sequence according to SEQ ID 4, whereat each mitochondria penetrating peptide is acetylated at the N-terminus.

The subject matter of the invention also comprises a cyanine dye as is described before, characterized in that it has the chemical structure according to formula ( A ), 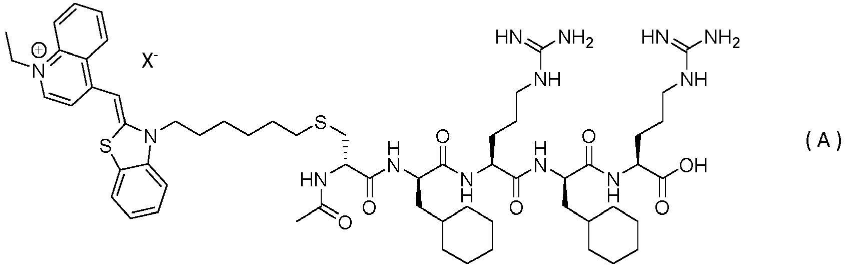 or according to formula ( B ) 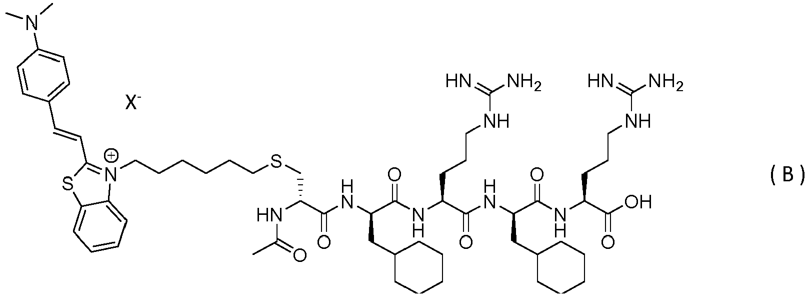 or according to formular ( C ),

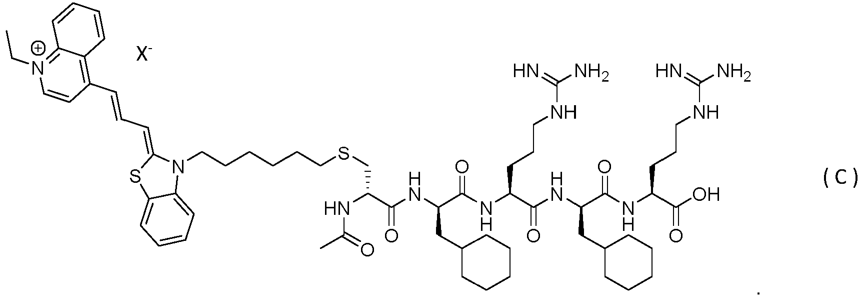

The subject matter of the invention also comprises any cyanine dye as is described before in its protonated or deprotonated form, whereat the counterion, resp. counterions, of the protonated or deprotonated form is, resp. are, independently selected from the list comprising halogenate, sulfate, triflate, trifluoro acetate, difluoro acetate or fluoro acetate.

The subject matter of the invention also comprises any cyanine dye as is described before, characterized in that it is for use in binding to oligomeric DNA or primer-DNA or DNA-aptameres.

The subject matter of the invention also comprises the usage of a cyanine dye as is described before for staining nucleic acid within a living cell.

The subject matter of the invention also comprises a kit for carrying out any one of the methods described above, the kit comprising the cyanine dye and optionally supportive substances.

The subject matter of the invention also comprises the synthesizing method of a cyanine dye as is described above, characterized in that the synthesizing method comprises the following steps:
a) performing a substitution reaction on a precursor compound according to formula **(IV),** 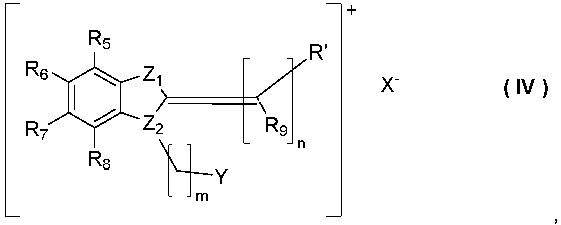 whereat
   the substituent Y in formula **(IV)** is selected from the list comprising the substituents chlorine, bromine, iodine, mesylate, tosylate, fluorinated mesylate, fluorinated tosylate and all other substituents in formula **(IV)** have the same meaning and are chosen in the same way as is denoted in claim 5 regarding formula **(I)** and indices m and n have the same values as are denoted in claim 5 regarding formula **(I),**
   by reacting the precursor compound according to formula **(IV)** in a reaction mixture with a nucleophilic compound, leading to the substitution of substituent Y by the nucleophilic compound;
b) extracting the reaction product from the reaction mixture from step a) and
c) purifying the reaction product retrieved from step b).

The subject matter of the invention also comprises a method as described before, characterized in that the nucleophilic compound in step a) is triphenylphosphine or 1,2,3-triazole or a mitochondria penetrating peptide.

The subject matter of the invention also comprises a method as described above, characterized in that the nucleophilic compound in step a) is the azide ion and the reaction product from step a) or the extracted reaction product from step b) or the purified reaction product from step c) is further reacted in an additional step d), the additional step d) being inserted between steps a) and b) or between steps b) and c) or being placed after step c).

The subject matter of the invention also comprises a method as described before, characterized in that the additional step d) comprises the reaction of a precursor compound according to formula **(V),** 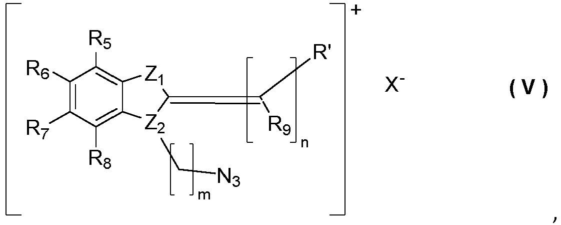
whereat in this case R' is restricted to a moiety according to formula **(III),**
with substituted or unsubstituted Tris((1-benzyl-4-triazolyl)methyl)amine, "TBTA", thus forming a cyanine dye according to any one of the
preceeding claims 5 through 9 having a substructure where R' is a moiety according to formula **(III)** and R₁ is a substituent with a substituted or non-substituted heterocyclic structure.

The subject matter of the invention also comprises a method as described above, characterized in that the additional step d) comprises the reaction of a precursor compound according to formula **(V),** 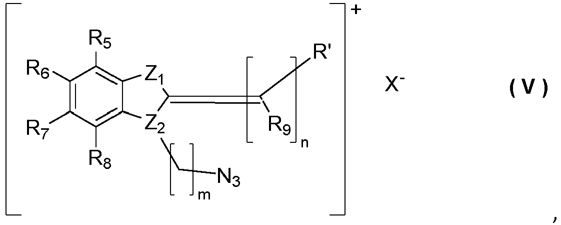
whereat R' is a moiety according to formula (II) or according to formula **(III),** whereat n is 1 or n is 2 or n is 3 or n is 4 or n is 5,
in a copper-catalyzed azide-alkyne cycloaddition reaction with a substituted or unsubstituted alkyne, thus forming a cyanine dye according to any one of the preceeding claims 5 through 9, whereat R₁ is a substituent with a substituted or non-substituted heterocyclic structure.

### Detailed embodiments of the invention

All experimental data revealed herein is provided by way of example only and is not to be understood as being confining the scope of the invention.

### Discussion of toxicity of some exemplary compounds

### Exemplary growth curves of some sample microorganisms, resp. sample cells exemplarily stained

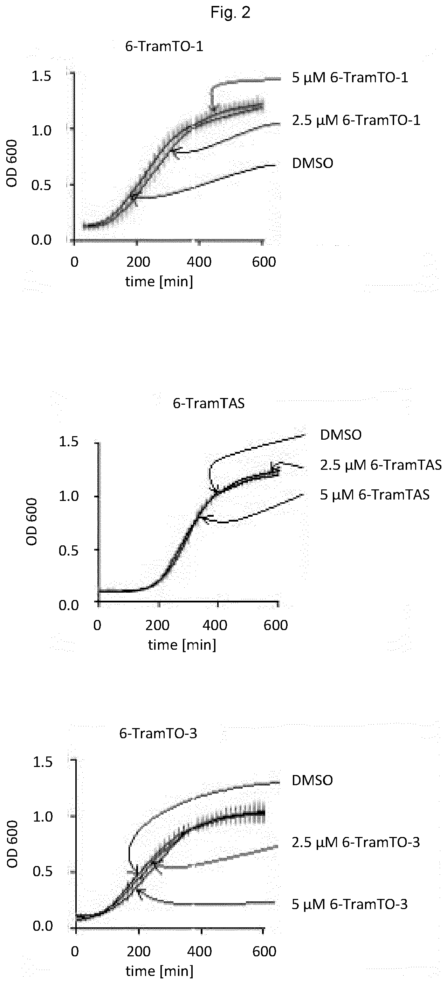

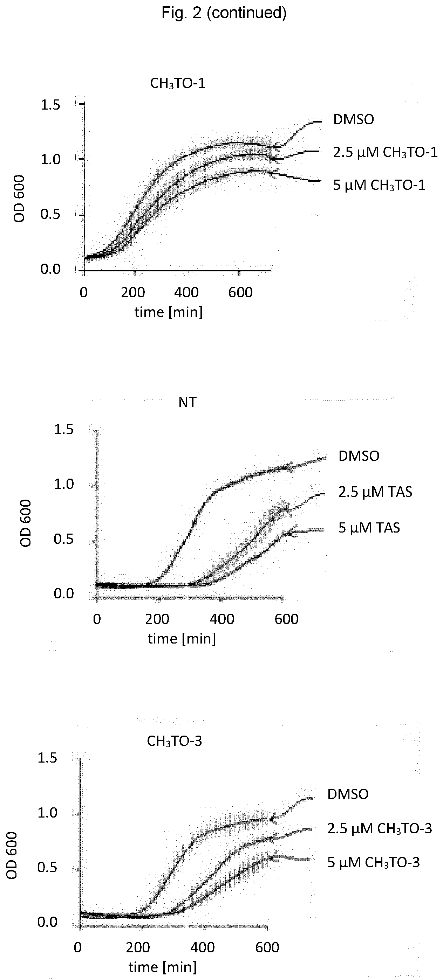

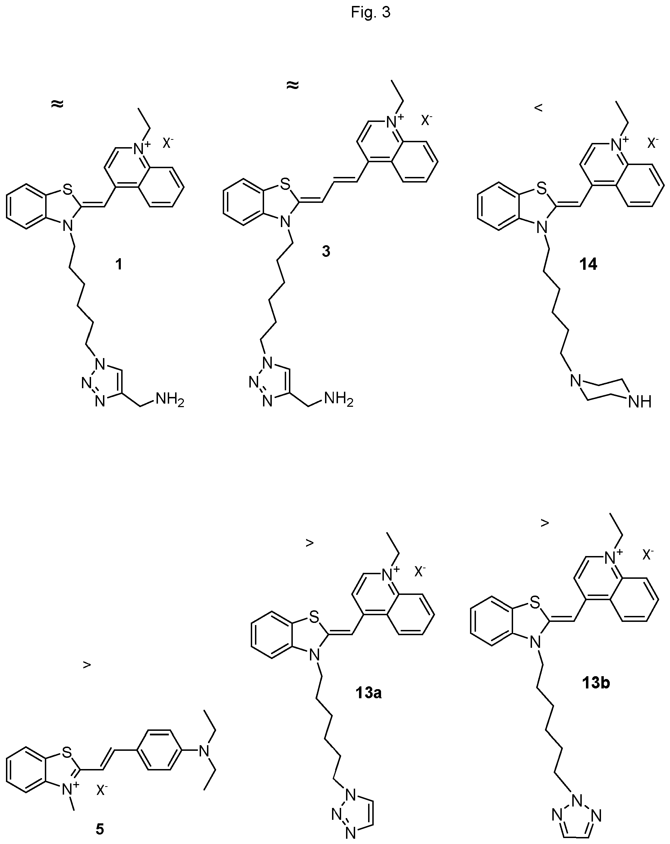

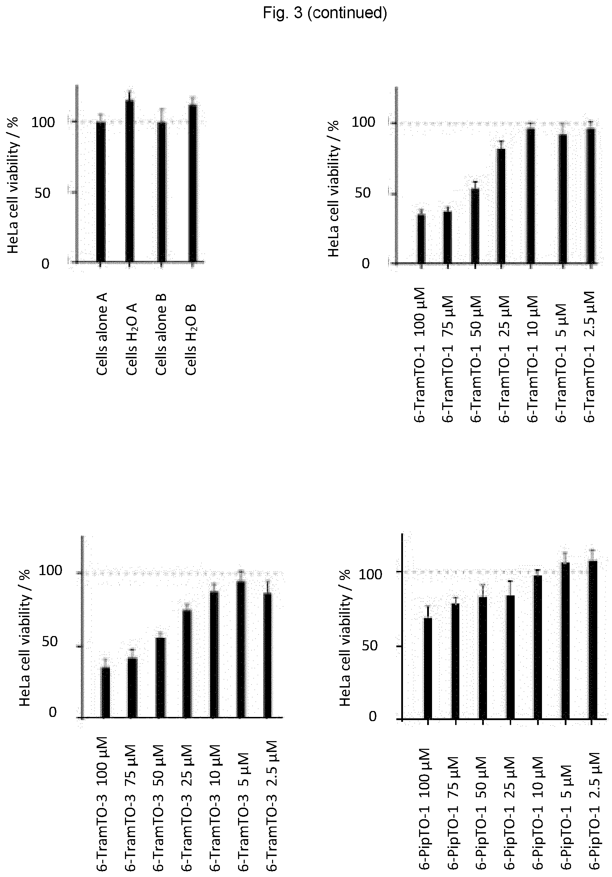

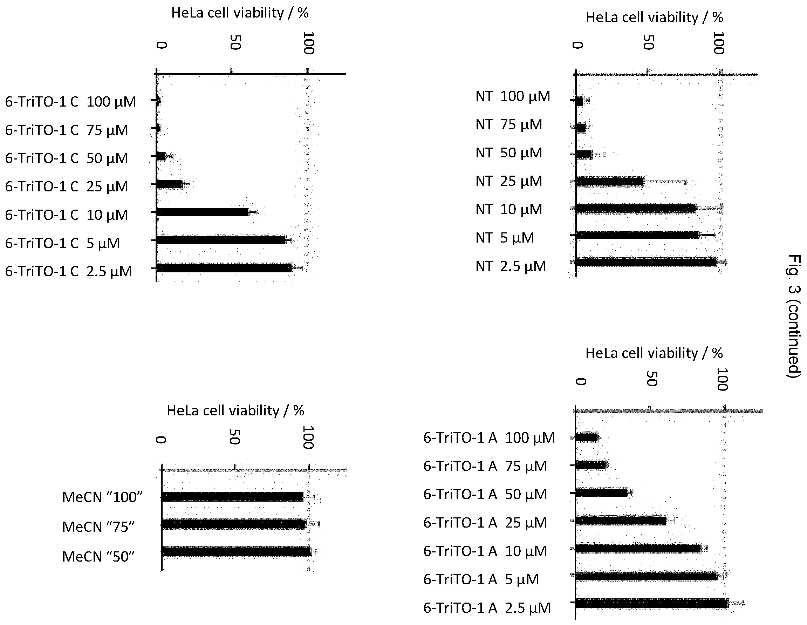

The triazole amine derivatives 6-TramTO-1 **(1),** 6-TramTAS **(2)** and 6-TramTO-3 (3) do not interfere with the growth of Escherichia coli (E. coli) NEB5α (New England Biolabs), while the previously described dyes, without the triazole amine side chain **(4-6)** do. Even at 5 µM concentration no toxicity is observed regarding compounds **1-3,** clearly proving that TramTOs and TramTAS (1-3) are non-toxic to microorganisms (cf. Fig. 2). Fig. 3 exemplarily shows the viability of HeLa cells upon staining with cyanine dyes. HeLa cells are eukaryotic cells, therefore these experiments show the widespread applicability of the invention.

### Experimental procedure for measuring of cell viability

HeLa cells are grown in a humidified cell-culture incubator (New Brunswick Scientific, USA) at 37 °C under CO₂ (g) (5%, v/v). Growth medium is DMEM nutrient medium supplemented with fetal bovine serum (FBS) (10%, v/v), penicillin (100 units/mL), and streptomycin (100 µg/mL). HeLa cells (2×10⁴) are seeded into black 96-well microtiter plates (Greiner Bio-One, Austria) in DMEM (200 µL, 2.5% FBS). For assessing the viability stocks of the compounds of interest in milliQ water are added at different concentrations and incubated for 24 h. Resazurin fluorescence-based cell viability assay is performed to evaluate cell viability.

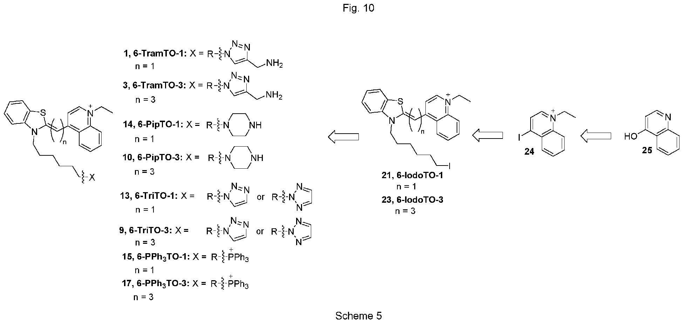

Measurements of cell viability is exemplarily performed with compounds 18, 19 and 20: HeLa cells are treated with different concentrations (10 µM, 7.5 µM, 5 µM, 2.5 µM, 1 µM and 0.5 µM) of the dyes (18, 19, 20) in triplicated. Viability is then examined after 21h incubation time by resazurin-based assay. The results of the tests are depicted in Figs. 10A and 10B.

### Experimental procedure for measuring bacterial growth curves

*E. coli* NEB5α are cultured in lysogeny broth (LB) liquid medium and incubated at 37°C, 200 rpm. In clear 96-well microtiter plates (Nunc, USA) in a final volume of 250 µL 5 µL of cell suspension at exponential growth phase were added and stocks of the compounds of interest in milliQ water, DMSO or MeCN (final vehicle concentration 0.8%, v/v). Bacterial growth is monitored at OD₆₀₀, utilizing a microplate reader (Tecan, Switzerland).

### Fluorescence titrations

The affinity of the TramTO derivatives towards dsDNA is usually higher than the affinity of the derivatives without the modified side chain according to this invention. Usually the TramTOs according to the invention were the stronger binders compared to the corresponding controls **(4-6)** with the similar fluorescence, however the green control CH₃TO-1 **(4)** is a stronger binder then the 6-TramTAS, the 6-TramTO-1 is superior to 6-TramTO-3 (about x2) and 6-TramTAS (about x3). *K*_{D} values are determined as is well known to any person being of ordinary skill in the art. Table 2 shows the results obtained.

**Table 2: Exemplary results of fluorescence titrations (affinity towards dsDNA)**

| | RFU -/+ DNA | Slits (nm) | *K*_{D} |
|---|---|---|---|
| 6-TramTO-1 **(1)** | 1.2/460 | 5/5 | 8.63 ± 4.34 µM |
| CH₃TO-1 **(4)** | 0.2/125 | 10/10 (for 5/5: ~10 RFU) | 20.56 ± 6.67 µM |
| 6-TramTAS **(2)** | 2.9/90 | 10/10 (for 5/5: ~8 RFU) | 30.09 ± 4.61 µM or 28.07 ± 2.33 µM |
| CH₃TAS **(5)** | 2.3/33 | 10/10 (for 5/5: ~2 RFU) | Not calculable |
| 6-TramTO-3 **(3)** | 11.8/280 | 5/5 | 22.96 ± 1.81 µM / 19.5±2.6 µM (literature value) |
| CH₃TO-3 **(6)** | 6.8/210 | 5/5 | 44.98 ± 1.05 µM |

### Flow Cytometry

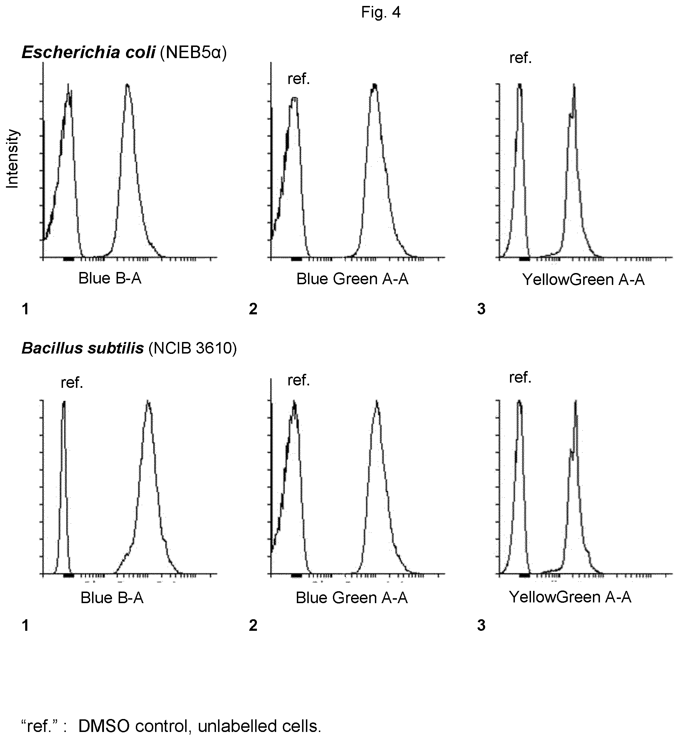

The flow cytometric measurements are performed by use of the LSR Fortessa cell analyser (BD Biosciences, USA) or other commercially available apparatuses (e.g. FACSAria III (BD Biosciences, USA) and Guava easyCyte (Merck Millipore, USA)). Any person of ordinary skill in the art knows how to adjust the different apparatuses commercially available in order to perform comparable experiments exerting the experimental parameters described herein. A minimum of 10000 events per sample is analysed. The measurements are each performed in at least two independent experiments. Bacteria are labelled for 15 min with 5 µM of the corresponding dyes. All dyes showed > 99.9% labelling. Fig. 4 shows graphs of the results, exemplarily obtained with *E. Coli* (NEB5α) and *B*. *Subtilis* (NCIB 3610).

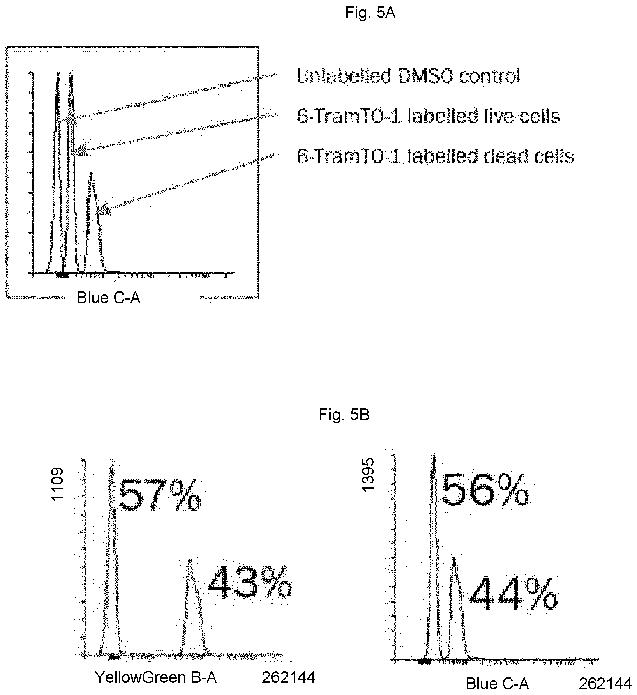

For 6-TramTO-1 **(1)** one can see clear labelling when 5 µM of the dye are added for only 5 min before the measurements, dead cells (treated with 70% isopropanol for 1h, then washed with saline 3x) being labelled about 10x stronger: MFI 135 vs 1337. Figs. 5A - 5C show additional data.

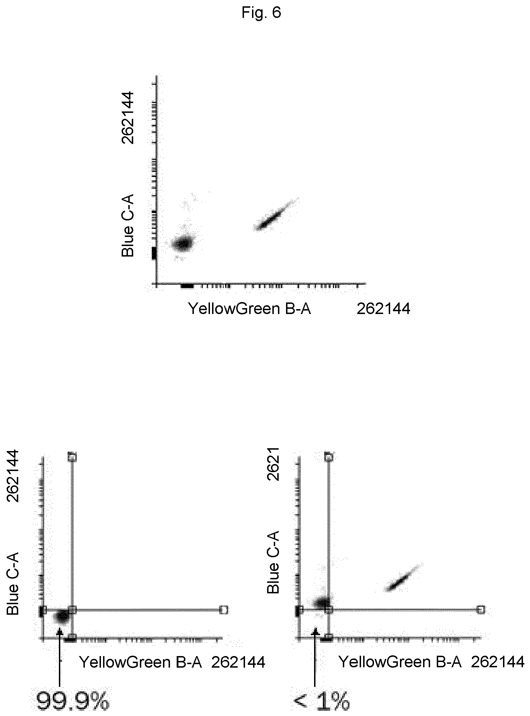

As can obviously be seen from the data shown in Fig. 6, co-staining with PI is not necessary upon usage of the inventive cyanine dyes in order to distinguish live from dead cells. The green (Blue C) channel gave the same results than the orange/ PI channel (YellowGreen B) in comparison with commercially available Kit BacLight^{™} of ThermoFisher.

### Dual and triple labelling

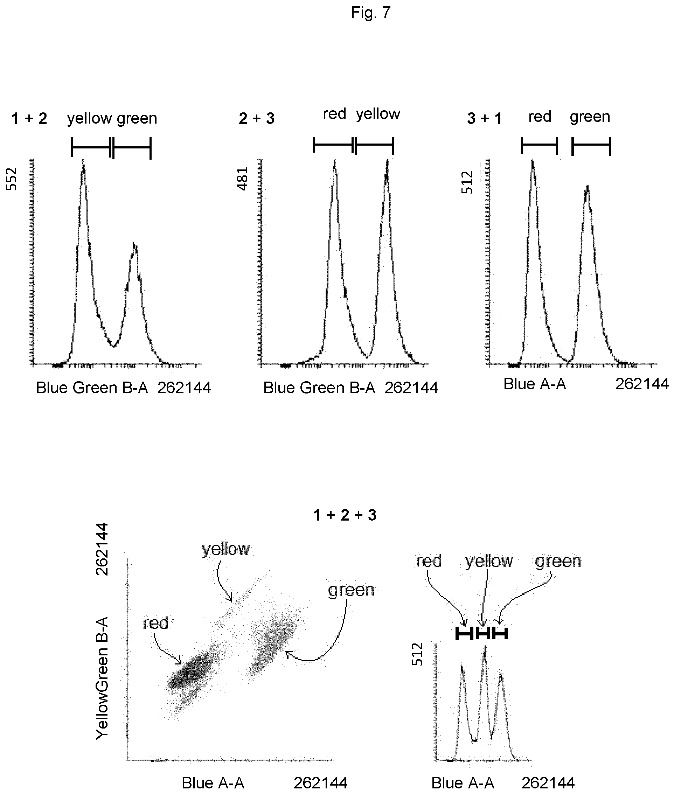

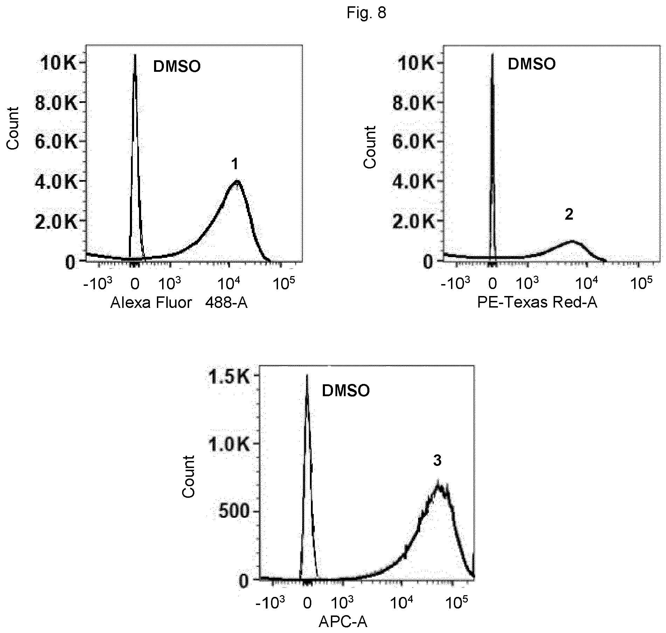

Experimental data shows that dual and even triple labelling is also possible with the TramTO dyes (examples presented in Fig. 7 using *Bacillus subtilis* and in Fig. 8 using *Klebsiella pneumoniae*)*.*

### Exemplary Phagocytosis studies with different bacterial strains exemplarily labelled with 1 or 3

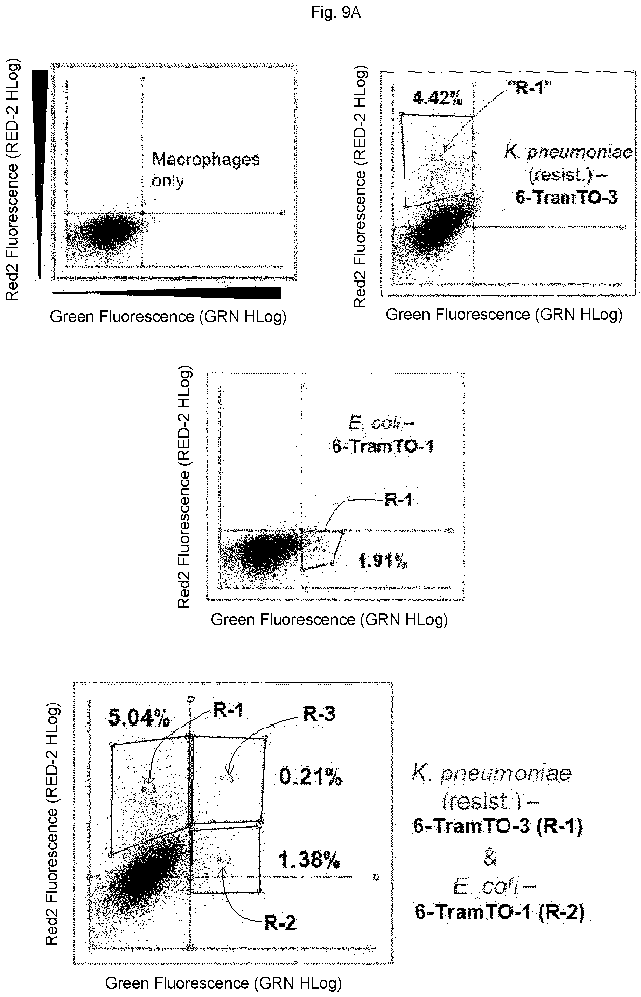

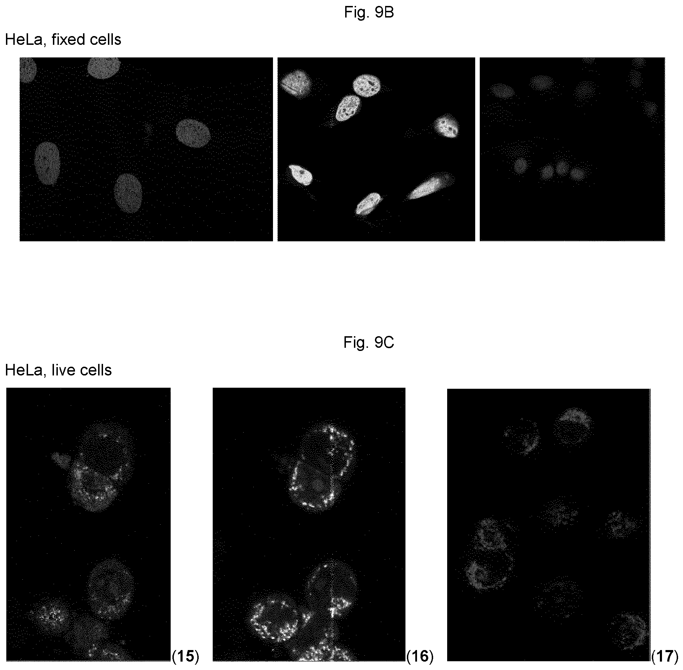

The applicability of the invention is proven by exemplary Phagocytosis studies as is described below (cf. Fig. 9A, Fig. 9B and Fig. 9C):
FACS data is recorded using a Guava easyCyte flow cytometer (Millipore). Bacteria are labelled for 15 min with 5 µM of the corresponding dye, washed 3 times with DPBS. After the last wash bacteria are brought to an OD₆₀₀ nm of 2.0 and 2.3 µL in such a way that an equivalent of bacteria are added to each well to achieve a multiplicity of infection (MOI) of 5 (MOI = 5 + 5 for experiments with two bacterial strains). Culture plates are then centrifuged for 10 min at 250 g and 37°C to establish physical contact between macrophages and bacteria. Subsequently, culture plates are transferred back into a 37°C incubator with CO₂ atmosphere for 50 min. After the stimulation period the cells are washed once with DPBS prior to flow cytometry. Macrophages are detached carefully from the culture dishes by using a rubber scraper.

### Nuclear Magnetic Resonance Spectroscopy (NMR)

All NMR spectra are automatically measured at 300 K either in a Bruker AV III HD 300 MHz at a frequency of 300 MHz (¹H) or 75 MHz (¹³C) or in a Bruker AV III HD 500 MHz at a frequency of 500 MHz (¹H) or 125 MHz (¹³C). All chemical shifts (δ) are relative to tetramethylsilane (TMS), i.e. δ(TMS) = 0 ppm. As internal standards, deuterated chloroform (CDCl₃), dimethyl sulfoxide (DMSO) with TMS are used. Solvent shifts (ppm): δ(CHCl₃) = 7.26 ppm (¹H), δ(CHCl₃) = 77.16 ppm (¹³C), *δ*(DMSO) = 2.50 ppm (¹H), *δ*(DMSO) = 39.52 ppm (¹³C). The assignment of each signal is based on two-dimensional nuclear magnetic resonance spectroscopy (2D NMR), i.e. heteronuclear single quantum coherence (HSQC), heteronuclear multiple bond correlation spectroscopy (HMBC) and correlation spectroscopy (COSY).

### Mass Spectrometry

Electrospray ionization mass spectrometry (ESI-MS) is performed on a Finnigan LTQ-FT (Thermo Fischer Scientific). EI-MS is performed on an AccuTOF GCv (JEOL).

### Analytic HPLC

Characterization is performed via analytical RP-HPLC-MS on an Agilent 1260 Infinity II HPLC-System (Agilent Technologies). Agilent eclipse XDB-C18 column (5 µm, 4.6 × 150 mm) using isocratic regime during the first 5 min, followed by a linear gradient over 30 min of solvent B as stated below with a flow rate of 0.2 mL/min at 55 °C. The detection was carried out by monitoring the absorbance at 220 nm. The eluents are ultrapure H₂O (solvent A) with addition of 0.05% TFA and MeCN (solvent B) with addition of 0.03% TFA.
Gradient 1: 5% to 75% of solvent B
Gradient 2: 5% to 95% of solvent B

### Synthetic Procedures

Scheme 5 (Fig. 10) shows the general retrosynthetic pathway of the production of the cyanine dyes according to the invention.

### Synthesis of 6-TramTO-1

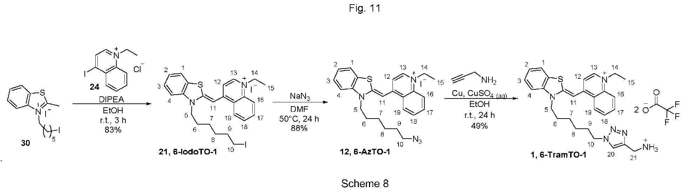

This synthesis is based on the scaffold of thiazole orange (TO), known within the state of the art, but proceeding beyond the state of the art (cf. Fig. 11, scheme 8).

**(Z)-1-ethyl-4-((3-(6-iodohexyl)benzo[d]thiazol-2(3H)-ylidene)methyl)quinolin-1-ium (6-lodoTO-1, 21):** Benzothiazolium **(30,** 521 mg, 1.07 mmol) and quinolinium **(24,** 342 mg, 1.07 mmol) are suspended in EtOH (HPLC-grade, 16.0 mL) and DIPEA (410 µL, 2.35 mmol) added. The solution is stirred in the dark at r.t. for 1 h, the solvent removed and the crude purified by flash column chromatography (allox, MeOH in CH₂Cl₂: 0% → 0.2% → 0.5%→ 0.7%) to yield the desired product **(21, 359** mg, 0.56 mmol, 52%) as red solid. **R*_{f}* =** 0.45 (CH₂Cl₂/MeOH 10:1). **¹H-NMR** (500 MHz, DMSO-*d₆*, δ): 8.75 (d, 1H, *J* = 8.7 Hz, H-16), 8.67 (d, 1H, *J* = 7.3 Hz, H-13), 8.16 (d, 1H, *J* = 8.6 Hz, H-19), 8.05 (d, 1H, *J* = 7.2 Hz, H-4), 8.03 - 7.99 (m, 1H, H-17), 7.81 - 7.76 (m, 2H, H-1, H-18), 7.63 - 7.59 (m, 1H, H-2), 7.44 - 7.39 (m, 2H, H-3, H-12), 6.93 (s, 1H, H-11), 4.70 - 4.60 (m, 4H, H-14, H-5), 3.24 (t, 2H, *J* = 6.9 Hz, H-10), 1.83 - 1.76 (m, 2H, H-9), 1.76 - 1.70 (m, 2H, H-6), 1.50 - 1.45 (m, 5H, H-8, H-15), 1.45 - 1.39 (m, 2H, H-7). **¹³C-NMR** (125 MHz, DMSO-*d₆*, δ): 159.4 (C_{q}), 148.7 (C_{q}), 144.0 (CH), 140.0 (C_{q}), 136.9 (C_{q}), 133.3 (CH), 128.3 (CH), 127.0 (CH), 125.7 (CH), 124.6 (CH), 124.3 (C_{q}), 123.9 (C_{q}), 123.0 (CH), 118.1 (CH), 113.0 (CH), 108.3 (CH), 87.7 (CH), 49.5 (CH₂), 45.6 (CH₂), 32.7 (CH₂), 29.6 (CH₂), 26.7 (CH₂), 24.9 (CH₂), 14.8 (CH₃), 8.8 (CH₂). **HRMS-ESI⁺** (*m*/*z*)*:* [M]⁺ calcd for C₂₅H₂₈IN₂S⁺, 515.1012; found, 515.1023.

**(Z)-1-ethyl-4-((3-(6-iodohexyl)benzo[d]thiazol-2(3H)-ylidene)methyl)quinolin-1-ium (6-AzTO-1, 12):** lodo derivative **6-lodoTO-1 (5, 13.1** mg, 23.8 µmol) and NaN₃ (2.00 mg, 30.9 µmol) are dissolved in DMF (500 µL) and stirred for 25 h at 50 °C in the dark. The solvent is removed and the crude purified by flash column chromatography (allox basic, MeOH in CH₂Cl₂: 0% → 2.0%) to yield the desired product **(12,** 10.8 mg, 19.9 µmol, 84%) as red solid. ***R_{f}*** = 0.43 (CH₂Cl₂/MeOH 10:1). **¹H-NMR** (500 MHz, DMSO-*d₆*, δ): 8.76 (d, 1H, ³*J* = 8.4 Hz, H-9), 8.67 (d, 1H, ³*J* = 7.2 Hz, H-10), 8.17 (d, 1H, ³*J* = 8.6 Hz, H-6), 8.06 (dd, 1H, ³*J* = 7.9 Hz, ⁴*J* = 1.0 Hz, H-4), 8.03- 7.99 (m, 1H, H-8), 7.81- 7.76 (m, 2H, H-1, H-7), 7.62 (td, 1H, ³*J* = 7.8 Hz, ⁴*J* = 1.1 Hz, H-2), 7.45- 7.40 (m, 2H, H-3, H-11), 6.94 (s, 1H, H-5), 4.69- 4.62 (m, 4H, H-12, H-14), 3.32- 3.27 (m, 2H, H-19), 1.84- 1.77 (m, 2H, H-15), 1.54- 1.44 (m, 9H, H-13, H-16, H-17, H-18) **ppm.¹³C-NMR** (75 MHz, DMSO-*d₆*, *δ*): 159.4 (C_{q}), 148.7 (C_{q}), 144.1 (C-10), 140.0 (C_{q}), 136.9 (C_{q}), 133.4 (C-8), 128.3 (C-2), 126.9 (C-7), 125.8 (C-9), 124.6 (C-3), 124.3 (C_{q}), 123.9 (C_{q}), 123.0 (C-4), 118.1 (C-6), 113.1 (C-1), 108.3 (C-11), 87.7 (C-5), 50.5 (C-19), 49.5 (C-12), 45.6 (C-14), 28.1 (C-18), 26.8 (C-15), 25.9 (C-17), 25.5 (C-16), 14.8 (C-13) ppm. **HRMS-ESI⁺** (*m*/*z*): [M]⁺ calcd for C₂₅H₂₈N₅S⁺, 430.2060; found, 430.2063.

**(Z)-4-((3-(6-(4-(ammoniomethyl)-1H-1,2,3-triazol-1-yl)hexyl)benzo[d]thiazol-2(3H)-ylidene)methyl)-1-ethylquinolin-1-ium (6-TramTO-1, 1):** Under inert atmosphere, azido derivative **6-AzTO-1 (12,** 60.0 mg, 108 µmol) is dissolved in ethanol (1.13 mL) and propargylamine (14.2 µL, 222 µmol), copper powder (8.12 mg, 128 µmol) and copper sulfate (100 mM, 53.8 µL, 5.38 µmol) are added. The resulting mixture is stirred for 20 h, filtered and the solvent removed. The product is purified by preparative HPLC (Gilson 5-75%) to yield the desired product **(1,** 38.0 mg, 53.3 µmol, 50%) as red solid. **R*ₜ*** = 20.2 min. **¹H-NMR** (500 MHz, DMSO-*d₆*, δ): 8.75 (d, 1H, *J* = 8.8 Hz, H-16), 8.67 (d, 1H, *J =* 7.2 Hz, H-13), 8.29 (br s, 3H, NH₃⁺), 8.17 (d, 1H, *J* = 8.8 Hz, H-19), 8.10 (s, 1H, H-20), 8.05 (dd, 1H, *J =* 7.9 Hz, *J =* 0.9 Hz, H-4), 8.03 - 7.99 (m, 1H, H-17), 7.80 - 7.75 (m, 2H, H-1, H-18), 7.64 - 7.59 (m, 1H, H-2), 7.45 - 7.41 (m, 2H, H-3, H-12), 6.94 (s, 1H, H-11), 4.66 (q, 2H, *J* = 7.2 Hz, H-14), 4.63 (t, 2H, *J* = 7.4 Hz, H-5), 4.37 (t, 2H, *J* = 7.0 Hz, H-10), 4.12 - 4.06 (m, 2H, H-21), 1.83 - 1.75 (m, 4H, H-6, H-9), 1.54 - 1.46 (m, 5H, H-8, H-15), 1.36 - 1.28 (m, 2H, H-7). **¹³C-NMR** (125 MHz, DMSO-*d₆*, δ): 159.4 (C_{q}), 148.7 (C_{q}), 144.0 (CH), 140.0 (C_{q}), 136.9 (C_{q}), 133.3 (CH), 128.2 (CH), 126.9 (CH), 125.7 (CH), 124.5 (CH), 124.3 (C_{q}), 124.1 (2C, CH, C_{q}), 123.9 (C_{q}), 123.0 (CH), 118.0 (CH), 113.0 (CH), 108.3 (CH), 87.6 (CH), 49.5 (CH₂), 49.3 (CH₂), 45.6 (CH₂), 33.9 (CH₂), 29.6 (CH₂), 26.8 (CH₂), 25.6 (CH₂), 25.4 (CH₂), 14.7 (CH₃). **HRMS-ESI⁺** (*m*/*z*): [M]⁺ calcd for C₂₈H₃₃N₆S⁺, 485.2482; found, 485.2487.

### Synthesis of 6-xxxTO-1 and of 6-xxxTO-3

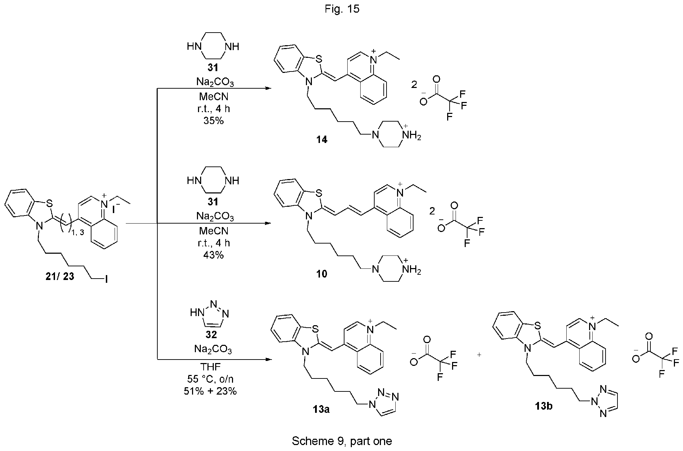

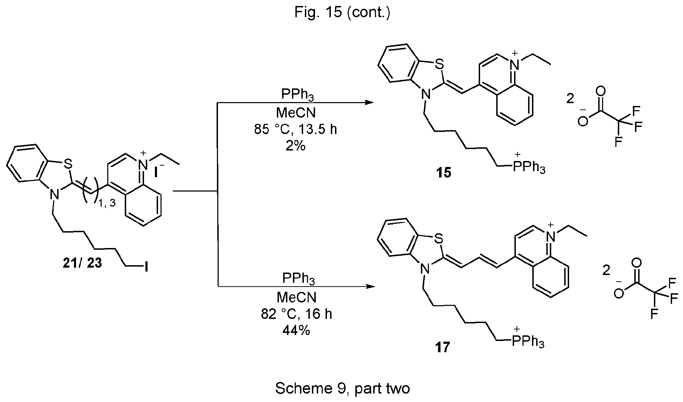

This synthesis is based on the nucleophilic substitution of the iodo substituent of 6-lodoTO-1 and 6-lodoTO-3 (cf. Fig. 15, scheme 9).

**(Z)-1-ethyl-4-((3-(6-(piperazin-1-ium-1-yl)hexyl)benzo[d]thiazol-2(3H)-ylidene)methyl)quinolin-1-ium (6-PipTO-1, 14):** lodo derivative **6-IodoTO-1 (21,** 100 mg, 156 µmol) and Na₂CO₃ (24.5 mg, 231 µmol) are suspended in MeCN (320 µL) and piperazine (48.9 µL, 624 µmol) added. The reaction mixture is stirred for 4 h at r.t., water added and the product extracted with CH₂Cl₂ and dried over MgSO₄. The product is purified by preparative HPLC (Gilson 5-75%) to yield the desired product **(14,** 38.3 mg, 54.7 µmol, 35%) as red solid. **R*ₜ*** = 18.0 min. **¹H-NMR** (500 MHz, DMSO-*d₆*, δ): 9.32 (br s, 2H, NH₂⁺), 8.76 (d, 1H, *J =* 8.0 Hz, H-16), 8.67 (d, 1H, *J =* 7.3 Hz, H-13), 8.17 (d, 1H, *J* = 8.6 Hz, H-19), 8.06 (dd, 1H, *J =* 7.8 Hz, *J* = 0.6 Hz, H-4), 8.03 - 7.99 (m, 1H, H-17), 7.80 - 7.75 (m, 2H, H-1, H-18), 7.64 - 7.59 (m, 1H, H-2), 7.46 - 7.41 (m, 2H, H-3, H-12), 6.95 (s, 1H, H-11), 4.69 - 4.62 (m, 4H, H-14, H-5), 3.49 - 3.09 (m, 8H, H-20, H-20', H-21, H-21'), 3.05 - 2.95 (m, 2H, H-10), 1.86 - 1.77 (m, 2H, H-9), 1.63 - 1.55 (m, 2H, H-6), 1.52 - 1.45 (m, 5H, H-8, H-15), 1.40 1.33 (m, 2H, H-7). **¹³C-NMR** (125 MHz, DMSO-*d₆*, δ): 159.4 (C_{q}), 148.8 (C_{q}), 144.0 (CH), 140.0 (C_{q}), 136.9 (C_{q}), 133.3 (CH), 128.2 (CH), 126.9 (CH), 125.7 (CH), 124.5 (CH), 124.3 (C_{q}), 123.9 (C_{q}), 123.0 (CH), 118.0 (CH), 113.0 (CH), 108.3 (CH), 87.6 (CH), 55.8 (CH₂), 49.5 (CH₂), 48.1 (CH₂), 45.6 (CH₂), 40.5 (CH₂), 26.8 (CH₂), 23.3 (CH₂), 25.8 (CH₂), 25.6 (CH₂), 14.7 (CH₃). **HRMS-ESI⁺** (*m*/*z*): [M]⁺ calcd for C₂₉H₃₇N₄S⁺, 473.2733; found, 473.2731.

**1-ethyl-4-((1E,3Z)-3-(3-(6-(piperazin-1-yl)hexyl)benzo[d]thiazol-2(3H)-ylidene)prop-1-en-1-yl)quinolin-1-ium (6-PipTO-3, 10):** The iodo derivate **23**(100 mg, 150 µmol, 1.00 eq) and Na₂CO₃ (24.5 mg, 231 µmol, 1.54 eq) are suspended in acetonitrile (320 µL) and piperazine (53.8 mg, 624 mmol, 4.16 eq) is added. The reaction mixture is stirred for 6 h at room temperature. Distilled water (1.0 mL) is added, and the product is extracted with CH₂Cl₂ and dried over MgSO₄. The solvent is evaporated under reduced pressure, and the crude is purified using a Büchi pure system with MeCN in H₂O (0 % → 45 %) as a solvent to yield the desired product **12** (40.3 mg, 64.3 µmol, 43 %) as a blue **solid.¹H-NMR** (500 MHz, DMSO-*d₆*, *δ*): 8.51- 8.44 (m, 2H, H-11, H-12), 8.16 (t, 1H, ³*J* = 12.7 Hz, H-6), 8.12- 8.08 (m, 1H, H-8), 7.97 (td, 1H, ³*J* = 7.9 Hz, ⁴*J* = 1.3 Hz, H-10), 7.91- 7.87 (m, 2H, H-4, H-13), 7.74- 7.69 (m, 1H, H-9), 7.63- 7.58 (m, 1H, H-1), td (7.49, 1H, ³*J* = 7.8 Hz, ⁴*J* = 1.1 Hz, H-3), 7.34- 7.27 (m, 1H, H-2), 7.19- 7.13 (m, 1H, H-7), 6.53 (d, 1H, ³*J* = 12.2 Hz, H-5), 4.61 (q, 2H, ³*J* = 7.2 Hz, H-14), 4.26-4.21 (m, 2H, H-16), 3.14-2.99 (m, 3H, H-21), 1.78- 1.70 (m, 2H, H-20), 1.70- 1.57 (m, 2H, H-17), 1.49- 1.42 (m, 4H, H-15, H-19), 1.41- 1.35 (m, 2H, H-18) **ppm.¹³C-NMR** (75 MHz, DMSO-*d₆*, *δ*): 160.8 (C_{q}), 158.4 (C_{q}), 158.1 (C_{q}), 150.5 (C_{q}), 141.5 (C_{q}), 137.8 (C_{q}), 144.0 (C-8), 142.3 (C-12), 133.5 (C-10), 127.7 (C-3), 126.7 (C-9), 125.3 (C-11), 124.3 (C-2), 122.6 (C-13), 118.1 (C-6), 112.5 (C-1), 110.1 (C-4), 109.6 (C-7), 98.5 (C-5), 55.7 (C-22, C-26), 49.4 (C-14), 47.9 (C-23, C-25), 45.5 (C-16), 26.9 (C-19), 25.8 (C-18), 25.7 (C-17), 14.8 (C-15) ppm.**HRMS-ESI⁺** (*m*/*z*): [M+2H]⁺ calcd. for C₃₁H₃₉N₄S⁺ 499.2890; found 501.2684.

**(Z)-4-((3-(6-(1H-1,2,3-triazol-1-yl)hexyl)benzo[d]thiazol-2(3H)-ylidene)methyl)-1-ethylquinolin-1-ium (6-Tri_{(asym.)}TO-1, 13a) and (Z)-4-((3-(6-(2H-1,2,3-triazol-2-yl)hexyl)benzo[d]thiazol-2(3H)-ylidene)methyl)-1-ethylquinolin-1-ium (6-Tri_{(sym.)}TO-1, 13b):** lodo derivative **6-IodoTO-1 (21,** 150.0 mg, 0.23 mmol) and sodium carbonate (99.0 mg, 0.93 mmol) are dissolved in THF (467 µL) and 1-H-1,2,3-triazole (16, 83.1 µL, 0.47 mmol) is added. The reaction mixture is stirred in the dark for 3 h at 55 °C. 1-*H*-1,2,3-triazole **(16,** 83.1 µL, 0.47 mmol) is added and the mixture is stirred overnight under the same conditions. The solution is washed with distilled water. The aqueous layer is extracted with CH₂Cl₂ and the organic phase is dried over magnesium sulfate. The crude products are purified by preparative HPLC (Gilson 5-75%) to yield **6-Tri_{(asym.)}TO-1 (13a,** 69.6 mg, 0.12 mmol, 51%) and **6-Tri_{(sym.)}TO-1 (13b,** 31.7 mg, 0.05 mmol, 23%) as pink solids.

### 6-Tri_{(asym.)}TO-1 (13a):

**R*ₜ*** = 21.6 min (gradient 2). **¹H-NMR** (300 MHz, DMSO-*d₆*, δ): 8.73 (d, 1H, *J* = 8.5 Hz, H-16), 8.66 (d, 1H, *J* = 7.2 Hz, H-13), 8.17 (d, 1H, *J* = 8.6 Hz, H-19), 8.10 - 7.97 (m, 3H, H-4, H-17, H-20), 7.82 - 7.74 (m, 2H, H-1, H-18), 7.67 (s, 1H, H-21), 7.65 - 7.58 (m, 1H, H-2), 7.47 - 7.39 (m, 2H, H-3, H-12), 6.93 (s, 1H, H-11), 4.70 - 4.59 (m, 4H, H-14, H-5), 4.35 (m, 2H, *J* = 7.0 Hz, H-10), 1.87 - 1.74 (m, 4H, H-9, H-6), 1.54 - 1.43 (m, 5H, H-8, H-15), 1.36 - 1.26 (m, 2H, H-7). **¹³C-NMR** (75 MHz, DMSO-*d₆*, δ): 159.4 (C_{q}), 148.7 (C_{q}), 144.0 (CH), 140.0 (C_{q}), 136.8 (C_{q}), 133.3 (CH), 133.1 (CH), 128.2 (CH), 126.9 (CH), 125.7 (CH), 124.5 (2C, CH), 124.3 (C_{q}), 123.9 (C_{q}), 122.9 (CH), 118.0 (CH), 112.9 (CH), 108.3 (CH), 87.6 (CH), 49.5 (CH₂), 49.0 (CH₂), 45.6 (CH₂), 29.5 (CH₂), 26.7 (CH₂), 25.6 (CH₂), 25.4 (CH₂), 14.6 (CH₃). **HRMS-ESI⁺** (*m*/*z*): [M]⁺ calcd for C₂₇H₃₀N₅S⁺, 456.2216; found, 456.2212.

### 6-Tri_{(sym)}TO-1 (13b):

**R*ₜ*** = 23.6 min (gradient 2). **¹H-NMR** (300 MHz, DMSO-*d₆*, δ): 8.72 (d, 1H, *J* = 8.2 Hz, H-16), 8.66 (d, 1H, *J* = 7.2 Hz, H-13), 8.16 (d, 1H, *J* = 8.6 Hz, H-19), 8.07 - 7.97 (m, 2H, H-4, H-17), 7.80 - 7.73 (m, 2H, H-1, H-18), 7.70 (s, 2H, H-20, H-20'), 7.64 - 7.57 (m, 1H, H-2), 7.45 - 7.38 (m, 2H, H-3, H-12), 6.91 (s, 1H, H-11), 4.70 - 4.56 (m, 4H, H-14, H-5), 4.38 (m, 2H, *J* = 6.9 Hz, H-10), 1.88 - 1.73 (m, 4H, H-9, H-6), 1.52 - 1.42 (m, 5H, H-8, H-15), 1.34 - 1.26 (m, 2H, H-7). **¹³C-NMR** (75 MHz, DMSO-*d₆*, δ): 159.4 (C_{q}), 148.7 (C_{q}), 144.0 (CH), 140.0 (C_{q}), 136.9 (C_{q}), 134.0 (2C, CH), 133.3 (CH), 128.2 (CH), 126.9 (CH), 125.7 (CH), 124.5 (CH), 124.3 (C_{q}), 123.9 (C_{q}), 122.9 (CH), 118.0 (CH), 112.9 (CH), 108.3 (CH), 87.6 (CH), 53.8 (CH₂), 49.4 (CH₂), 45.6 (CH₂), 28.9 (CH₂), 26.7 (CH₂), 25.6 (CH₂), 25.3 (CH₂), 14.6 (CH₃). **HRMS-ESI⁺** (*m*/*z*): [M]⁺ calcd for C₂₇H₃₀N₅S⁺, 456.2216; found, 456.2213.

**(Z)-1-ethyl-4-((3-(6-(triphenylphosphonio)hexyl)benzo[d]thiazo1-2(3H)-ylidene)methyl)quinolin-1-ium (6-PPh₃TO-1, 15):** lodo derivative **6-IodoTO-1 (21,** 118 mg, 184 µmol) and PPh₃ (145 mg, 551 µmol) are suspended in MeCN (3.00 mL) and stirred for1 h at r.t. and 13.5 h at 85 °C. The solvent is removed and the crude filtered over whool. The product is purified by preparative HPLC (Gilson 5-75%) to yield the desired product **(15, 3.50** mg, 3.88 µmol, 2%) as red solid. **R*ₜ*** = 25.1 min (gradient 1). **HRMS-ESI⁺** (*m*/*z*)*:* [M]²⁺ calcd for C₄₃H₄₃N₂PS²⁺, 325.1437; found, 325.1437.

**1-ethyl-4-((1E,3Z)-3-(3-(6-(triphenylphosphonio)hexyl)benzo[d]thiazol-2(3H)-ylidene)prop-1-en-1-yl)quinolin-1-ium (6-PPh₃TO-3, 17):** derivative **6-IodoTO-3 (23,** 49.9 mg, 74.7 µmol) and PPh₃ (58.9 mg, 224 µmol) are suspended in MeCN (3.00 mL) and stirred 16 h at 82 °C. The solvent is removed and the crude filtered overwhool. The product is purified by preparative HPLC (Gilson 5-75%) to yield the desired product **(17,** 1.90 mg, 2.10 µmol, 44%) as blue solid. **R*ₜ*** = 26.9 min (gradient 1); **¹H-NMR (500 MHz, DMSO-*d₆*)** *δ* = 8.45 (d, *³J_{H-H}* = 7.1 Hz, 1H, H23), 8.42 (d, *³J_{H-H} =* 8.2 Hz, 1H, H26), 8.14 (t, *³J_{H-H} =* 12.8 Hz, 1H, H18), 8.09 (d, *³J_{H-H} =* 8.8 Hz, 1H, H29), 7.95 (t, *³J_{H-H} =* 7.3 Hz, 1H, H31), 7.87 (m, 5H, H3, H24, H37, H42, H47), 7.76 (m, 12H, H35, H36, H38 - 41, H43 - 46, H48, H49), 7.67 (t, *³J_{H-H} =* 7.7 Hz, 1H, H30), 7.56 (d, *³J_{H-H} =* 8.3 Hz, 1H, H6), 7.46 (t, *³Js.ₙ =* 7.3 Hz, 1H, H2), 7.30 (t, *³J_{H-H} =* 7.8 Hz, 1H, H1), 7.11 (d, *³J_{H-H}* = 13.3 Hz, 1H, H19), 6.50 (d, *³J_{H-H} =* 12.3 Hz, 1H, H10), 4.61 (q, *³J_{H-H} =* 7.1 Hz, 2H, H21), 4.19 (t, *³J_{H-H} =* 7.4 Hz, 2H, H11), 3.59 (m, 2H, H16), 1.68 (m, 2H, H12), 1.59 (s, 4H, H13, H14), 1.34 (t, *³J_{H-H} =* 7.1 Hz, 5H, H15, H20) ppm; **¹³C-NMR (126 MHz, DMSO-*d₆*) *δ*** = 160.9 (s, 1C, C25), 150.4 (s, 1C, C8), 143.9 (s, 1C, C18), 142.3 (s, 1C, C23), 141.5 (s, 1C, C27), 137.7 (s, 1C, C4), 134.9 (s, 3C, C37, C42, C47), 133.6 (d, 6C, C35, C39, C40, C44, C45, C49), 133.5 (s, 1C, C31), 130.3 (d, 3C, C36, C38, C41, C43, C46, C48), 127.7 (s, 1C, C2), 126.7 (s, 1C, C30), 125.2 (s, 1C, C26), 124.8 (s, 1C, C1), 124.3 (s, 1C, C5), 122.7 (s, 1C, C24), 118.9 (s, 3C, C32-34), 118.2 (s, 1C, C29), 112.4 (s, 1C, C6), 110.0 (s, 1C, C3), 109.5 (s, 1C, C19), 98.5 (s, 1C, C10), 49.4 (s, 1C, C21), 45.4 (s, 1C, C11), 29.4 (s, 1C, C13), 26.6 (s, 1C, C12), 25.0 (s, 1C, C15), 21.6 (s, 1C, C14), 20.1 (s, 1C, C16), 14.7 (s, 1C, C20) ppm. **HRMS-ESI⁺** (*m*/*z*): [M]²⁺ calcd for C₄₅H₄₅N₂PS²⁺, 338.1515; found, 338.1516.

### Synthesis of CH₃TAS

The synthesis of the CH₃TAS **(5)** is known to the person skilled in the art.

### Synthesis of CH₃TO dyes 4 and 6

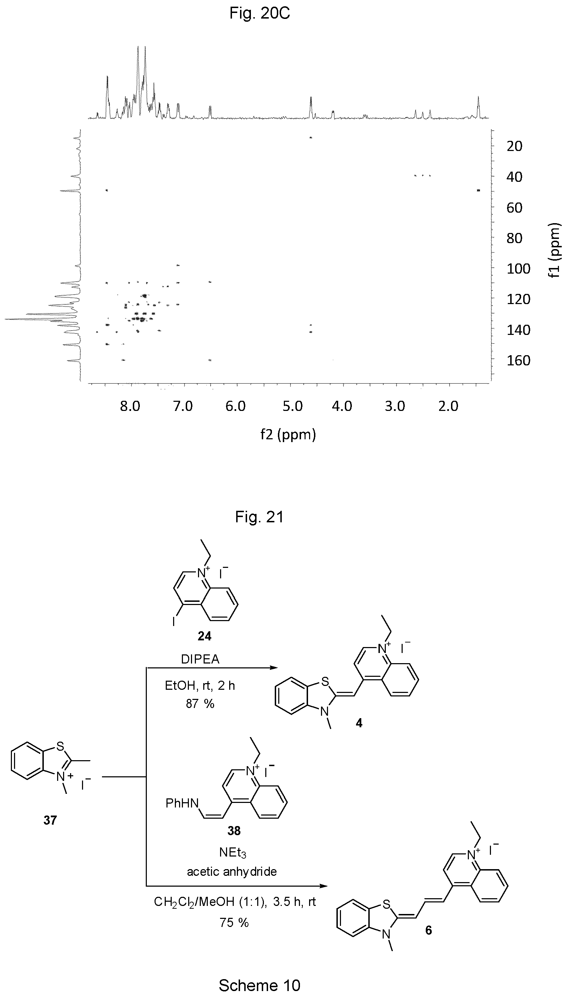

The Synthesis route of dyes CH₃TO-1 **(4)** and CH₃TO-3 **(6),** starting from benzothiazole **37,** is depicted in Fig. 21 (scheme 10).

**(Z)-1-ethyl-4-((3-methylbenzo[d]thiazol-2(3H)-ylidene)methyl)quinolin-1-ium (4):** Benzothiazolium **37** (117mg, 400 µmol, 1.00 eq) and quinilinium **24** (128 mg, 400 µmol, 1.00 eq) are suspended in ethanol (6.0 mL), diisopropylethylamine (0.15 mL, 884 µmol, 2.21 eq) is added and the solution stirred at room temperature in the dark for 2 h. The solvent is evaporated, and the crude purified by flash column chromatography (basic allox, MeOH in CH₂Cl₂: 0% → 0.2% → 0.5% → 0.7%) to yield the desired product **4** (156 mg, 350 µmol, 87 %) as red solid. ***R_{f}* =** 0.39 (CH₂Cl₂/MeOH 10:1 + 1 drop formic acid). **¹H-NMR** (500 MHz, DMSO-*d*₆, *δ*): 8.76 (d, 1H, ³*J* = 8.3 Hz, H-16), 8.64 (d, 1H, ³*J* = 7.1 Hz, H-21), 8.10 (d, 1H, ³*J* = 8.6 Hz, H-18), 8.01 (d, 1H, ³*J* = 7.8 Hz, H-3), 7.96 (t, 1H, ³*J* = 7.9 Hz, H-20), 7.75 - 7.70 (m, 2H, H-19, H-6), 7.56 (t, 1H, ³*J* = 7.5 Hz, H-1), 7.37 (t, 1H, ³*J* = 7.5 Hz, H-2), 7.29 (d, 1H, ³*J* = 7.1 Hz, H-17), 6.85 (s, 1H, H-10), 4.61 (q, 2H, ³*J* = 7.2 Hz, H-22), 3,97 (s, 3H, H-11), 1.46 (t, 3H, ³*J* = 7.2 Hz, H-23) ppm.**¹³C-NMR** (75 MHz, DMSO-*d₆*, *δ*): 159.8 (C-12), 148.4 (C-8), 143.93 (C-21), 140.4 (C-14), 136.8 (C-4), 133.3 (C-20), 128.1 (C-1), 126.8 (C-19), 125.9 (C-16), 124.4 (C-2), 124.2 (C-13), 123.8 (C-5), 122.9 (C-3), 118.0 (C-18), 112.9 (C-6), 108.0 (C-17), 88.0 (C-10), 49.4 (C-22), 33.9 (C-11), 14.7 (C-23) ppm. **HRMS-ESI⁺** (*m*/*z*): [M]⁺ calcd. for C₂₀H₁₉N₂S⁺: 319.1263, found: 319.1259.

**(Z)-1-ethyl-4-((3-methylbenzo[d]thiazol-2(3H)-ylidene)methyl)quinolin-1-ium (6):** Benzothiazolium **37** (147 mg, 505 µmol, 1.26 eq) and quinilinium **38** (161 mg, 400 µmol, 1.00 eq) are dissolved in CH₂Cl₂/Methanol (1:1, 4 mL), Acetic anhydride (0.4 mL) and triethylamine (0.4 mL) are added and the reaction is stirred for 3.5 h at room temperature. The crude dye is precipitated by pouring the reaction mixture in diethylether. The precipitate is washed with diethylether and purified by flash column chromatography (basic allox, MeOH in CH₂Cl₂: 0% → 0.2% → 0.5% → 0.7%) to yield the desired product **6** (178 mg, 377 µmol, 75 %) as dark blue solid. ***R_{f}* =** 0.31 (CH₂Cl₂/MeOH 10:1 + 1 drop formic acid). **¹H-NMR** (500 MHz, DMSO-*d*₆, *δ*): 8.44 (d, 1H, ³*J* = 8.6 Hz, H-23), 8.42 (d, 1H, ³*J* = 7.2 Hz, H-17), 8.11 (t, 1H, ³*J* = 12.8 Hz, H-11), 8.06 (d, 1H, ³*J* = 8.8 Hz, H-20), 7.94 (t, 1H, ³*J* = 7.5 Hz, H-22), 7.84 (t, 2H, ³*J* = 7.0 Hz, H-18, H-3), 7.69 (t, 1H, ³*J* = 7.7 Hz, H-21), 7.54 (d, 1H, ³*J* = 8.2 Hz, H-6), 7.44 (t, 1H, ³*J* = 7.3 Hz, H-2), 7.27 (t, 1H, ³*J* = 7.5 Hz, H-1), 7.08 (d, 1H, ³*J* = 13.3 Hz, H-12), 6.47 (d, 1H, , ³*J* = 12.3 Hz, H-10), 4.58 (q, 2H, ³*J* = 7.0 Hz, H-24), 3.71 (s, 3H, H-19), 1.44 (t, 3H, ³*J* = 7.0 Hz, H-25) ppm. **¹³C-NMR** (75 MHz, DMSO-*d*₆, *δ*): 161.5 (C-13), 150.3 (C-8), 143.7 (C-11), 142.2 (C-17), 141.9 (C-15), 137.7 (C-4), 133.4 (C-22), 127.6 (C-2), 126.7 (C-21), 125.2 (C-23), 124.2 (C-14), 124.1 (C-1), 122.5 (C-18), 117.8 (C-20), 112.4 (C-6), 109.3 (C-12), 98.8 (C-10), 49.3 (C-24), 32.9 (C-19), 14.7 (C-25) ppm. **HRMS-ESI⁺** (*m*/*z*): [M]⁺ calcd. for C₂₂H₂₁N₂S⁺: 345.1420, found: 345.1417.

### Synthesis of 6-TramTAS

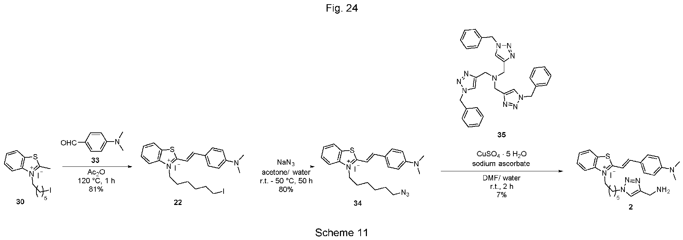

This synthesis is based on the scaffold of cyanine dyes described within the state of the art (cf. Fig. 24, scheme 11).

**(E)-2-(4-(dimethylamino)styryl)-3-(6-iodohexyl)benzo[d]thiazol-3-ium (6-IodoTAS, 22)** : Benzothiazolium **(30,** 1.00 g, 2.05 mmol) and dimethyl aminobenzaldehyd (306 mg, 2.05 mmol) are dissolved in Ac₂O (26.8 mL) and the mixture stirred at 120°C for 1 h. Water (26.8 mL) is added and the mixture stirred at 100 °C for 30 min. The reaction mixture was cooled to 25°C, the solvent removed, the product is filtered off and washed with acetone to yield the desired product **(22,** 1.03 mg, 1.67 mmol, 81%) as purple solid. **R*_{f}* =** 0.42 (CH₂Cl₂/MeOH 10:1). **¹H NMR** (300 MHz, DMSO-*d₆*, δ): 8.30 (d, 1H, *J* = 7.9 Hz, C*H*-4), 8.16 - 8.05 (m, 2H, C*H*-1, C*H*-11), 7.92 (d, 2H, J *=* 8.9 Hz, C*H*-14, C*H*-14'), 7.82 - 7.74 (m, 1H, C*H*-2), 7.72 - 7.64 (m, 1H, C*H*-3), 7.60 (d, 1H, *J =* 15.3 Hz, C*H*-12), 6.86 (d, 2H, *J* = 8.9 Hz, CH-13, C*H*-13'), 4.79 (t, 2H, *J* = 7.3 Hz, C*H*₂-5), 3.25 (t, 2H, *J =* 6.8 Hz, C*H*₂-10), 3.12 (s, 6H, C*H*₃-15, C*H*₃-15'), 1.88-1.78 (m, 2H, C*H*₂-6), 1.78-1.67 (m, 2H, C*H*₂-9), 1.53-1.35 (m, 4H, C*H*₂-7, C*H*₂-8). **¹³C NMR** (75 MHz, DMSO-*d₆*, δ): 171.0 (C_{q}), 153.5 (C_{q}), 150.5 (CH), 141.0 (C_{q}), 132.9 (2C, CH), 128.9 (CH), 127.3 (CH), 127.0 (C_{q}), 123.9 (CH), 121.4 (C_{q}), 115.8 (CH), 111.9 (2C, CH), 105.6 (CH), 47.8 (CH₂), 39.7 (2C, CH₃), 32.6 (CH₂), 29.4 (CH₂), 28.1 (CH₂), 24.6 (CH₂), 8.6 (CH₂). **HRMS-ESI⁺** (*m*/*z*): [M]⁺ calcd for C₂₃H₂₈IN₂S⁺, 491.1012; found, 491.1008.

**(E)-3-(6-azidohexyl)-2-(4-(dimethylamino)styryl)benzo[d]thiazol-3-ium (6-azindoTAS, 34)** : lodo-derivative **(22, 500** mg, 0.81 mmol) and sodium azide (210 mg, 3.24 mmol) are dissolved in acetone (3.3 mL) and water (3.3 mL) and the mixture is stirred at r.t. for 17 h. Sodium azide (105 mg, 1.62 mmol) is added and the mixture is stirred at r.t. 25 h. Sodium azide (210 mg, 3.24 mmol) is added and the mixture is stirred at 50 °C for 3 h. The solvent is evaporated and the crude extracted in CH₂Cl₂ and water. The organic layers are dried over MgSO₄ and purified by flash column chromatography (allox, MeOH in CH₂Cl₂: 0.2% → 0.5%→ 1.0% → 5.0%→ 10%) to yield the desired product **(34, 350** mg, 0.65 mmol, 80%) as purple solid. **R*_{f}* =** 0.38 (CH₂Cl₂/MeOH 10:1). **¹H NMR** (300 MHz, DMSO-*d₆*, δ): 8.31 (d, 1H, *J =* 7.9 Hz, C*H*-4), 8.17 - 8.05 (m, 2H, C*H-*1, C*H*-11), 7.93 (d, 2H, *J* = 8.8 Hz, C*H*-14, C*H*-14'), 7.82 - 7.74 (m, 1H, C*H*-2), 7.72 - 7.57 (m, 2H, C*H*-3, C*H*-12), 6.85 (d, 2H, *J* = 8.8 Hz, C*H*-13, C*H*-13'), 4.80 (t, 2H, *J* = 7.1 Hz, C*H*₂-5), 3.31 - 3.24 (m, 2H, C*H*₂-10), 3.12 (s, 6H, C*H*₃-15, C*H*₃-15'), 1.89-1.74 (m, 2H, C*H*₂-6), 1.53-1.36 (m, 6H, C*H*₂-7, C*H*₂-8, C*H*₂-9). ¹³C **NMR** (75 MHz, DMSO-*d₆*, δ): 171.1 (C_{q}), 153.5 (C_{q}), 150.5 (CH), 141.1 (C_{q}), 132.9 (2C, CH), 128.9 (CH), 127.4 (CH), 127.0 (C_{q}), 123.9 (CH), 121.4 (C_{q}), 115.9 (CH), 111.9 (2C, CH), 105.7 (CH), 50.5 (CH₂), 47.8 (CH₂), 39.7 (2C, CH₃), 28.2 (CH₂), 28.0 (CH₂), 25.7 (CH₂), 25.2 (CH₂). **HRMS-ESI⁺** (*m*/*z*): [M]⁺ calcd for C₂₃H₂₈N₅S⁺, 406.2060; found, 406.2059.

**(E)-3-(6-(4-(aminomethyl)-1H-1,2,3-triazol-1-yl)hexyl)-2-(4-(dimethylamino)styryl)benzo[d]thiazol-3-ium (6-TramTAS, 2):** 6-azindoTAS **(34,** 41.9 mg, 78.5 µmol, 1.00 equiv.), copper sulfate pentahydrate (13.4 mg, 53.7 µmol, 0.68 equiv.), sodium ascorbate (10.6 mg, 53.5 µmol, 0.68 equiv.) and TBTA **(35, 7.6** mg, 14.3 µmol, 0.18 equiv.) are dissolved in water (0.8 mL) and DMF (2.4 mL). Propargylamine (48 µL, 749 µmol, 9.54 equiv.) is added and the reaction mixture is stirred at room temperature in the dark for 2 h. Afterward, the reaction mixture is treated with aqueous sodium sulfide solution and filtered over Celite. After removal of the solvent under reduced pressure the crude is purified by preparative HPLC (Varian 5-35%) to yield the desired product **(2, 3.3** mg, 5.74 µmol, 7%) as dark red solid. **R*ₜ*** = 19.7 min (gradient 1); **¹H-NMR** (500 MHz, DMSO-*d₆*) δ = 8.30 (dd, *³J_{H-H} =* 8.1 Hz, *⁴J_{H-H} =* 0.8 Hz, 1H, H3), 8.28 (s, 2H, H33), 8.10 (m, 3H, H1, H2, H3), 7.92 (d, *³J_{H-H}* = 9.0 Hz, 2H, H17, H21), 7.78 (m, 1H, H20), 7.68 (t, *³J_{H-H} =* 7.7 Hz, 1H, H18), 7.60 (d, *³J_{H-H} =* 15.2 Hz, H6), 6.85 (d, *³J_{H-H} =* 9.0 Hz, 2H, H22, H23), 4.79 (t, *³J_{H-H} =* 7.4 Hz, 2H, H10), 4.37 (t, *³J_{H-H} =* 7.1 Hz, 2H, H15), 4.10 (q, *³J_{H-H} =* 5.6 Hz, 2H, H32), 3.12 (s, 6H, H25, H26), 1.78 (dt, *³J_{H-H} =* 14.6 Hz, *³J_{H-H} =* 7.2 Hz, 4H, H11, H14), 1.46 (m, 2H, H12), 1.30 (m, 2H, H13) ppm; **¹³C-NMR** (125.76 MHz, DMSO-*d*₆) δ = 171.2 (s, 1C, C8), 153.6 (s, 1C, C19), 150.6 (s, 1C, C1), 141.2 (s, 1C, C4), 140.0 (s, 1C, C31), 133.0 (s, 1C, C2), 128.9 (s, 1C, C5), 127.5 (s, 1C, C18), 127.1 (s, 1C, C20), 124.1 (s, 2C, C17, C21), 124.0 (s, 1C, C16), 121.4 (s, 1C, C3), 115.9 (s, 1C, C30), 112.0 (s, 2C, C22, C23), 105.7 (s, 1C, C6), 49.3 (s, 1C, C15), 47.8 (s, 1C, C10), 40.0 (s, 2C, C25, C26), 33.9 (s, 1C, C32), 29.6 (s, 1C, C11), 28.2 (s, 1C, C14), 25.5 (s, 1C, C13), 25.2 (s, 1C, C12) ppm; **HRMS (ESI⁺)** m/z: calculated for C₂₆H₃₃N₆S: 461.2500; found: 461.2481.

### Synthesis of yellow PPh3 dye 16

Under exclusion of light 88.0 mg of **22** (0.14 mmol, 1.00 eq.) is dissolved in 1.8 mL MeCN. 112 mg of PPh₃ (0.43 mmol, 3.00 eq.) is added. The reaction mixture is heated to 85 °C for 3 h. After concentrating to dryness, the crude product is purified by preparative HPLC (gradient: 25-40% MeCN in water over 40 min). 90 mg of **16** (0.10 mmol, 72%) were obtained as dark red solid. ***R_{f}*** = 0.22 (DCM/MeOH 10:1); **¹H-NMR (500 MHz, DMSO-*d₆*)** δ = 8.31 (d, *³J_{H-H} =* 8.1 Hz, 1H, H3), 8.09 (t, *³J_{H-H} =* 11.6 Hz, 2H, H18, H22), 7.91 *(d, ³J_{H-H} =* 9.0 Hz, 2H, H19, H21), 7.87 (m, 3H, H33, H38, H43), 7.75 (m, 12H, H31, H32, H34 - 37, H39 - 42, H44, H45), 7.72 (d, *³J_{H-H}* = 3.9 Hz, 1H, H2), 7.68 (m, 1H, H1), 7.58 (d, *³J_{H-H} =* 15.2 Hz, 1H, H6), 6.81 (d, *³J_{H-H} =* 9.1 Hz, 2H, H23, H24), 4.76 (t, *³J_{H-H} =* 7.4 Hz, 2H, H10), 3.54 (t, *³J_{H-H} =* 11.3 Hz, 2H, H15), 3.11 (s, 6H, H26, H27), 1.74 (m, 2H, H11), 1.47 (m, 6H, H12 - 14) ppm; **¹³C-NMR (126 MHz, DMSO-*d₆*)** *δ* = 170.8 (s, 1C,C8), 153.2 (s, 1C, C20), 150.6 (s, 1C, C22), 141.0 (s, 1C, C4), 134.9 (d, 3C, C33, C38, C43), 133.6 (d, 6C, C31, C35, C36, C40, C41, C45), 133.0 (s, 2C, C19, C21), 130.3 (d, 6C, C32, C34, C37, C39, C42, C44), 129.0 (s, 1C, C2), 127.5 (s, 1C, C1), 129.0 (s, 1C, C5), 127.1 (s, 1C, C17), 124.0 (s, 1C, C3), 119.1 (s, 3C, C28-C30), 115.9 (s, 1C, C18), 111.9 (s, 2C, C23, C24), 105.7 (s, 1C, C6), 47.8 (s, 1C, C10), 39.8 (s, 2C, C26, C27), 29.4 (d, 1C, C12), 28.1 (s, 1C, C11), 24.8 (s, 1C, C13), 21.6 (d, 1C, C14), 20.1 (d, 1C, C15) ppm; **HRMS (ESI⁺)** m/z: calculated for C₄₁H₄₂N₂PS: 626.2900; found: 625.2806.

### Synthesis of yellow 6-AzTO-2 (40):

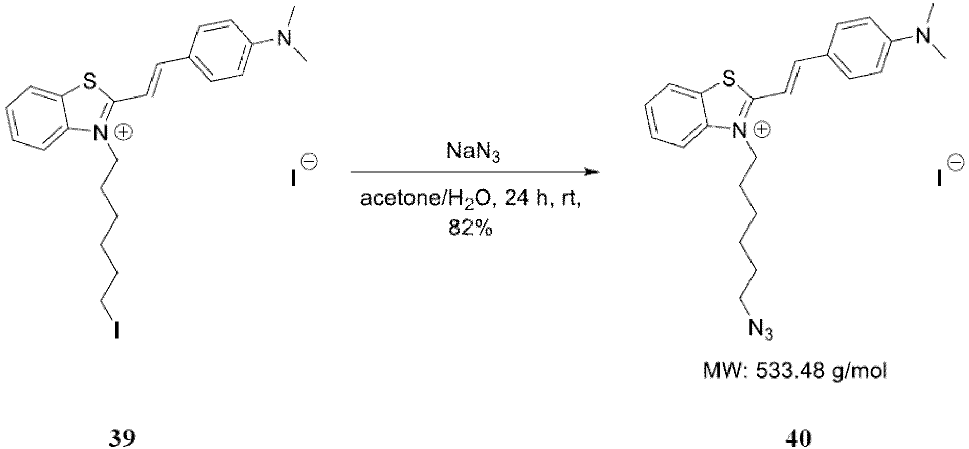

**Yellow 6-AzTO-2 (40):** Under exclusion of light and N₂-atmosphere 1.13 g of **39** (1.82 mmol, 1.00 eq.) and 1.18 g NaN₃ (18.2 mmol, 10.0 eq.) was dissolved in in 14.0 mL acetone/water (1:1) and stirred for 24 h at room temperature. The reaction mixture was heated to 50 °C for 1 h and further 2.00 eq. NaN₃ (0.24 g, 3.64 mmol) was added. The dark red solution was stirred at 50 °C for 1.5 h. 16 h at room temperature and again at 50 °C for 3 h. The solvent was evaporated and the residue dissolved in DCM and an aqueous NaCO₃ solution (1:1, 20 mL). The aqueous layer was extracted with DCM (2x 10 mL). The combined organic layers was washed with 5 mL brine, dried over MgSO₄, and concentrated to dryness under reduced pressure. The crude product was purified by flash column chromatography (gradient of MeOH in DCM: 0% → 0,2% → 0,5% → 1% → 5% → 10%). 0.8 g of **40** (14.9 mmol, 82%) were obtained as dark red solid. **R_{F}** = 0.64 (DCM/MeOH 10:1): **¹H-NMR (500 MHz, DMSO-*d₆*)** δ = 8.31 (d, *³J_{H-H} =* 8.0 Hz, 1H, H3), 8.14 (d, *³J_{H-H} =* 8.4 Hz, 1H, H22), 8.09 (d, *³J_{H-H} =* 15.2 Hz, 1H, H18), 7.93 (d, *³J_{H-H} =* 8.8 Hz, 2H, H19, H21), 7.78 (t, *³J_{H-H} =* 7.7 Hz, 1H, H2), 7.68 *(d, ³J_{H-H}* = 7.6 Hz, 1H, H1), 7.61 (d, *³J_{H-H} =* 15.2 Hz, 1H. H6), 6.85 (d, *³J_{H-H} =* 8.8 Hz, 2H. H23, H24), 4.80 (t, *³J_{H-H} =* 7.3 Hz, 2H, H10), 3.29 (t, *³J_{H-H}* = 6.9 Hz, 2H, H15), 3.12 (s, 6H, H26, H27), 1.81 (m, 4H, H11), 1.45 (m, 4H, H12 - 14) ppm; **¹³C-NMR (500 MHz, DMSO-*d₆*)** *δ* = 171.1 (s, 1C, C8), 153.6 (s, 1C, C20), 150.6 (s, 1C, C18), 141.1 (s, 1C, C4), 13.2.9 (s, 2C, C19, C21) 128.9 (s, 1C, C2), 127.4 (s, 1C, C1), 127.1 (s, 1C, C17), 123.9 (s, 1C, C3), 121.4 (s, 1C, C5), 115.9 (s, 1C, C22), 111.9 (s, 2C, C23, C24), 105.7 (s, 1C, C6), 50.5 (s, 2C, C26, C27), 47.8 (s, 1C, C10), 40.0 (s, 1C, C15), 28.2 (s, 1C, C11). 28.0 (s, 1C, C14), 25.8 (s, 1C, C12), 25.2 (s, 1C, C13) ppm: **HRMS (ESI⁺)** m/z: calculated for C₂₃H₂₈N₅S: 406.2100; found: 406.2061.

### Synthesis of TBTA (54):

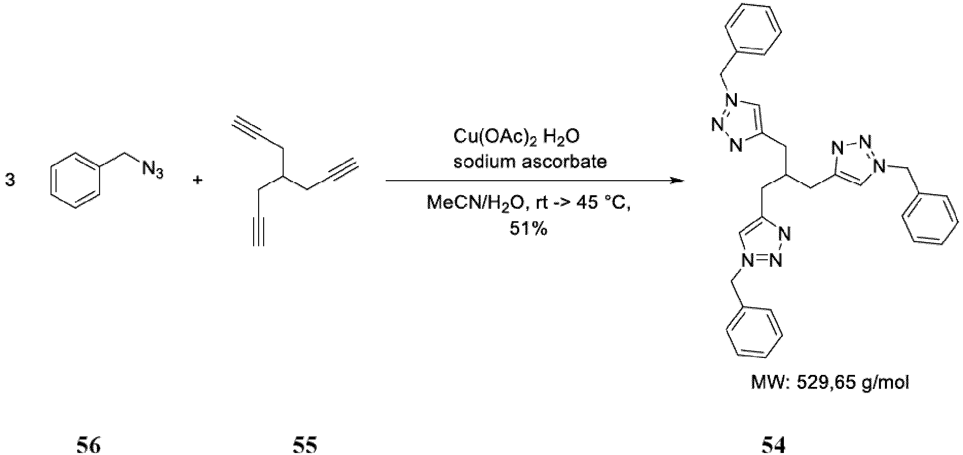

**Tris(benzyltriazolmethyl)amine (TBTA, 54):** 7.5 mL MeCN and 13.1 mg CuOAc₂ H₂O (66.0 µmol, 0.02 eq.) was stirred at room temperature until a blue solution was obtained. After addition of 0.7 mL benzylazide **(56.** 0.74 g, 5.6 mmol, 1.70 eq.) and 0.46 mL tripropargylamine **(55,** 430 mg, 3.28 mmol, 1.00 eq.) in 2.5 mL tvleCN the reaction mixture was stirred for further 5 min at room temperature. 13.0 mg sodium ascorbate (66.0 µmol, 0.02 eq.) was added and the solution was stirred at room temperature for 30 min and then at 45 °C for 4 h. After addition of further 0.7 mL benzylazide **(56,** 0.74 g, 5.6 mmol, 1.70 eq.) the yellowish solution was stirred for 16 h at 45 °C. The reaction mixture was concentrated to dryness and the residue was dissolved in 90 mL DCM/ NH₄OH (2:1). The aqueous layer was extracted with DCM (2x 20 mL) and the combined organic layers was washed with NH₄OH/brine 1:1 (2x 20 mL) and dried over MgSO₄. After filtration the solvent was concentrated to dryness under reduced pressure. The crude product was dissolved in a 5 mL. DCM and 10 mL DEE was added. The resulting suspension was centrifuged and the precipitate was dried under reduced pressure. 880 mg TBTA **(54,** 1.66 mmol, 51%) was obtained as a colorless solid. **R_{F}** = 0.60 (DCM/MeOH 10:1); **¹H-NMR (300 MHz, CDCl₃)** *δ* = 7.47 (s, 3H, H4), 7.08 (ddd, 15H, *³J_{H-H} =* 8.4 Hz, *³J_{H-H}= 5.9 Hz, ⁴J_{H-H} =* 2.0 Hz, X10 - 14), 5.28 (s, 6H, H8), 3.51 (s, 6H, H2) ppm; **¹³C-NMR (75.48 MHz, CDCl₃)** *δ* = 144.0 (s, 3C, C9), 134.9 (s, 3C, C3), 129.2 (s, 6C, C11, C13), 128.8 (s, 3C, C12), 128.1 (s, 6C, C10, C14), 124.1 (s, 3C, C4), 54.3 (s, 3C, C8), 47.2 (s, 3C, C2) ppm.

### Synthesis of yellow 6-TramTO (42):

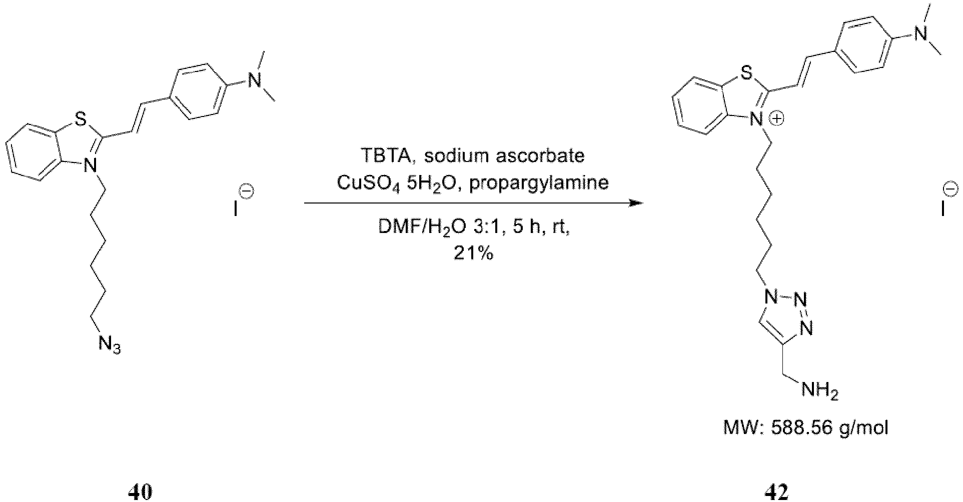

**Yellow 6-TramTO (42):** Under exclusion of light and stirring 6.9 mg CuSO₄ · 5 H₂O (28.0 µmol, 0.13 eq.) and 17.7 mg **54** (33.0 µmol, 0.15 eq.) were dissolved in 16.0 mL DMF/water (3:1). After 5 min 10 µL propargylamine (12.2 mg, 0.22 mmol, 1.00 eq.) was added. After further 5 min 119 mg of **40** (0.22 mmol, 1.00 eq.) was added to the green/blue solution. After further 5 min 11.0 mg sodium ascorbate (56.0 µmol, 0.25 eq.) was added. The dark red solution was stirred for 5 h at room temperature. The solvent was evaporated and the residue was dissolved in MeCN. The red solution was filtered over *Celite* and the solvent was evaporated. The crude product was purified by preparative HPLC (MeCN in H₂O (gradient: 15 - 35% over 40 min)). The different fractions were lyophilized to obtain 24.8 mg of product **42** (42.1 µmol, 21%) as a red solid. **R_{F}** = 0.60 (DCM/MeOH 10:1); **¹H-NMR** (500 MHz, DMSO-d₆) *δ* = 8.30 (dd, *³J_{H-H} =* 8.1 Hz, *⁴J_{H-H} =* 0.8 Hz, 1H, H3), 8.28 (s, 2H, H33), 8.10 (m, 3H, H1, H2, H3), 7.92 (d, *³J_{H-H} =* 9.0 Hz, 2H, H17, H21), 7.78 (m, 1H, H20), 7.68 (t, *³J_{H-H} =* 7.7 Hz, 1H, H18), 7.60 (d, *³J_{H-H} =* 15.2 Hz, H6), 6.85 (d, *³J_{H-H} =* 9.0 Hz, 2H, H22, H23), 4.79 (t, *³J_{H-H}* = 7.4 Hz, 2H, H10), 4.37 (t, *³J_{H-H}* = 7.1 Hz, 2H, H15), 4.10 (q, *³j_{H-H}* = 5.6 Hz, 2H. H32), 3.12 (s, 6H, H25, H26), 1.78 (dt, *³J_{H-H}* = 14.6 Hz. *³J_{H-H}* = 7.2 Hz, 4H, H11, H14), 1.46 (m, 2H, H12), 1.30 (m, 2H, H13) ppm; **¹³C-NMR** (125.76 MHz, DMSO-*d*₆) *δ* = 171.2 (s, 1C, C8), 153.6 (s, 1C, C19), 150.6 (s, 1C, C1), 141.2 (s, 1C, C4), 140.0 (s, 1C, C31), 133.0 (s, 1C, C2), 128.9 (s, 1C, C5), 127.5 (s, 1C, C18), 127.1 (s, 1C, C20), 124.1 (s, 2C, C17, C21), 124.0 (s, 1C, C16), 121.4 (s, 1C, C3), 115.9 (s, 1C, C30), 112.0 (s, 2C, C22, C23), 105.7 (s, 1C, C6), 49.3 (s, 1C, *C15),* 47.8 (s, 1C, C10), 40.0 (s. 2C, C25, C26), 33.9 (s, 1C, C32), 29.6 (s, 1C, C11), 28.2 (s, 1C, C14), 25.5 (s, 1C, C13). 25.2 (s, 1C, C12) ppm; **HRMS (ESI⁺)** m/z: calculated for C₂₆H₃₃N₆S: 461.2500; found: 461.2481.

### Synthesis of Mitochondria penetrating peptides (MPPs)

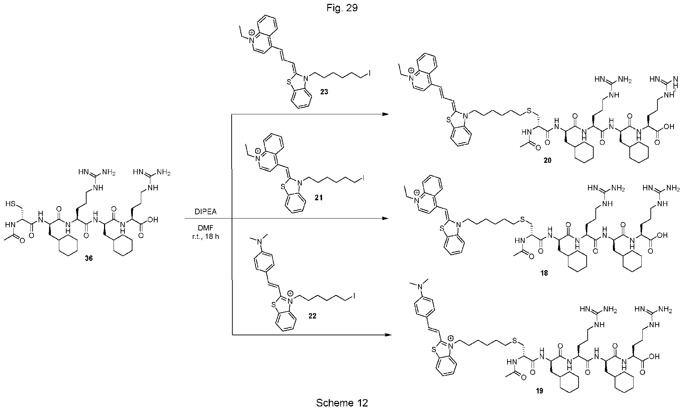

The following exemplary synthesis route is based on the nucleophilic attack of the cysteine in peptide **36** at the iodine substituted carbon atom of the alkyl chain of the dye-derivative **(21, 22, 23)** (cf. Fig. 29, scheme 12).

**Ac-Cys-Cha-dArg-Cha-dArg-OH (36):** For loading of the resin with the first amino acid, 2-chlorotrityl chloride resin (CTC resin, 50.0 mg, loading by supplier 0.50 mmol/g) is swollen in DMF for 30 min and a solution of the first amino acid (Fmoc-D-Arg(Pbf)-OH, 130 mg, 200 µmol 8.00 equiv.) and DIPEA (136 µL, 800 µmol, 32 equiv.) in NMP (286 µL) is added to the resin which is shaken at room temperature for 90 min. After the coupling, the resin is washed with DMF (5x) and CH₂Cl₂ (5x). A loading of *f* = 0.35 mmol/g is determined by UV/Vis spectroscopy (1.00 equiv. = 16.5 µmol). The resin is washed with DMF (5x) and capping is performed by adding 2,6-lutidine/Ac₂O/DMF (6:5:89, 500 µL) to the resin and shaking for 5 min. After washing with DMF (5x), CH₂Cl₂ (5x), DMF (5x), the first coupling cycle begins mainly analogous to the synthesis described for peptide **26.** Deprotecting of the Fmoc-groups is performed by adding piperidine (20% in DMF, 2 x 500 µL) and shaking for 2 x 5 min. The resin is washed with DMF (5x), CH₂Cl₂ (5x), DMF (5x) and the coupling solution consisting of the Fmoc-amino acid (66.0 µmol, 4.00 equiv.), Oxyma (9.4 mg, 66.0 µmol, 4.00 equiv.) and DIC (10 µL, 66.0 µmol, 4.00 equiv.) in DMF (200 µL) is added to the resin which is shaken at room temperature for 45 min. After the coupling, the resin is washed with DMF (5x), CH₂Cl₂ (5x), DMF (5x) and capped by adding 2,6-lutidine/Ac₂O/DMF (6:5:89, 500 µL) to the resin and shaking for 5 min. After washing with DMF (5x), CH₂Cl₂ (5x), DMF (5x), the next cycle begins. After the last coupling cycle, the final Fmoc group is deprotected as described above and the resin is washed with DMF (5x), CH₂Cl₂ (5x), DMF (5x) and capped as described above. After washing with DMF (5x), CH₂Cl₂ (5x), DMF (5x), the peptide is cleaved from the resin by adding 1.5 mL SH-cleavage mix (2.5% DODT, 2.5% H₂O, 1% TIPS, 94% TFA) and shaking for 2.5 h. Afterward, the resin is washed with cleavage mix (500 µL) and TFA (2 x 500 µL) and the combined solutions are drained into ice-cold Et₂O (~ 25 mL). After centrifugation, the precipitate is washed with cold Et₂O (2x) and the obtained pellet is dried *in vacuo.* After purification by preparative HPLC (Gilson, 15-75%), the desired peptide (**36**, 6.0 mg, 5.32 µmol, 32%) is obtained as a white solid. **R*ₜ*** = 19.1 min (gradient 1). **HRMS** (ESI): *m*/*z* calc. for C₃₅H₆₄N₁₁O₇S [M+H]⁺ 782.4708, found 782.4730. This synthesis of the MPP's is provided by way of example. Any person of ordinary skill in the art knows that the amino acid sequence of MPP's may be varied in order to adjust the penetrating ability of the MPP's. Thus all MPP's are comprised by the scope of the invention.

**Red MPP (20):** Peptide (**36**, 0.9 mg, 801 nmol, 1.00 equiv.) and lodo derivative **6-lodoTO-3 (14,** 1.5 mg, 2.24 µmol, 2.80 equiv.) are dissolved in DMF (100 µL), DIPEA (1.6 µL, 8.91 µmol, 11.1 equiv.) is added and the reaction mixture is stirred at room temperature for 18 h. Afterward, the solvent is removed via centrifugation *in vacuo* and the crude is purified by preparative HPLC (Varian, 35-95%) to yield the desired Red MPP (20, 44 nmol, 6%) as a blue solid. **R*ₜ*** = 23.4 min (gradient 1). **HRMS** (ESI): *m*/*z* calc. for C₆₂H₉₄N₁₃O₇S₂³⁺ [M+2H]³⁺ 398.89, found 399.42.

**Green MPP (18):** Peptide (**36**, 0.9 mg, 801 nmol, 1.00 equiv.) and lodo derivative **6-lodoTO-1 (5,** 1.0 mg, 1.56 µmol, 1.95 equiv.) are dissolved in DMF (100 µL), DIPEA (1.6 µL, 8.91 µmol, 11.1 equiv.) is added and the reaction mixture is stirred at room temperature for 18 h. Afterward, the solvent is removed via centrifugation *in vacuo* and the crude is purified by preparative HPLC (Varian, 35-95%) to yield the desired Green MPP (**18**, 116 nmol, 15%) as a red solid. **R*ₜ*** = 22.3 min (gradient 1). **HRMS** (ESI): *m*/*z* calc. for C₆₀H₉₀N₁₃O₇S₂⁺ [M]⁺ 1168.65, found 1168.69.

**Yellow MPP (19):** Peptide (**36,** 0.9 mg, 801 nmol, 1.00 equiv.) and lodo derivative **22** (1.0 mg, 1.62 µmol, 2.02 equiv.) are dissolved in DMF (100 µL), DIPEA (1.6 µL, 8.91 µmol, 11.1 equiv.) is added and the reaction mixture is stirred at room temperature for 18 h. Afterward, the solvent is removed via centrifugation *in vacuo* and the crude is purified by preparative HPLC (Varian, 35-95%) to yield the desired Yellow MPP (**19,** 44 nmol, 6%) as a red solid. **R*ₜ*** = 16.9 min (gradient 1). **HRMS** (ESI): *m*/*z* calc. for C₅₈H₉₀N₁₃O₇S₂⁺ [M]⁺ 1144.65, found 1144.69.

### List of abbreviations

Besides abbreviations, well known to any person of ordinary skill in the art, following table 3 lists some further abbreviations used herein.

**Table 3: Abbreviations used herein**

| | |
|---|---|
| Cha | L-cyclohexylalanine |
| dArg | D-arginine |
| MFI | mean fluorescence intensity |
| MOI | multiplicity of infection |
| MPP | mitochondria penetrating peptide |
| NT | nucleoli tracker |
| RFU | relative fluorescence untis |
| TBTA | tris((1-benzyl-4-triazolyl)methyl)amine |
| TO | thiazole orange |
| TAS | benzoThiazole AminoStyryl |

### Description of the drawings

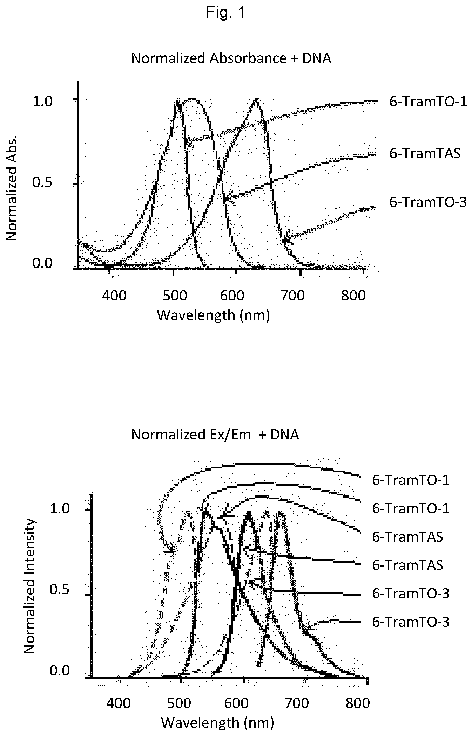

- Fig. 1:: Fig. 1 exemplarily shows Normalized spectral properties of TramTO dyes (**1-3**) in 10 mM Tris buffer, 50 mM KCl, pH= 7.6; DNA is dsDNA_{CT} double-stranded calf thymus DNA.
- Fig. 2:: Fig. 2 exemplarily shows bacterial growth curves at 2.5 µM and 5 µM dye concentration and with DMSO control (final DMSO concentration 0.4%). Mean of three measurements in triplicate.
- Fig. 3:: Fig. 3 exemplarily shows the viability of HeLa cells stained with cyanine dyes.
- Fig. 4:: Fig. 4 exemplarily shows the results of flow-cytometric measurements with *E. Coli* and *B. Subtilis.* Each experiment is labelled via subtitle, the subtitles indicating the following parameter settings (using equipment LSR Fortressa cell analyser):
"Blue B-A" indicates an excitation wavelength of 488 nm, emission filter of 575/25 nm;
"Blue Green A-A" indicates an excitation wavelength of 514 nm, emission filter of 610/20 nm;
"Yellowgreen A-A" indicates an excitation wavelength of 561 nm, leading to an emission filter 685/35 nm;
- Fig. 5A:: The graphs of Fig. 5A show that for 6-TramTO-1 (1) there is clear labelling when added 5 µM of the dye for only 5 min before the measurements, dead cells (treated with 70% isopropanol for 1h, then washed with saline 3x) are labelled about 10x stronger: MFI 135 vs 1337.
Additionally to the parameter settings regarding excitation/emission described in the legend to Fig. 4 the following parameter settings are used:
"Blue A-A" indicates an excitation wavelength of 488 nm, emission filter of 670/30 nm;
"Blue Green B-A" indicates an excitation wavelength of 514 nm, emission filter of 542/27 nm;
"Blue C-A" indicates an excitation wavelength of 488 nm, emission filter of 510/20 nm;
"Yellow Green B-A" indicates an excitation wavelength of 561 nm, emission filter of 632/22 nm.
- Fig. 5B:: The graphs of Fig. 5B show flow cytometric data of mixtures of dead and live E. Coli treated with left graph: PI (20 µM) and right graph: 6-TramTO-1 (1, 5 µM) for 5 min showing that the TramTO and PI give the same results.
The parameter settings regarding excitation/emission (as far as they are applied) are equivalent to those described within the legend of Fig. 5A.

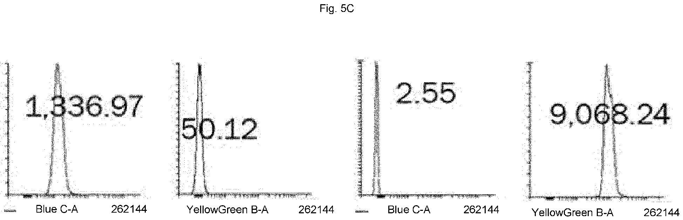

- Fig. 5C:: The graphs of Fig. 5C show dead bacteria treated with the two on the left 6-TramTO-1 (1) only and the two on the right PI only. (DMSO treated cells MFI: Blue C-A 4.26 and Yellow Green B-A 2.96). The value "262144" on the x-axis of each graph of Figs. 5B and 5C is always referring to the rightmost scale mark.
The parameter settings regarding excitation/emission (as far as they are applied) are equivalent to those described within the legend of Fig. 5A.
- Fig. 6:: Fig. 6 exemplarily shows data regarding comparatively staining mixtures of dead and live cells with PI and 6-TramTO-1 (**1**). Upper density plot with green (Blue C-A) and orange (Yellow Green B-A) labelling in the axis and lower plots: all cells are labelled (right plot) compared to DMSO control (left plot).
The parameter settings regarding excitation/emission (as far as they are applied) are equivalent to those described within the legend of Fig. 5A.
- Fig. 7:: Fig. 7 exemplarily shows experimental data proving that dual and even triple labelling is also possible with the TramTO dyes, using *Bacillus subtilis.* Suspensions of bacteria are labelled for 15 min with the different dyes at 5 µM, and are mixed in ratio 1:1 for dual labelling and in ratio 1:1:1 for the triple labelling experiments. (LSR Fortessa cell analyser). The value "262144" on the axes of each graph is always referring to the rightmost resp. uppermost scale mark.
The parameter settings regarding excitation/emission (as far as they are applied) are equivalent to those described within the legend of Fig. 5A. The dyes applied are denoted on top of each graph, e.g. "**1 + 2**".
- Fig. 8:: Fig. 8 shows further examples of labelling, using *Klebsiella pneumoniae.* There the same protocol is applied (15 min labelling at 5 µM dye concentration). BD FACS Aria Ill. 100,000 events per sample are analysed. Left signal in each graph: DMSO/background fluorescence control. Channels: Alexa Fluor 488 (excitation wavelength of 488 nm, emission filter of 530/30 nm), PE-Texas Red (excitation wavelength of 561 nm, emission filter of 610/20 nm) and APC (excitation wavelength of 633 nm, emission filter of 660/20 nm).
- Fig. 9A:: Fig. 9A shows the results of phagocytosis studies with different bacterial strains labelled with 1 or 3. Here the following channels were used (apparatus "Guava easyCyte"):
Green: excitation wavelength of 488 nm, emission filter of 525/30 nm;
Yellow: excitation wavelength of 488 nm, emission filter of 582/26 nm;
Red: excitation wavelength of 488 nm, emission filter of 611/319 nm;
Red2: excitation wavelength of 640 nm, emission filter of 680/30 nm.
- Fig. 9B:: Fig. 9B shows examples of fluorescence microscopy pictures of stained HeLa cells; left: "green" 6-TramTO-1 (**1**), center: "yellow" 6-TramTAS (**2**); right: "red" 6-TramTO-3 (**3**), each one showing stained nuclei of fixed HeLa cells. (3 µM, 20 min)
- Fig. 9C:: Fig. 9C shows further examples of fluorescence microscopy pictures of stained HeLa cells (dyes in complete live cells: PPh₃-TOs (**15-17**) in living HeLa cells (staining being done with dye concentration = 3 µM (in FluoroBrite media), duration of staining being 20 min, the cells being rinsed afterwards and the images being acquired after further 30 min)). left: "green" cyanine dye **15**, center: "yellow" cyanine dye **16**; right: "red" cyanine dye **17.**

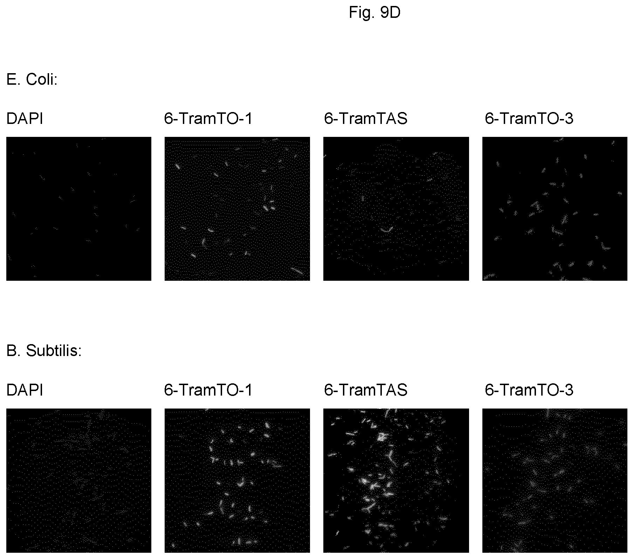

- Fig. 9D:: Fig. 9D shows exemplarily fluorescence microscopy pictures of bacterial labelling (5µM, 15 min for TramTO dyes, 1 µM for DAPI) using exemplarily *E. Coli* and B. *Subtilis.*
- Fig. 10:: Scheme 5 shows the general retrosynthetic approach for the preparation of different TO-derivatives (e.g. 6-xxxTO-1 and 6-xxxTO-3 derivatives, "xxx" denoting "Tram" or "lodo" or "Tri" or "PPh₃").
- Fig. 11:: Scheme 8 shows the synthesis route of 6-TramTO-1 (**1**) starting from benzothiazolium (**30**).

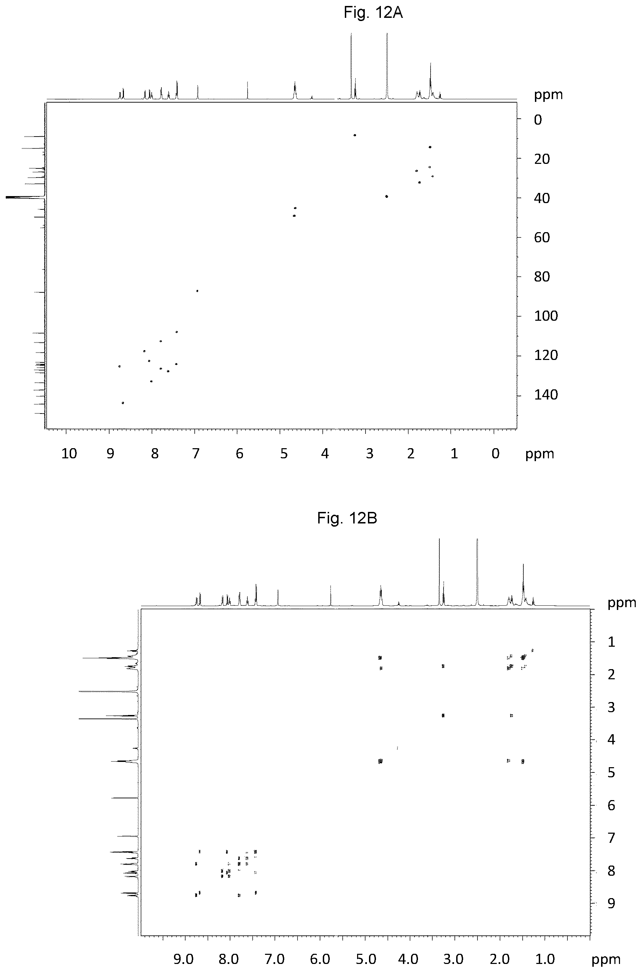

- Fig. 12A:: HSQC-NMR spectrum of 6-lodoTO-1 (**21**).
- Fig. 12B:: COSY-NMR spectrum of 6-lodoTO-1 (**21**).

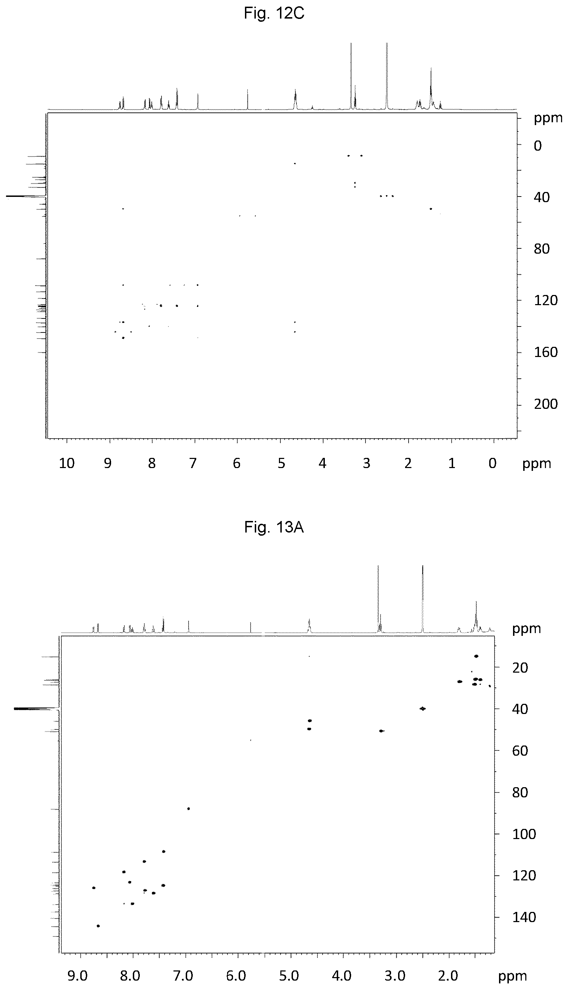

- Fig. 12C:: HMBC-NMR spectrum of 6-lodoTO-1 (**21**).
- Fig. 13A:: HSQC-NMR spectrum of **12.**

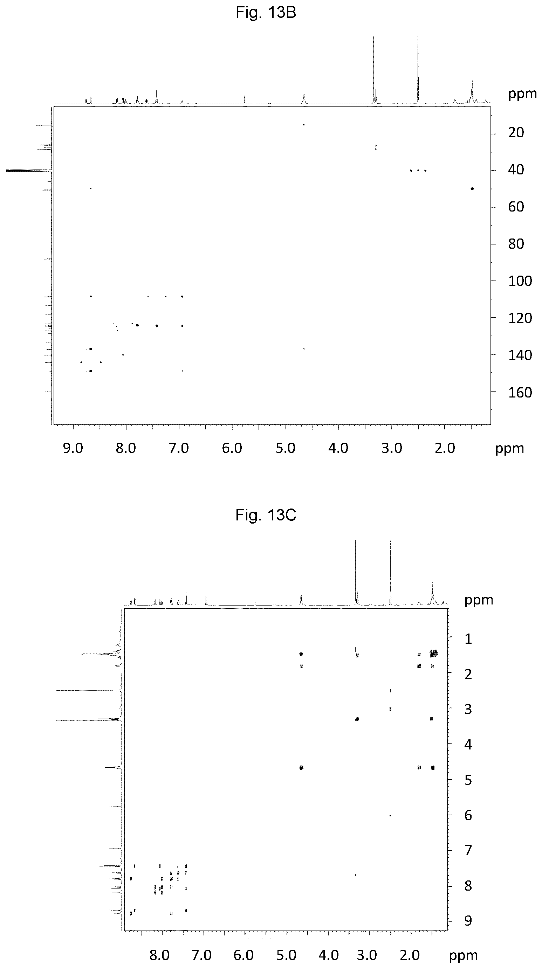

- Fig. 13B:: HMBC-NMR spectrum of **12.**
- Fig. 13C:: COSY-NMR spectrum of **12.**

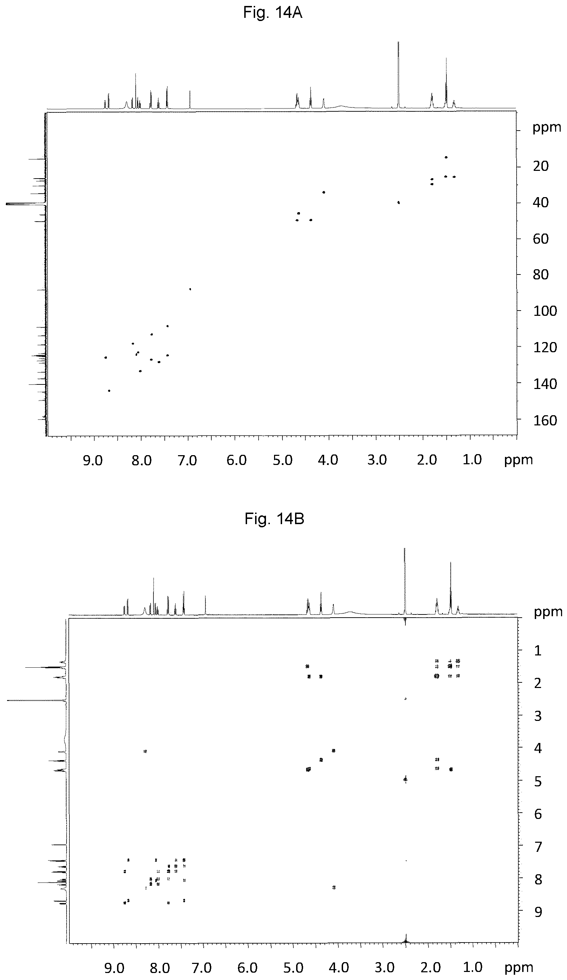

- Fig. 14A:: HSQC-NMR spectrum of 6-TramTO-1 (**1**).
- Fig. 14B:: COSY-NMR spectrum of 6-TramTO-1 (**1**).

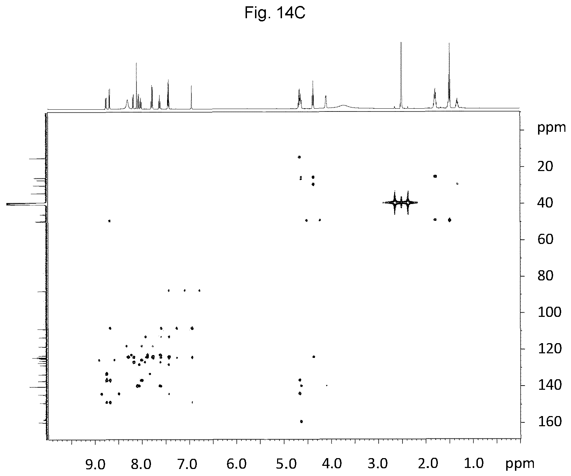

- Fig. 14C:: HMBC-NMR spectrum of 6-TramTO-1 (**1**).
- Fig. 15:: Scheme 9 shows the synthesis routes of 6-PipTO-1 (**14**), 6-PipTO-3 (**10**), 6-TriTO-1 (**13**), 6-PPh3TO-1 (**15**) and 6-PPh3TO-3 (**17**) starting from 6-IodoTO-1 (**21**) or 6-lodoTO-3 (**23**).

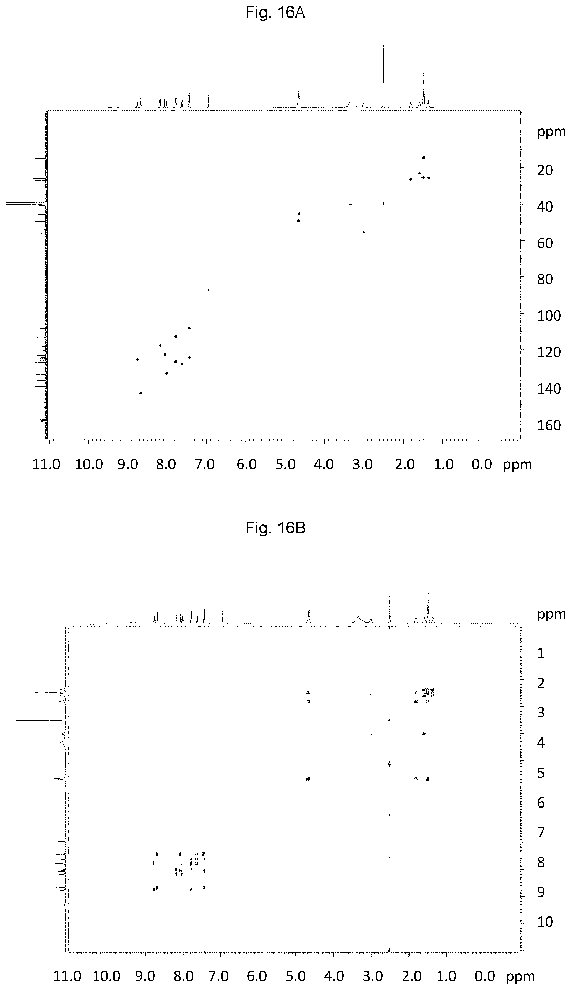

- Fig. 16A:: HSQC-NMR spectrum (500 MHz, 125 MHz) of 6-PipTO-1 (**14**).
- Fig. 16B:: COSY-NMR spectrum (300 MHz, 300 MHz) of 6-PipTO-1 (**14**).

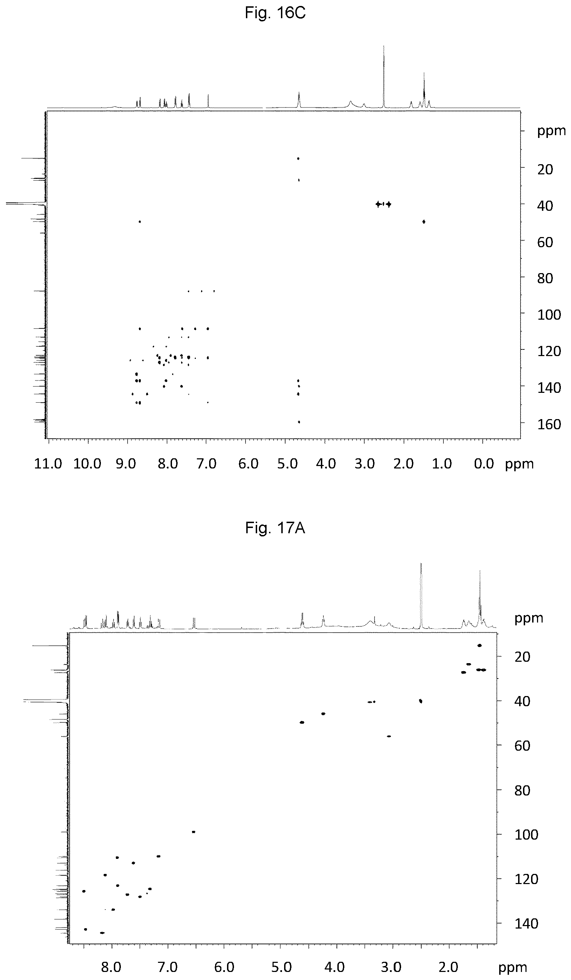

- Fig. 16C:: HMBC-NMR spectrum (500 MHz, 125 MHz) of 6-PipTO-1 (**14**).
- Fig. 17A:: HSQC-NMR spectrum of **10** in DMSO-d6.

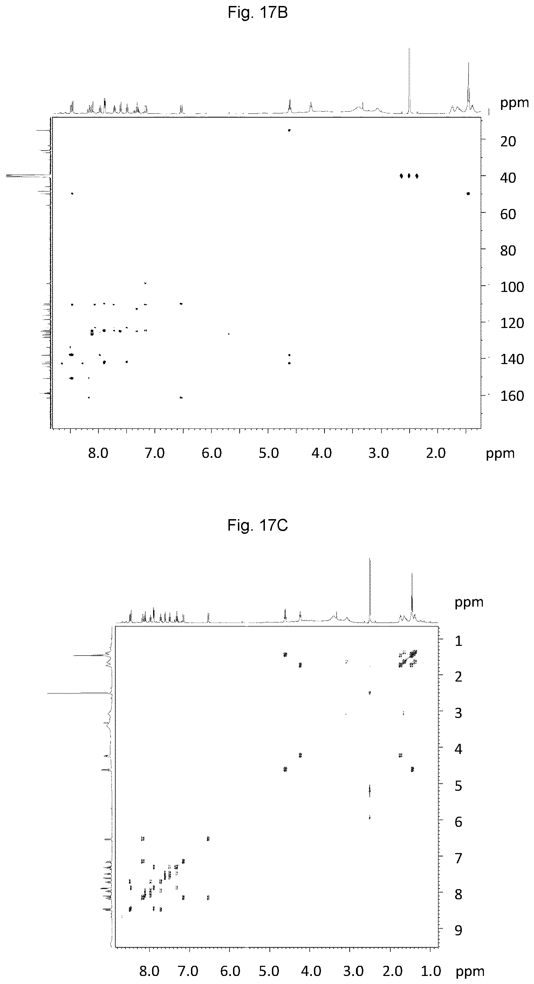

- Fig. 17B:: HMBC-NMR spectrum of **10** in DMSO-d6.
- Fig. 17C:: COSY-NMR spectrum of 10 in DMSO-d6.

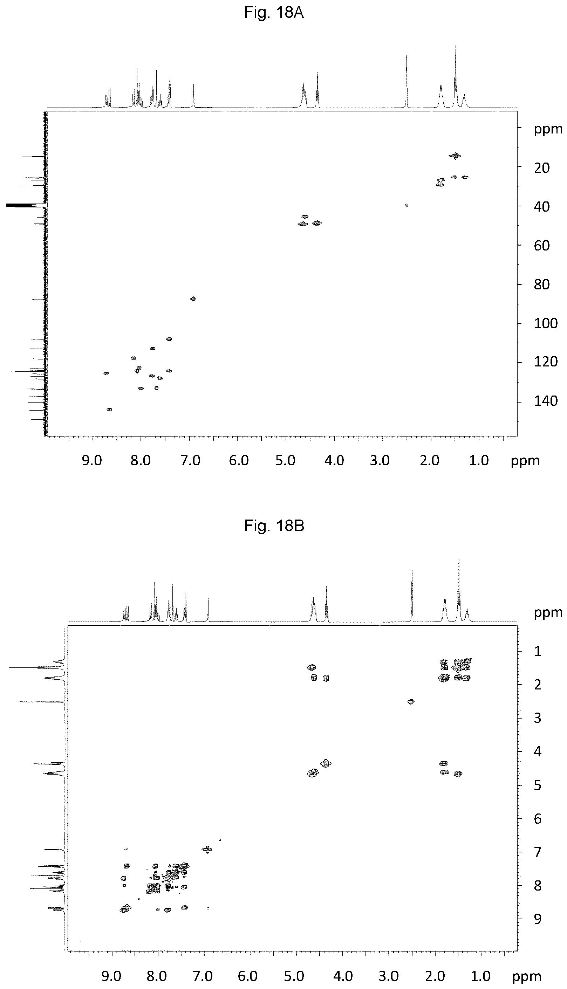

- Fig. 18A:: HSQC-NMR spectrum (300 MHz, 75 MHz) of 6-Tri(asym.)TO-1 (**13a**).
- Fig. 18B:: COSY-NMR spectrum (300 MHz, 300 MHz) of 6-Tri(asym.)TO-1 (**13a**).

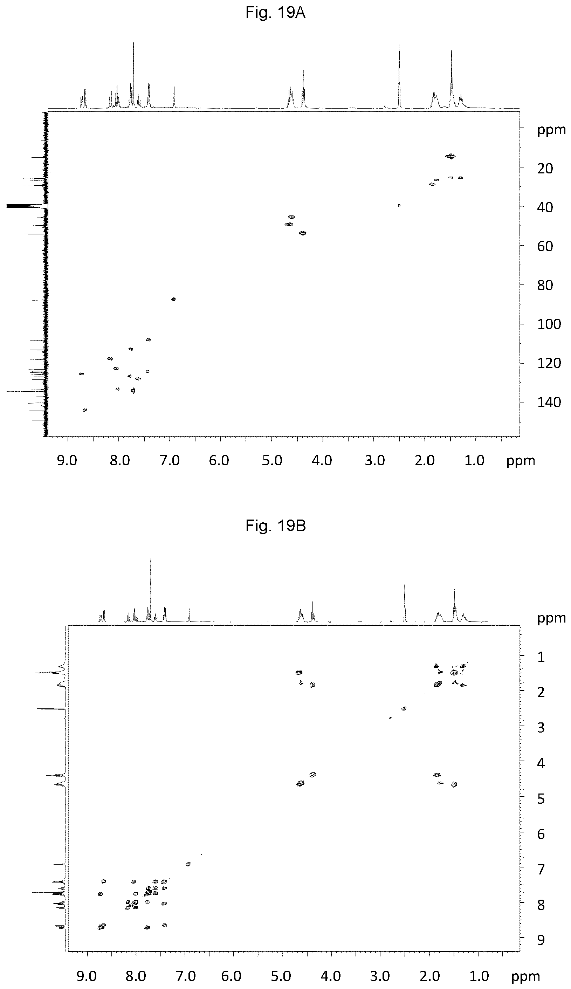

- Fig. 19A:: HSQC-NMR spectrum (300 MHz, 75 MHz) of 6-Tri(sym.)TO-1 (**13b**).
- Fig. 19B:: COSY-NMR spectrum (300 MHz, 300 MHz) of 6-Tri(sym.)TO-1 (**13b**).

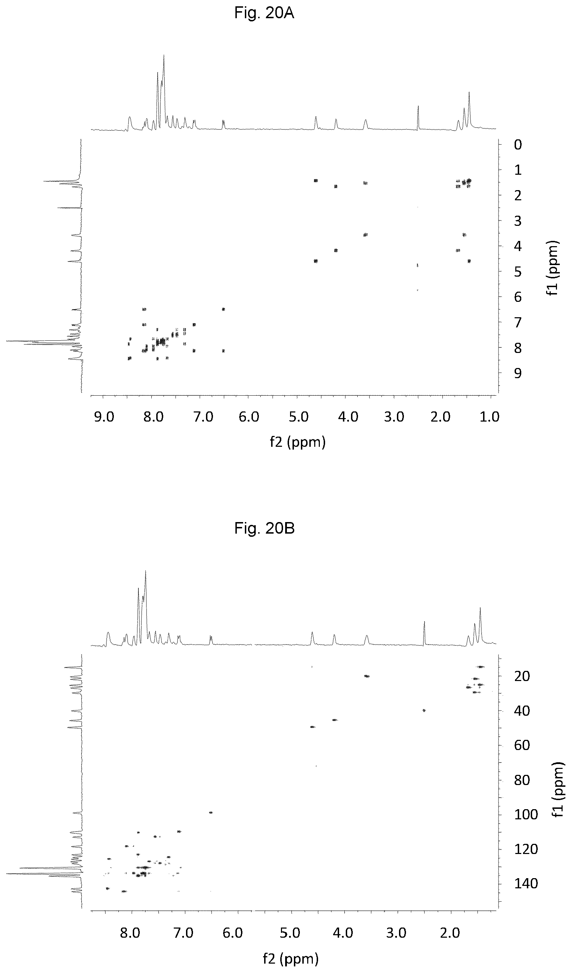

- Fig. 20A:: COSY-NMR spectrum of 6-PPh3TO-3 (17).
- Fig. 20B:: HSQC-NMR spectrum of 6-PPh3TO-3 (17).
- Fig. 20C:: HMBC-NMR spectrum of 6-PPh3TO-3 (17).
- Fig. 21:: Scheme 10 shows the synthesis route of dyes CH₃TO-1 (**4**) and CH₃TO-3 (**6**), starting from benzothiazole **37.**

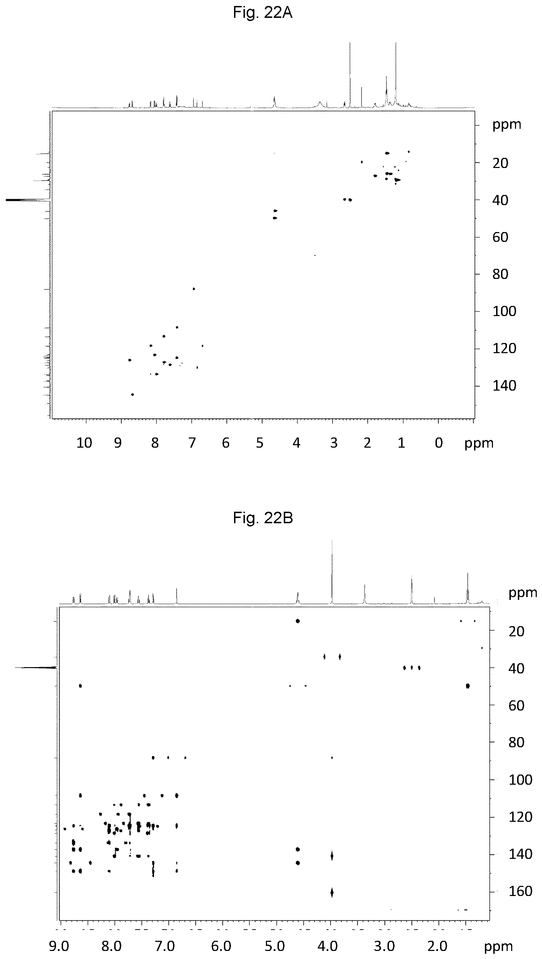

- Fig. 22A:: HSQC-NMR spectrum of **4** in DMSO-d₆.
- Fig. 22B:: HMBC-NMR spectrum of **4** in DMSO-d₆.

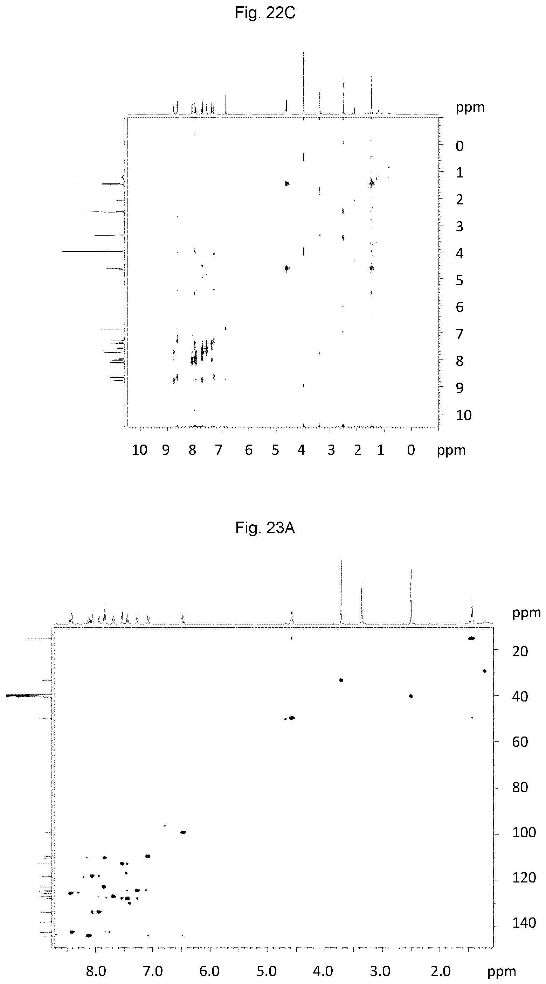

- Fig. 22C:: COSY-NMR spectrum of **4** in DMSO-d₆.
- Fig. 23A:: HSQC-NMR spectrum of **6** in DMSO-d₆.

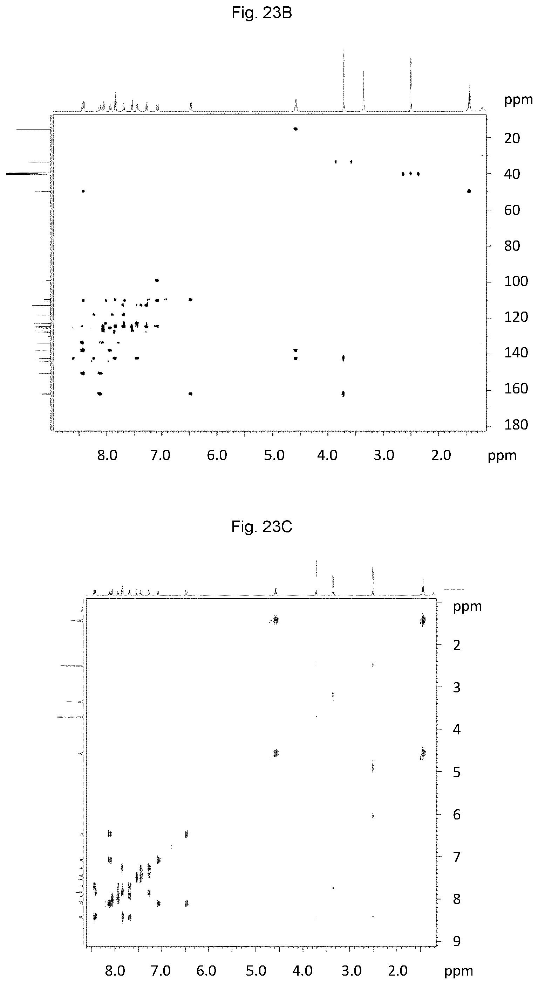

- Fig. 23B:: HMBC-NMR spectrum of **6** in DMSO-d₆.
- Fig. 23C:: COSY-NMR spectrum of **6** in DMSO-d₆.
- Fig. 24:: Scheme 11 shows the Synthesis route of "yellow" 6-TramTAS (**2**) starting from benzothiazole (**30**).

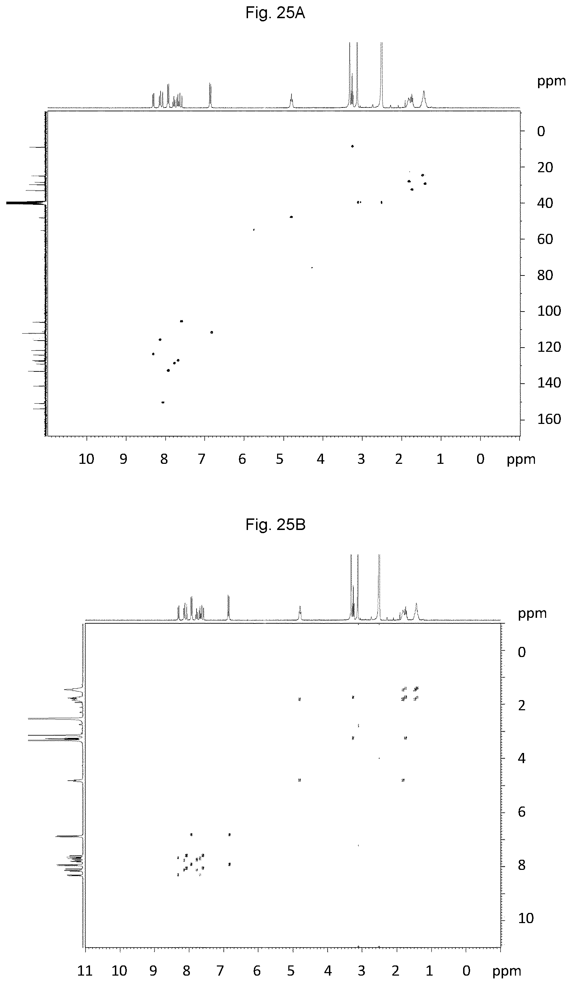

- Fig. 25A:: HSQC-NMR spectrum of 6-lodoTAS (**22**).
- Fig. 25B:: COSY-NMR spectrum of 6-lodoTAS (**22**).

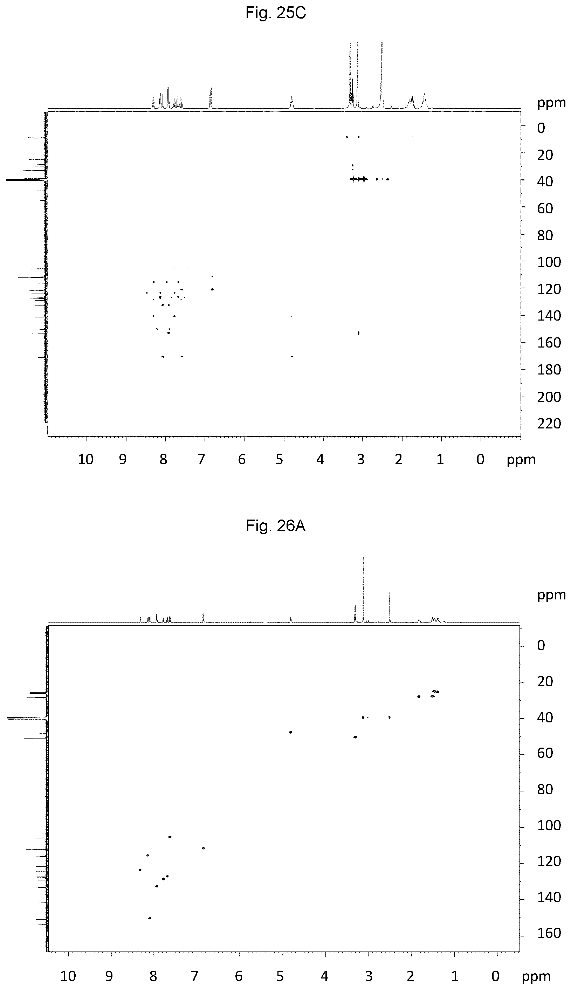

- Fig. 25C:: HMBC-NMR spectrum of 6-lodoTAS (**22**).
- Fig. 26A:: HSQC-NMR spectrum of 6-azindoTAS (**34**).

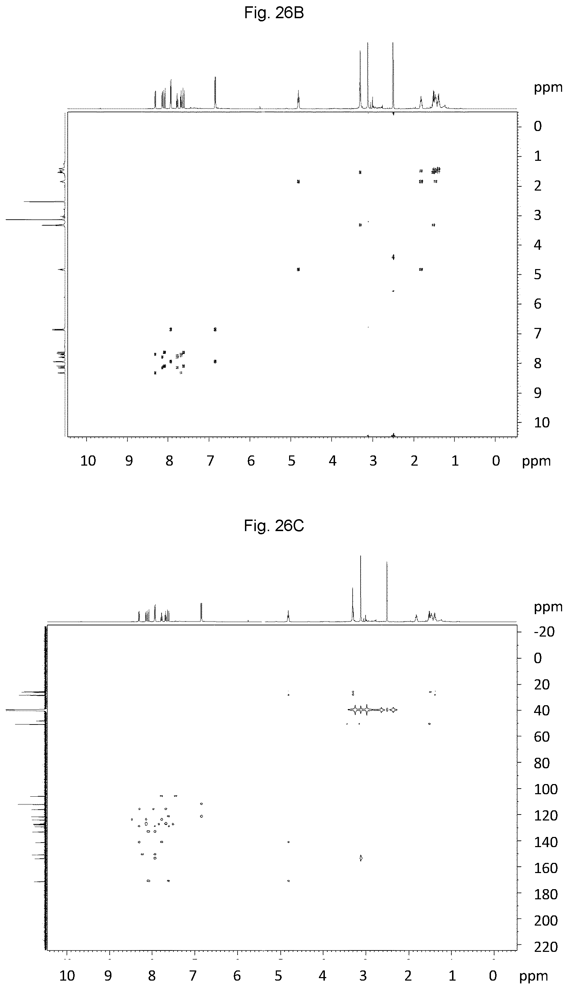

- Fig. 26B:: COSY-NMR spectrum of 6-azindoTAS (**34**).
- Fig. 26C:: HMBC-NMR spectrum of 6-azindoTAS (**34**).

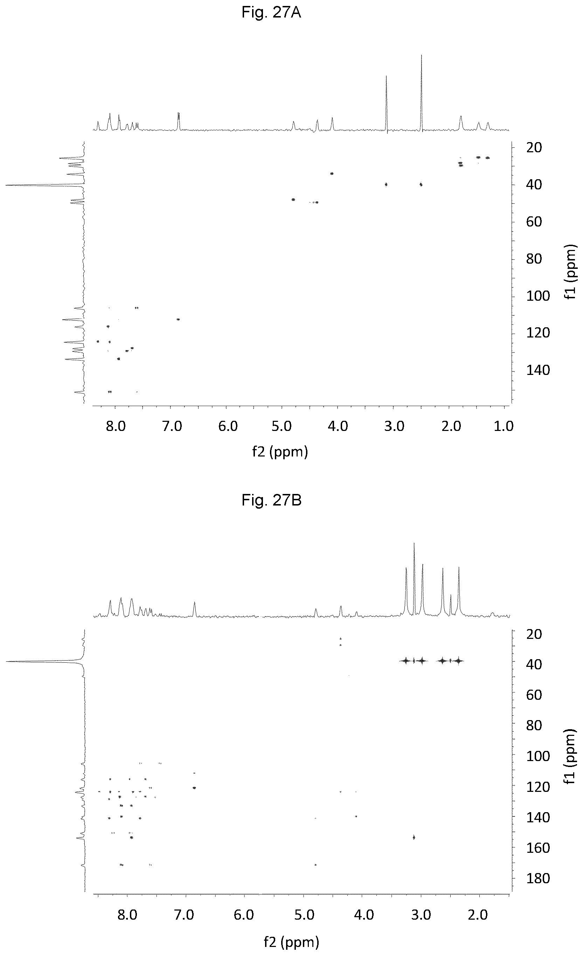

- Fig. 27A:: HSQC-NMR spectrum of **2.**
- Fig. 27B:: HMBC-NMR spectrum of **2.**

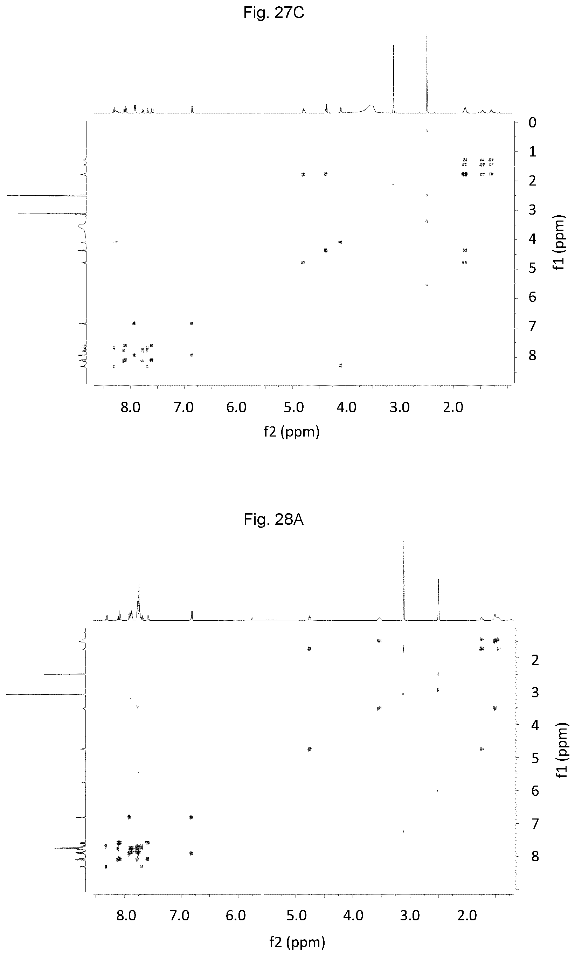

- Fig. 27C:: COSY-NMR spectrum of **2.**
- Fig. 28A:: COSY-NMR spectrum of **16.**

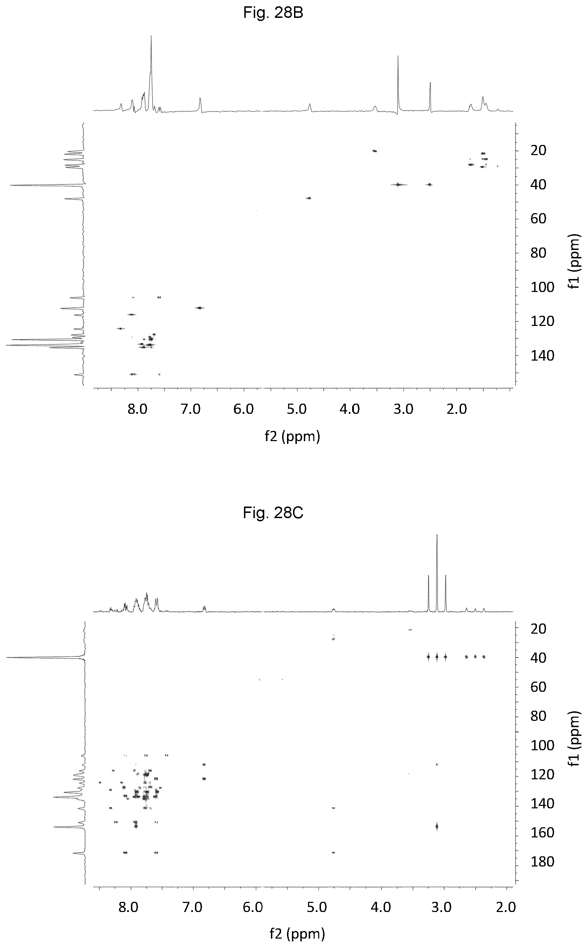

- Fig. 28B:: HSQC-NMR spectrum of **16.**
- Fig. 28C:: HMBC-NMR spectrum of **16.**
- Fig. 29:: Scheme 12 shows the synthesis route of Red- (**20**), Green- (**18**) and Yellow-MPP (**19**) starting from Ac-Cys-Cha-dArg-Cha-dArg-OH (**36**).

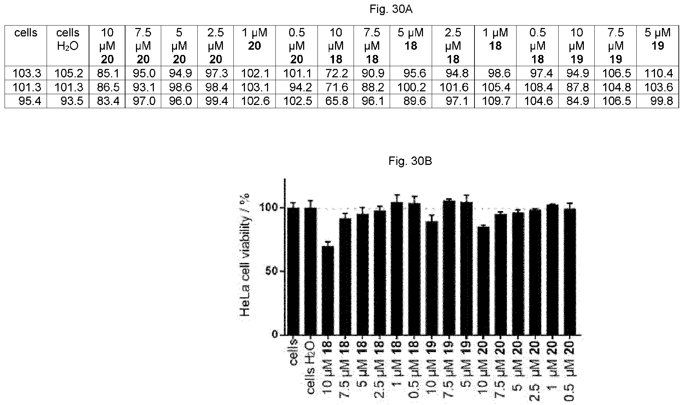

- Fig. 30A:: Table showing experimentally measured values of viability of HeLa cells (acquired via resazurin-based assay) under different growth conditions: pure and in H₂O (both serving as possible reference values) and under different dye concentrations (examples: dyes 18, 19 and 20).
- Fig. 30B:: Graphical depictions of experimental results of viability-testing, exemplarily obtained by use of compounds 18, 19 and 20 (graphical depictions of data presented in Fig. 30A).

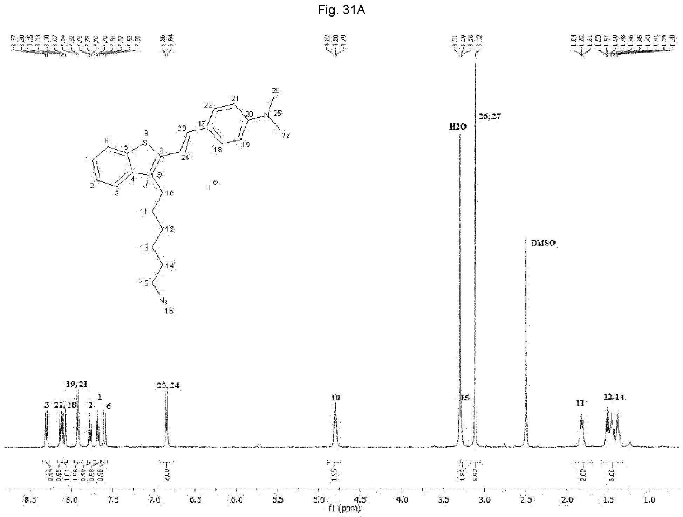

- Fig. 31A:: ***¹H-NMR spectrum of 40.***

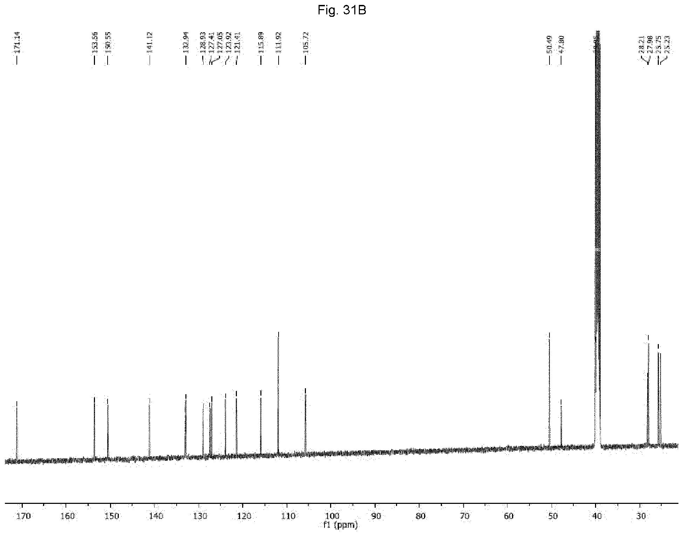

- Fig. 31B:: ***¹³C-NMR spectrum of 40.***

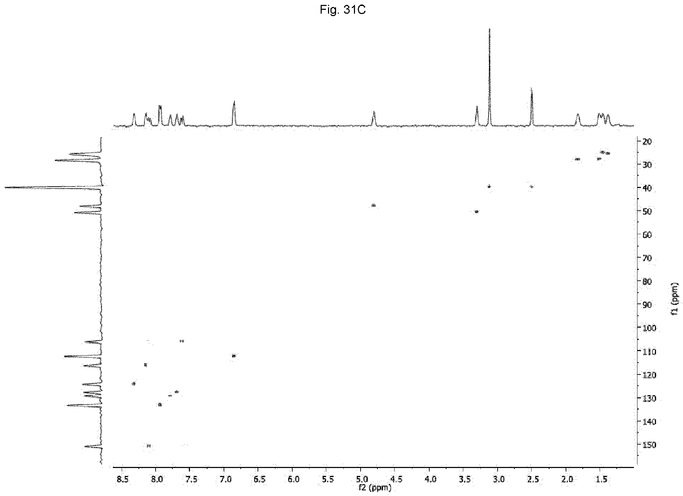

- Fig. 31C:: ***HSQC-NMR spectra of 40.***

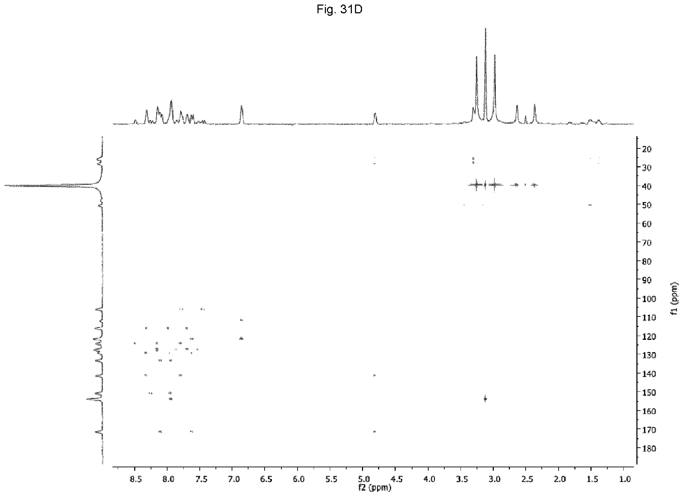

- Fig. 31D:: ***HMBC-NMR spectra of 40.***

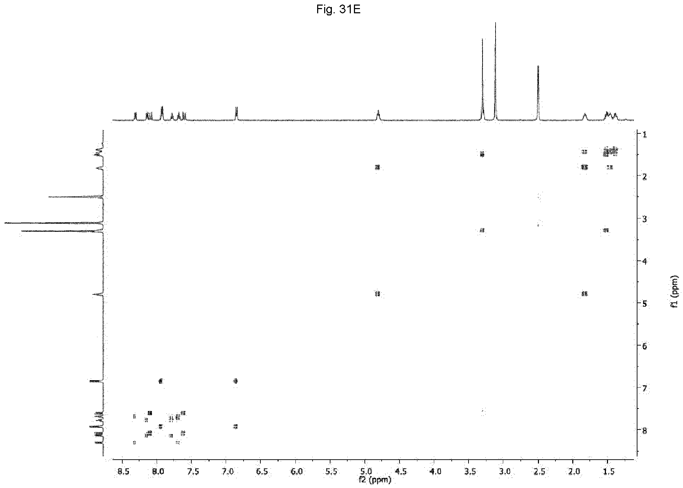

- Fig. 31E:: ***COSY-NMR spectro of 40.***

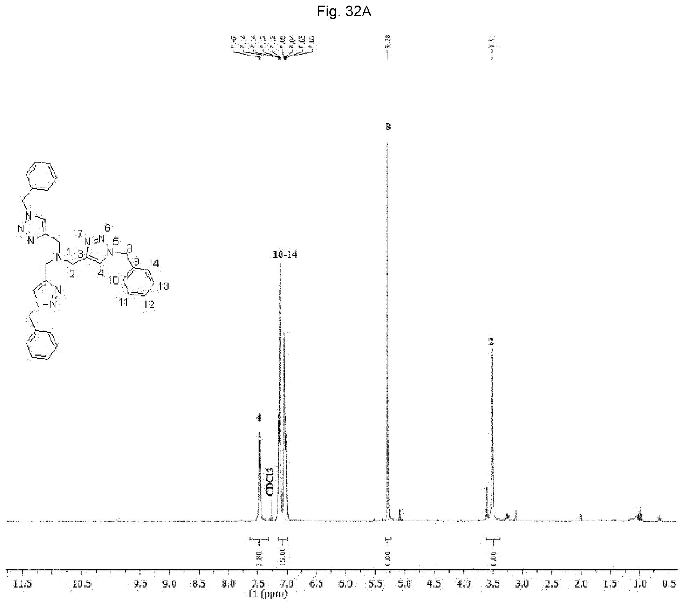

- Fig. 32A:: ***¹H-NMR spectrum of TBTA (54).***

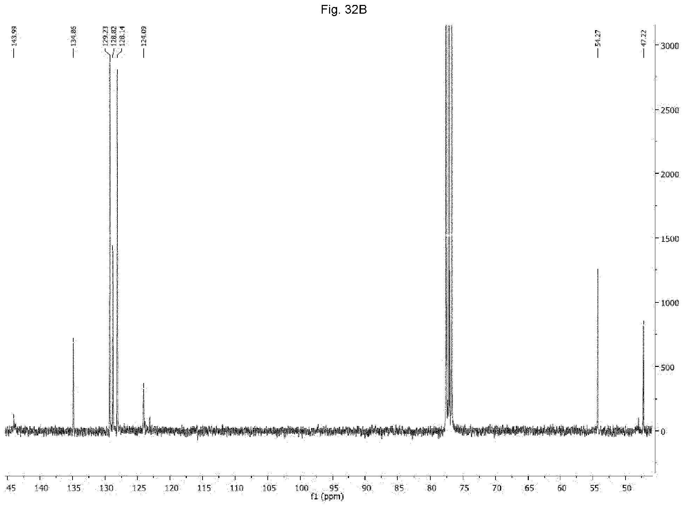

- Fig. 32B:: ***¹³C-NMR spectrum of TBTA (54).***

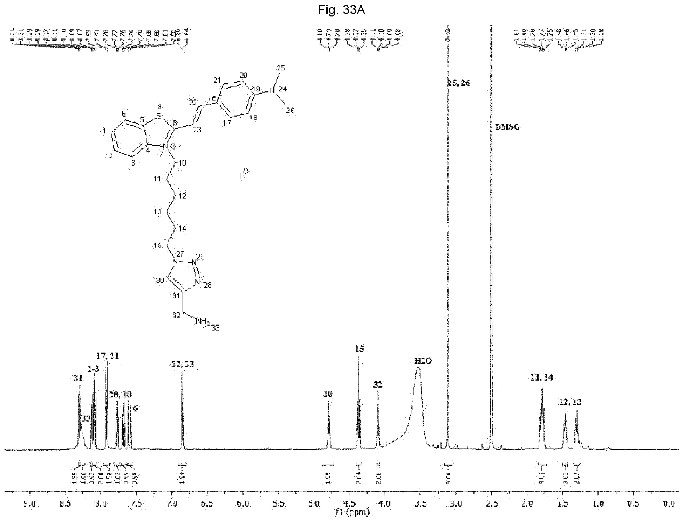

- Fig. 33A:: ***¹H-NMR spectrum of 42.***

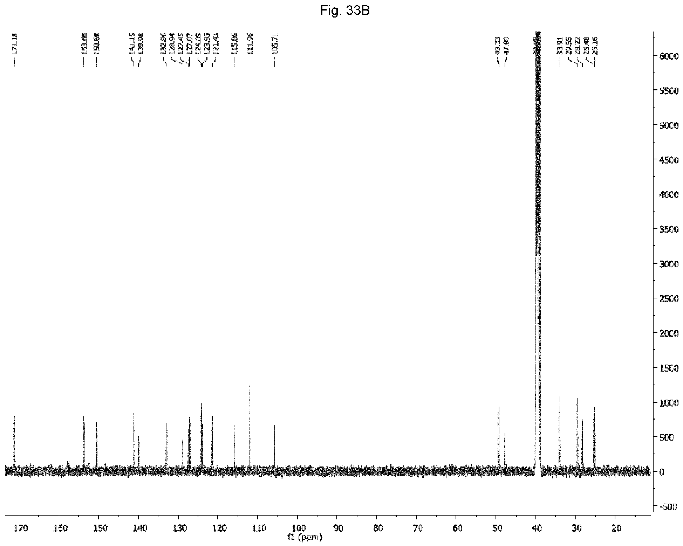

- Fig. 33B:: ***¹³C-NMR spectrum of 42.***

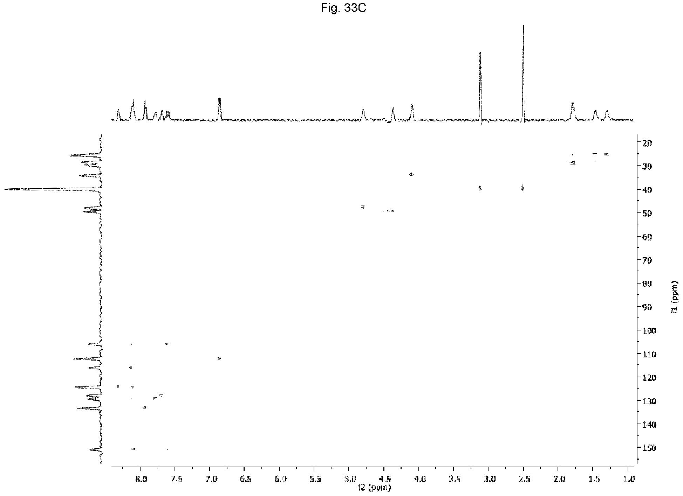

- Fig. 33C:: ***HSQC-NMR spectra of 42.***

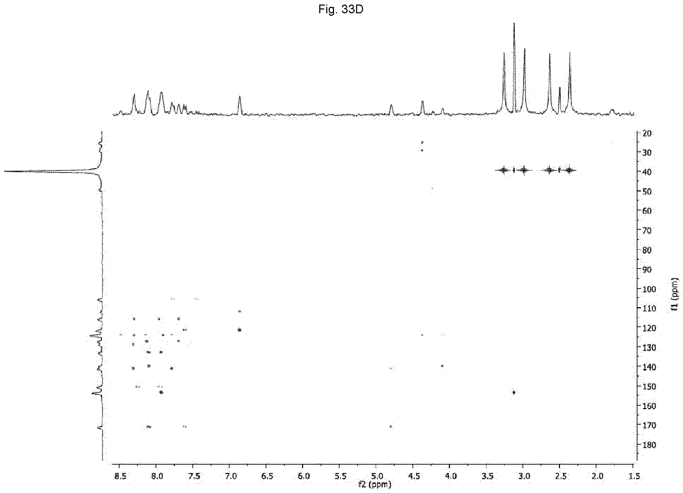

- Fig. 33D:: ***HMBC-NMR spectra of 42.***

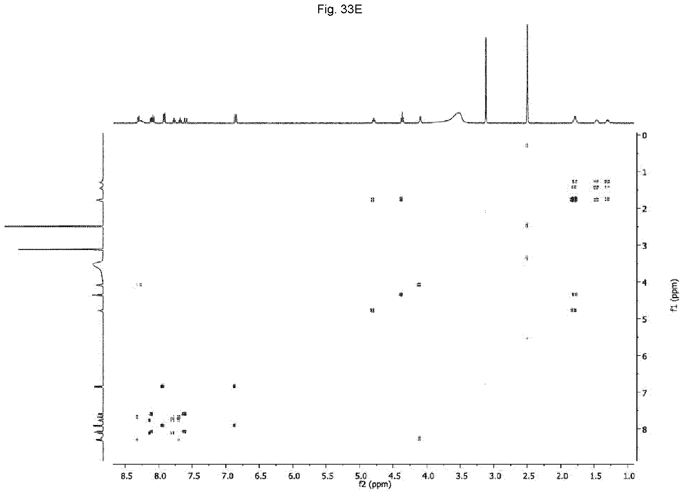

- Fig. 33E:: ***COSY-NMR spectra of 42.***

## Claims

1. A cyanine dye for staining living cells having the general formula (**I**), 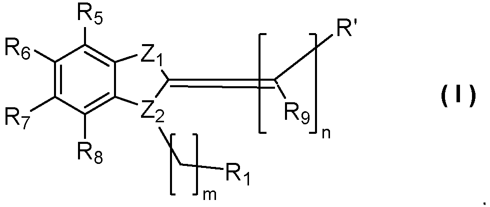 wherein
- R' is a moiety according to formula (**II**) or a moiety according to formula (**III**), 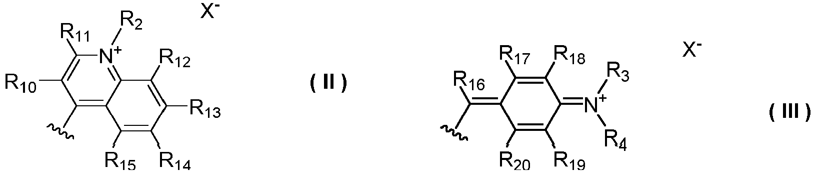
- R₂ is **selected** from the list comprising H, methyl, ethyl, propyl, iso-propyl, butyl, iso-butyl, tert-butyl, alkyl, alkinyl, alkylidene insofar as substituent R₂ is present according to the choice of R',
- R₃ and R₄ are independently from each other selected from the list comprising H, methyl, ethyl, propyl, iso-propyl, butyl, iso-butyl, tert-butyl, alkyl, alkinyl, alkylidene insofar as substituents R₃, R₄ are present according to the choice of R',
- R₅ to R₂₀ are independently from each other selected from the list comprising the substituents H, halogen, methyl. ethyl, propyl, isopropyl, methoxy, ethoxy, propoxy, amino, methylamino, ethylamino, monoalkylamino, dialkylamino, a nitro group, a sulfo group insofar as any one of substituents R₅ to R₂₀ is present according to the choice of R',
- Z₁ is S and Z₂ is N,
- X⁻ is selected from the list comprising halogenate, sulfate, triflate, trifluoro acetate, difluoro acetate or fluoro acetate,
- n is 1 to 3,
- m is 6,
**characterized by**
- R₁ is independently chosen from the list comprising the substituents triphenylphosphine, substituted triphenylphosphine, 1,2,3-triazol-1-yl, 1,2,3-triazol-2-yl,
mitochondria penetrating peptide - whereat the mitochondria penetrating peptide is chemically bound to the alkyl-chain of length m via peptide-bond or via sulfide-bond or via ester-bond or via thioester-bond -
if R' is chosen to be a moiety according to formula (**II**);
or
- R₁ is independently chosen from the list comprising the substituents OH, SH, N₃, NH₂, NH₃⁺, NHR₃, NH₂R₃⁺, NR₂₁R₂₂, NR₂₁R₂₂H⁺ - whereat R₂₁ and R₂₂ are independently from each other chosen from the list comprising methyl, ethyl or propyl -, substituted or non-substituted heterocyclic structure, substituted or non-substituted cyclic structure, triphenylphosphine,
substituted triphenylphosphine, 1,2,3-triazol-1-yl, 1,2,3-triazol-2-yl,
substituted 1,2,3-triazol-1-yl, substituted 1,2,3-triazol-2-yl,
mitochondria penetrating peptide - whereat the mitochondria penetrating peptide is chemically bound to the alkyl-chain of length m via peptide-bond or via sulfide-bond or via ester-bond or via thioester-bond -
if R' is chosen to be a moiety according to formula (**III**).

2. A cyanine dye according to claim 1, **characterized in that** the mitochondria penetrating peptide has the amino acid sequence H₂N-Cys-Cha-dArg-Cha-dArg-COOH according to SEQ ID 1 or the amino acid sequence according to SEQ ID 2 or the amino acid sequence according to SEQ ID 3 or the amino acid sequence according to SEQ ID 4, wherein each mitochondria penetrating peptide is acetylated at the N-terminus.

3. A cyanine dye according to claim 1, **characterized in that** it has the chemical structure according to formula ( A ), 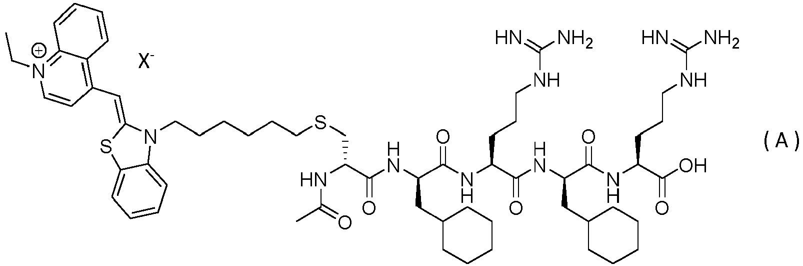 or according to formula ( B ) 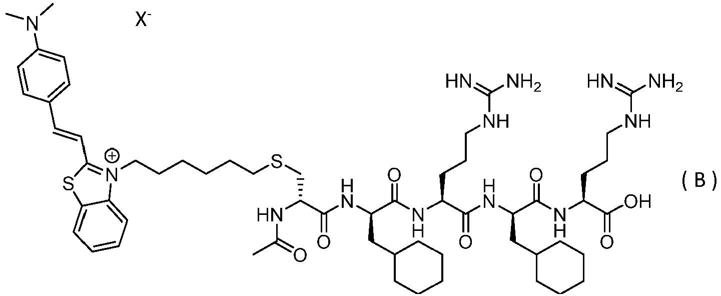 or according to formular ( C ),

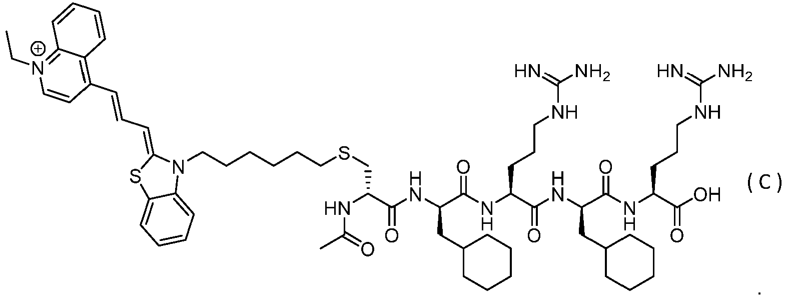

4. A cyanine dye according to one of the preceeding claims 1 to 3 in its protonated or deprotonated form, wherein the one or more counterions, of the protonated or deprotonated form are independently selected from the list comprising halogenate, sulfate, triflate, trifluoro acetate, difluoro acetate or fluoro acetate.

5. A cyanine dye according to any one of the preceeding claims 1 to 4, wherein the dye is adapted to bind to oligomeric DNA or primer-DNA or DNA-aptameres.

6. A method for staining living cells **characterized in that** the method comprises the following steps:
i) at least one cyanine dye as defined in claims 1 - 5_is applied to at least one sample of cells by adding a
solution of the at least one cyanine dye to the at least one sample of cells, thus providing at least one sample of cells, being stained with at least one cyanine dye;
ii) incubating the at least one sample of cells according to step i) for at least 15 minutes, so that stained living cells are existent.

7. A method for staining living cells according to claim 6, **characterized in that** the method comprises the following steps i) and ii) and the additional step iii):
i) at least two cyanine dyes are applied to at least two separate samples of cells, wherein each sample is stained with one of the at least two cyanine dyes by adding a solution of the cyanine dye to the sample of cells, thus providing at least two separate samples of cells, each one being stained with at least one cyanine dye;
ii) each sample of cells according to modified step i) is incubated for at least 15 minutes, so that stained living cells are existent;
iii) the at least two separate samples of cells provided according to step ii),
each one being stained with at least one cyanine dye, are combined within one sample vessel with each other thus forming one combined sample.

8. A method according to claim 6 or claim 7, **characterized in that** the cyanine dye is a nucleic acid binding cyanine dye.

9. A method according to claim 6 or claim 7 or claim 8, **characterized in that** the method comprises the following additional preparation step before step i):
a) obtaining and isolating at least one sample of cells to be introduced into step i),
wherein this obtaining and isolation-step includes one or more steps for concentrating and/or breeding of the cells.

10. A kit for carrying out the method according to any of claims 6-9, the kit comprising the cyanine dye and optionally supportive substances.

11. _The synthesizing method of a cyanine dye according to any one of the preceding claims 1 through 5, **characterized in that** the synthesizing method comprises the following steps:
a) performing a substitution reaction on a precursor compound according to formula (**IV**), 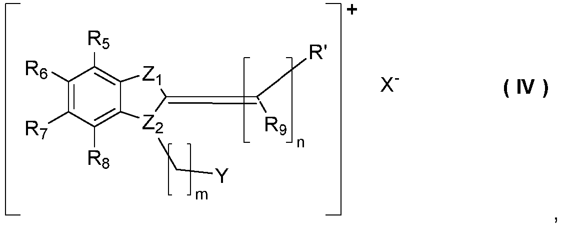 whereat
the substituent Y in formula (**IV**) is selected from the list comprising the substituents chlorine, bromine, iodine, mesylate, tosylate, fluorinated mesylate, fluorinated tosylate and all other substituents in formula (**IV**) have the same meaning and are chosen in the same way as is denoted in claim 1 regarding formula (**I**) and indices m and n have the same values as are denoted in claim 1 regarding formula (**I**),
by reacting the precursor compound according to formula (**IV**) in a reaction mixture with a nucleophilic compound, leading to the substitution of substituent Y by the nucleophilic compound;
b) extracting the reaction product from the reaction mixture from step a) and
c) purifying the reaction product retrieved from step b).

12. Method according to claim 11 **characterized in that** the nucleophilic compound in step a) is triphenylphosphine or 1,2,3-triazole or a mitochondria penetrating peptide.

13. Method according to claim 12 **characterized in that** the nucleophilic compound in step a) is the azide ion and the reaction product from step a) or the extracted reaction product from step b) or the purified reaction product from step c) is further reacted in an additional step d), the additional step d) being inserted between steps a) and b) or between steps b) and c) or being placed after step c).

14. Method according to claim 13, **characterized in that** the additional step d) comprises the reaction of a precursor compound according to formula (**V**), 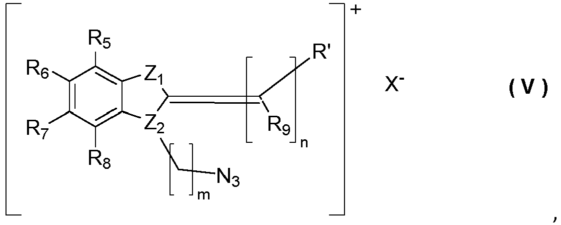
whereat in this case R' is restricted to a moiety according to formula (**III**),
with substituted or unsubstituted Tris((1-benzyl-4-triazolyl)methyl)amine, "TBTA",
thus forming a cyanine dye according to any one of the preceeding claims 1 through 5 having a substructure where R' is a moiety according to formula (**III**) and R₁ is a substituent with a substituted or non-substituted heterocyclic structure.

15. Method according to claim 13, **characterized in that** the additional step d) comprises the reaction of a precursor compound according to formula (V), 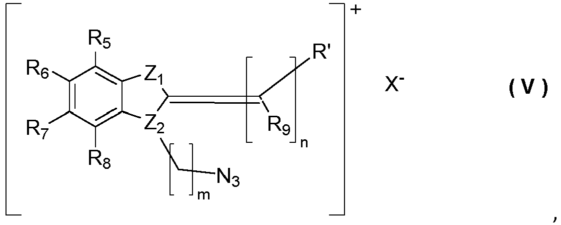
whereat R' is a moiety according to formula (**II**) or according to formula (**III**), whereat n is 1 or n is 2 or n is 3,
in a copper-catalyzed azide-alkyne cycloaddition reaction with a substituted or unsubstituted alkyne, thus forming a cyanine dye according to any one of the preceeding claims 1 through 5, whereat R₁ is a substituent with a substituted or non-substituted heterocyclic structure.

## Patentansprüche

1. Cyaninfarbstoff zum Färben lebender Zellen mit der allgemeinen Formel (I), 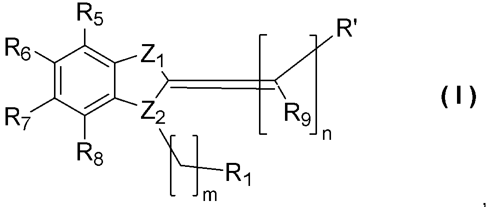 wobei
- R' eine Gruppe gemäß Formel (**II**) oder eine Gruppe gemäß Formel (**III**) ist, 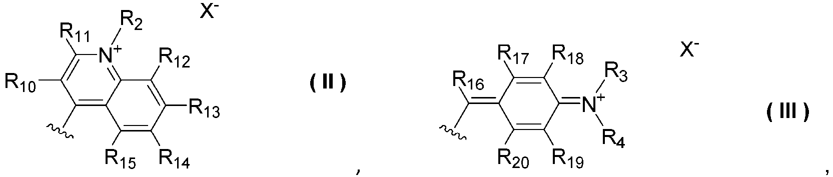
- R₂ ausgewählt ist aus der Liste umfassend H, Methyl, Ethyl, Propyl, Isopropyl, Butyl, Isobutenyl, tert-Butyl, Alkyl, Alkinyl, Alkyliden, sofern der Substituent R₂ gemäß der Wahl von R' vorhanden ist,
- R₃ und R₄ unabhängig voneinander ausgewählt sind aus der Liste umfassend H, Methyl, Ethyl, Propyl, Isopropyl, Butyl, Isobuthyl, tert-Butyl, Alkyl, Alkinyl, Alkyliden, sofern die Substituenten R₃, R₄ entsprechend der Wahl von R' vorhanden sind,
- R₅ bis R₂₀ unabhängig voneinander ausgewählt sind aus der Liste umfassend die Substituenten H, Halogen, Methyl, Ethyl, Propyl, Isopropyl, Methoxy, Ethoxy, Propoxy, Amino, Methylamino, Ethylamino, Monoalkylamino, Dialkylamino, einer Nitrogruppe, einer Sulfogruppe, sofern einer der Substituenten R₅ bis R₂₀ gemäß der Wahl von R' vorhanden ist,
- Z₁ S ist und Z₂ N ist,
- X⁻ aus der Liste ausgewählt ist, umfassend Halogenat, Sulfat, Triflat, Trifluoracetat, Difluoracetat oder Fluoracetat,
- n Werte von 1 bis 3 aufweist,
- m den Wert 6 hat,
**dadurch gekennzeichnet, daß**
- R₁ unabhängig ausgewählt ist, aus der Liste umfassend die Substituenten Triphenylphosphin, substituiertes Triphenylphosphin, 1,2,3-Triazol-1-yl, 1,2,3-Triazol-2-yl, Mitochondrien-penetrierendes Peptid -, wobei das Mitochondrien-penetrierende Peptid chemisch an die Alkylkette der Länge m über eine Peptidbindung oder über eine Sulfidbindung oder über eine Esterbindung oder über eine Thioesterbindung gebunden ist - , wenn Rest R' gemäß Formel (**II**) ausgewählt ist;
oder
- R₁ unabhängig ausgewählt ist aus der Liste umfassend die Substituenten OH, SH, N₃, NH₂, NH₃⁺, NHR₃, NH₂R₃⁺, NR₂₁R₂₂, NR₂₁R₂₂H⁺ - wobei R₂₁ und R₂₂ unabhängig voneinander aus der Liste ausgewählt sind, umfassend Methyl, Ethyl oder Propyl -, substituierte oder nicht substituierte heterocyclische Struktur, substituierte oder nicht substituierte cyclische Struktur, Triphenylphosphin, substituiertes Triphenylphosphin, 1,2,3-Triazol-1-yl, 1,2,3-Triazol-2-yl, substituiertes 1,2,3-Triazol-1-yl,
substituiertes 1,2,3-Triazol-2-yl,
mitochondrienpenetrierendes Peptid - wobei das mitochondrienpenetrierende Peptid chemisch über eine Peptidbindung oder über eine Sulfidbindung oder
über eine Esterbindung oder über eine Thioesterbindung an die Alkylkette der Länge m gebunden ist -, wenn Rest R' gemäß Formel (**III**) ausgewählt ist.

2. Cyaninfarbstoff gemäß Anspruch 1, **dadurch gekennzeichnet, dass** das Mitochondrien-penetrierende Peptid die Aminosäuresequenz H₂N-Cys -Cha-dArg-Cha-dArg-COOH gemäß SEQ ID 1 oder die Aminosäuresequenz gemäß SEQ ID 2 oder die Aminosäuresequenz gemäß SEQ ID 3 oder die Aminosäuresequenz gemäß SEQ ID 4 aufweist, wobei jedes Mitochondrien-penetrierende Peptid am N-Terminus acetyliert ist.

3. Cyaninfarbstoff gemäß Anspruch 1, **dadurch gekennzeichnet, dass** er die chemische Struktur gemäß Formel (A), 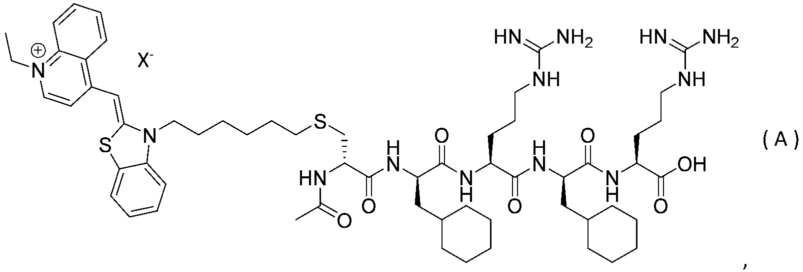 oder gemäß Formel (B), 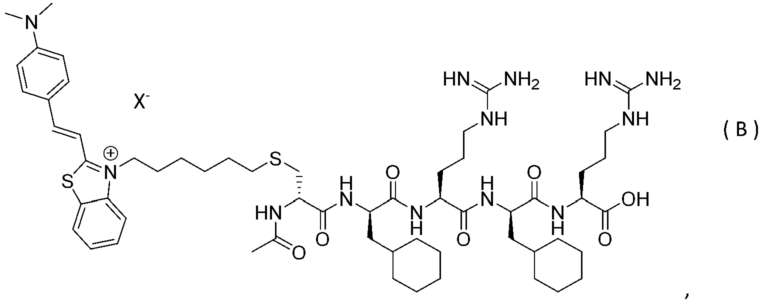 oder gemäß Formel (C), 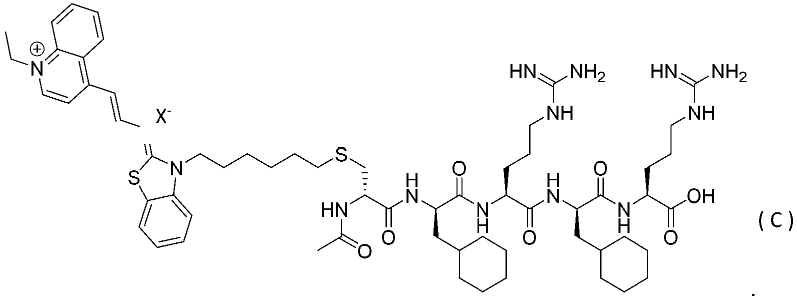 aufweist.

4. Cyaninfarbstoff gemäß einem der vorstehenden Ansprüche 1 bis 3 in seiner protonierten oder deprotonierten Form, wobei das Gegenion bzw. die Gegenionen der protonierten oder deprotonierten Form unabhängig voneinander aus der Liste ausgewählt sind, umfassend Halogenat, Sulfat, Triflat, Trifluoracetat, Difluoracetat oder Fluoracetat.

5. Cyaninfarbstoff gemäß einem der vorstehenden Ansprüche 1 bis 4, wobei der Farbstoff dazu angepasst ist, an oligomere DNA oder Primer-DNA oder DNA-Aptamere zu binden.

6. Verfahren zum Färben lebender Zellen, **dadurch gekennzeichnet, dass** das Verfahren die folgenden Schritte umfasst:
i) Aufbringung mindestens eines Cyaninfarbstoffs gemäß einem der Ansprüche 1 bis 5 auf mindestens eine Zellprobe, indem eine Lösung des mindestens einen Cyaninfarbstoffs zu der mindestens einen Zellprobe gegeben wird, wodurch mindestens eine Zellprobe bereitgestellt wird, die mit mindestens einem Cyaninfarbstoff gefärbt ist;
ii) Inkubation der mindestens einen Zellprobe gemäß Schritt i) für mindestens 15 Minuten, sodass gefärbte lebende Zellen vorhanden sind.

7. Verfahren zum Färben lebender Zellen gemäß Anspruch 6, **dadurch gekennzeichnet, dass** das Verfahren die folgenden Schritte i) und ii) und den zusätzlichen Schritt iii) umfasst:
i) mindestens zwei Cyaninfarbstoffe werden auf mindestens zwei getrennte Zellproben aufgebracht, wobei jede Probe mit einem der mindestens zwei Cyaninfarbstoffe gefärbt wird, indem eine Lösung des Cyaninfarbstoffs zu der Zellprobe hinzugefügt wird, wodurch mindestens zwei getrennte Zellproben bereitgestellt werden, von denen jede mit mindestens einem Cyaninfarbstoff gefärbt ist;
ii) jede Zellprobe gemäß dem modifizierten Schritt i) wird mindestens 15 Minuten lang inkubiert, sodass gefärbte lebende Zellen vorhanden sind;
iii) die mindestens zwei getrennten Zellproben, die gemäß Schritt ii) bereitgestellt wurden und von denen jede mit mindestens einem Cyaninfarbstoff gefärbt ist, werden in einem Probengefäß miteinander kombiniert, wodurch eine kombinierte Probe gebildet wird.

8. Verfahren nach Anspruch 6 oder Anspruch 7, **dadurch gekennzeichnet, dass** der Cyaninfarbstoff ein nukleinsäurebindender Cyaninfarbstoff ist.

9. Verfahren nach Anspruch 6 oder Anspruch 7 oder Anspruch 8, **dadurch gekennzeichnet, dass** das Verfahren vor Schritt i) den folgenden zusätzlichen Vorbereitungsschritt umfasst:
a) Gewinnen und Isolieren mindestens einer Probe von Zellen, die in Schritt i) eingeführt werden sollen, wobei dieser Gewinnungs- und Isolierungsschritt einen oder mehrere Schritte zum Aufkonzentrieren und/oder Züchten der Zellen umfasst.

10. Ein Kit zur Durchführung des Verfahrens gemäß einem der Ansprüche 6 bis 9, wobei das Kit den Cyaninfarbstoff und gegebenenfalls unterstützende Substanzen umfasst.

11. Verfahren zur Synthese eines Cyaninfarbstoffs gemäß einem der vorstehenden Ansprüche 1 bis 5, **dadurch gekennzeichnet, dass** das Syntheseverfahren die folgenden Schritte umfasst:
a) Durchführen einer Substitutionsreaktion an einer Vorläuferverbindung gemäß Formel (**IV**), 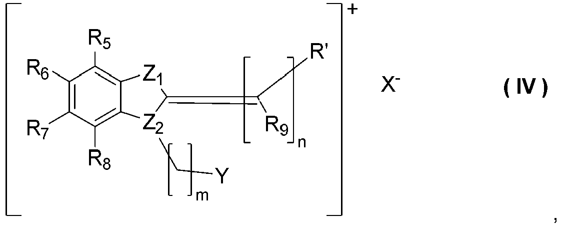 wobei
der Substituent Y in Formel (**IV**) ausgewählt ist aus der Liste umfassend die Substituenten Chlor, Brom, Iod, Mesylat, Tosylat, fluoriertes Mesylat,
fluoriertes Tosylat und alle anderen Substituenten in Formel (**IV**) die gleiche Bedeutung haben und auf die gleiche Weise ausgewählt werden, wie in Anspruch 1 bezüglich Formel (**I**) angegeben, und die Indizes m und n die gleichen Werte haben, wie in Anspruch 1 bezüglich Formel (**I**) angegeben,
durch Umsetzen der Vorläuferverbindung gemäß Formel (**IV**) in einem Reaktionsgemisch mit einer nukleophilen Verbindung, was zur Substitution des Substituenten Y durch die nukleophile Verbindung führt;
b) Extrahieren des Reaktionsproduktes aus dem Reaktionsgemisch aus Schritt a) und
c) Reinigen des aus Schritt b) gewonnenen Reaktionsproduktes.

12. Verfahren gemäß Anspruch 11, **dadurch gekennzeichnet, dass** die nukleophile Verbindung in Schritt a) Triphenylphosphin oder 1,2,3-Triazol oder ein mitochondrienpenetrierendes Peptid ist.

13. Verfahren gemäß Anspruch 12, **dadurch gekennzeichnet, dass** die nukleophile Verbindung in Schritt a) das Azidion ist und das Reaktionsprodukt aus Schritt a) oder das extrahierte Reaktionsprodukt aus Schritt b) oder das gereinigte Reaktionsprodukt aus Schritt c) in einem zusätzlichen Schritt d) weiter umgesetzt wird, wobei der zusätzliche Schritt d) zwischen die Schritte a) und b) oder zwischen die Schritte b) und c) eingefügt wird oder nach Schritt c) vorgenommen wird.

14. Verfahren gemäß Anspruch 13, **dadurch gekennzeichnet, dass** der zusätzliche Schritt d) die Umsetzung einer Vorläuferverbindung gemäß Formel (**V**) umfasst, 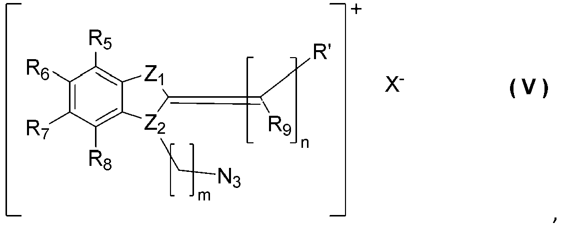 wobei in diesem Fall R' auf einen Rest gemäß Formel (**III**) beschränkt ist, mit substituiertem oder unsubstituiertem Tris((1-benzyl-4-triazolyl)methyl)amin, "TBTA", wodurch ein Cyaninfarbstoff gemäß einem der vorstehenden Ansprüche 1 bis 5 mit einer Unterstruktur gebildet wird, bei der R' eine Gruppe gemäß Formel (**III**) ist und R₁ ein Substituent ist, der eine substituierte oder nicht substituierte heterocyclische Struktur aufweist.

15. Verfahren gemäß Anspruch 13, **dadurch gekennzeichnet, dass** der zusätzliche Schritt d) die Umsetzung einer Vorläuferverbindung gemäß Formel (**V**) umfasst, 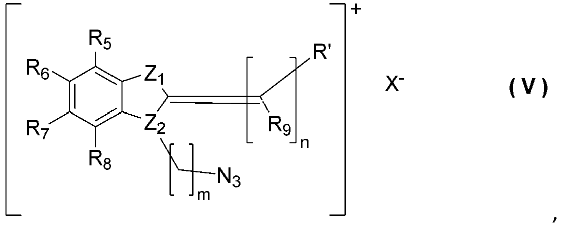 wobei R' eine Gruppe gemäß Formel (**II**) oder gemäß Formel (**III**) ist, wobei n gleich 1 ist oder n gleich 2 ist oder n gleich 3 ist, in einer kupferkatalysierten Azid-Alkin-Cycloadditionsreaktion mit einem substituierten oder unsubstituierten Alkin, wodurch ein Cyaninfarbstoff gemäß einem der vorstehenden Ansprüche 1 bis 5 gebildet wird, wobei R₁ ein Substituent ist, der eine substituierte oder nicht-substituierte heterocyclische Struktur aufweist.

## Revendications

1. Colorant cyanine pour la coloration de cellules vivantes, répondant à la formule générale (**I**), 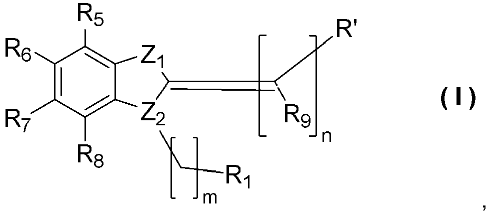 dans laquelle
- R' représente un groupement répondant à la formule (**II**) ou un groupement répondant à la formule (**III**), 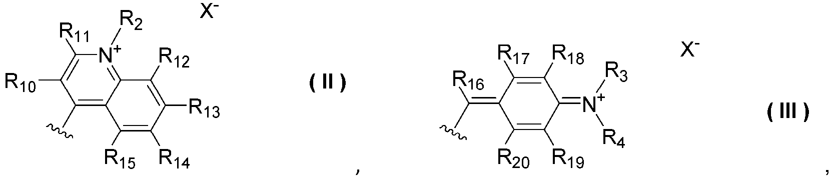
- R₂ est choisi parmi la liste comprenant H, méthyle, éthyle, propyle, isopropyle, butyle, isobutyle, tert-butyle, alkyle, alcynyle, alkylidène dans la mesure où le substituant R₂ est présent selon le choix de R',
- R₃ et R₄ sont choisis indépendamment l'un de l'autre parmi la liste comprenant H, méthyle, éthyle, propyle, isopropyle, butyle, isobutyle, tert-butyle, alkyle, alcynyle, alkylidène dans la mesure où les substituants R₃, R₄ sont présents selon le choix de R',
- R₅ à R₂₀ sont choisis indépendamment les uns des autres parmi la liste comprenant les substituants H, halogène, méthyle, éthyle, propyle, isopropyle, méthoxy, éthoxy, propoxy, amino, méthylamino, éthylamino, monoalkylamino, dialkylamino,
un groupe nitro, un groupe sulfo dans la mesure où l'un quelconque des substituants R₅ à R₂₀ est présent selon le choix de R',
- Z₁ est S et Z₂ est N,
- X⁻ est choisi parmi la liste comprenant halogénate, sulfate, triflate, trifluoro acétate, difluoro acétate ou fluoro acétate,
- n est 1 à 3,
- m est 6,
**caractérisé par**
- R₁ est choisi indépendamment dans la liste comprenant les substituants triphénylphosphine, triphénylphosphine substituée, 1,2,3-triazol-1-yle, 1,2,3-triazol-2-yle,
peptide pénétrant dans les mitochondries - où le peptide pénétrant dans les mitochondries est chimiquement lié à la chaîne alkyle de longueur m via une liaison peptidique ou via une liaison sulfure ou via une liaison ester ou via une liaison thioester - si R' est choisi pour être un fragment selon la formule (**II**);
où
- R1 est choisi indépendamment dans la liste comprenant les substituants OH, SH, N₃, NH₂, NH₃⁺, NHR₃, NH₂R₃⁺, NR₂₁R₂₂, NR₂₁R₂₂H⁺ - où R₂₁ et R₂₂ sont choisis indépendamment l'un de l'autre parmi la liste comprenant méthyle, éthyle ou propyle -, structure hétérocyclique substituée ou non substituée, structure cyclique substituée ou non substituée, triphénylphosphine, triphénylphosphine substituée, 1,2,3-triazol-1-yle, 1,2,3-triazol-2-yle, 1,2,3-triazol-1-yle substitué, 1,2,3-triazol-2-yle substitué,
peptide pénétrant dans les mitochondries - où le peptide pénétrant dans les mitochondries est chimiquement lié à la chaîne alkyle de longueur m via une liaison peptidique ou via une liaison sulfure ou via une liaison ester ou via une liaison thioester - si R' est choisi pour être un fragment selon la formule (**III**).

2. Colorant cyanine selon la revendication 1, **caractérisé en ce que** le peptide pénétrant dans les mitochondries a la séquence d'acides aminés H₂N-Cys -Cha-dArg-Cha-dArg-COOH selon SEQ ID 1 ou la séquence d'acides aminés selon SEQ ID 2 ou la séquence d'acides aminés selon SEQ ID 3 ou la séquence d'acides aminés selon SEQ ID 4, dans lequel chaque peptide pénétrant dans les mitochondries est acétylé à l'extrémité N-terminale.

3. Colorant cyanine selon la revendication 1, **caractérisé en ce qu'**il a la structure chimique selon la formule ( A ), 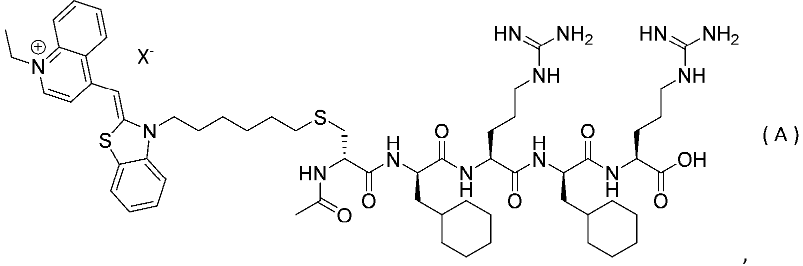 ou selon la formule ( B ), 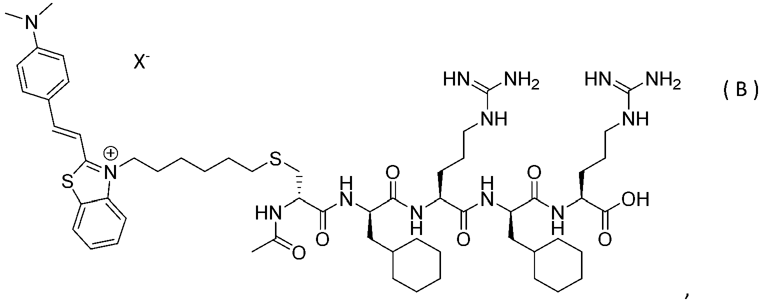 ou selon la formule ( C ),

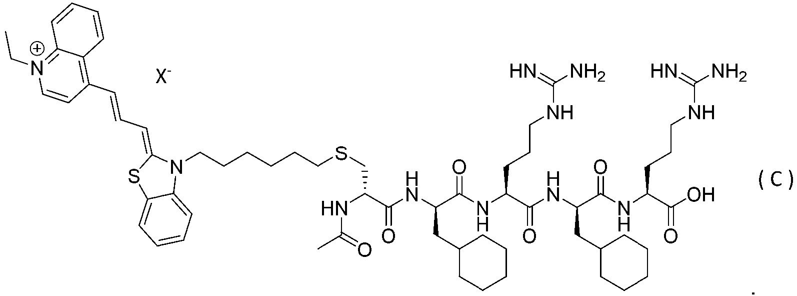

4. Colorant cyanine selon l'une des revendications 1 à 3 précédentes sous sa forme protonée ou déprotonée, dans lequel le ou les contre-ions de la forme protonée ou déprotonée sont choisis indépendamment dans la liste comprenant un halogénate, un sulfate, un triflate, un trifluoroacétate, un difluoroacétate ou un fluoroacétate.

5. Colorant cyanine selon l'une quelconque des revendications 1 à 4 précédentes, dans lequel le colorant est adapté pour se lier à l'ADN oligomérique ou à l'ADN amorce ou aux aptamères d'ADN.

6. Procédé de coloration de cellules vivantes, **caractérisé en ce qu'**il comprend les étapes suivantes :
i) au moins un colorant cyanine tel que défini dans les revendications 1 à 5 est appliqué à au moins un échantillon de cellules en ajoutant une solution dudit au moins un colorant cyanine audit au moins un échantillon de cellules, fournissant ainsi au moins un échantillon de cellules, coloré avec au moins un colorant cyanine ;
ii) l'incubation d'au moins un échantillon de cellules selon l'étape i) pendant au moins 15 minutes, de sorte que des cellules vivantes colorées soient présentes.

7. Procédé de coloration de cellules vivantes selon la revendication 6, **caractérisé en ce que** le procédé comprend les étapes suivantes i) et ii) et l'étape supplémentaire iii) :
i) au moins deux colorants cyanines sont appliqués à au moins deux échantillons distincts de cellules, chaque échantillon étant coloré avec l'un des au moins deux colorants cyanines en ajoutant une solution du colorant cyanine à l'échantillon de cellules, fournissant ainsi au moins deux échantillons distincts de cellules, chacun étant coloré avec au moins un colorant cyanine ;
ii) chaque échantillon de cellules selon l'étape i) modifiée est incubé pendant au moins 15 minutes, de sorte que des cellules vivantes colorées soient présentes ;
iii) les au moins deux échantillons distincts de cellules obtenus selon l'étape ii), chacun étant coloré avec au moins un colorant cyanine, sont combinés dans un récipient à échantillon, formant ainsi un échantillon combiné.

8. Procédé selon la revendication 6 ou la revendication 7, **caractérisé en ce que** le colorant cyanine est un colorant cyanine se liant à l'acide nucléique.

9. Procédé selon la revendication 6 ou la revendication 7 ou la revendication 8, **caractérisé en ce que** le procédé comprend l'étape de préparation supplémentaire suivante avant l'étape i) :
a) obtenir et isoler au moins un échantillon de cellules à introduire dans l'étape i), dans lequel cette étape d'obtention et d'isolement comprend une ou plusieurs étapes de concentration et/ou de culture des cellules.

10. Kit permettant de mettre en œuvre le procédé selon l'une quelconque des revendications 6 à 9, le kit comprenant le colorant cyanine et, éventuellement, des substances auxiliaires.

11. Procédé de synthèse d'un colorant cyanine selon l'une quelconque des revendications 1 à 5 précédentes, **caractérisé en ce que** le procédé de synthèse comprend les étapes suivantes :
a) réaliser une réaction de substitution sur un composé précurseur selon la formule (**IV**), 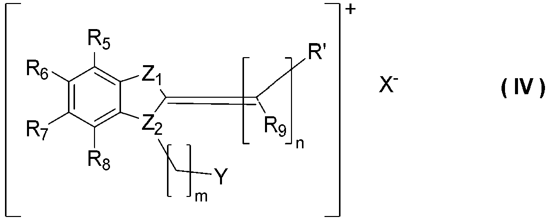 où
le substituant Y dans la formule (**IV**) est choisi dans la liste comprenant les substituants chlore, brome, iode, mésylate, tosylate, mésylate fluoré, tosylate fluoré et tous les autres substituants dans la formule (**IV**) ont la même signification et sont choisis de la même manière que celle indiquée dans la revendication 1 concernant la formule (**I**) et les indices m et n ont les mêmes valeurs que celles indiquées dans la revendication 1 concernant la formule (**I**),
en faisant réagir le composé précurseur selon la formule (**IV**) dans un mélange réactionnel avec un composé nucléophile, conduisant à la substitution du substituant Y par le composé nucléophile ;
b) extraire le produit de réaction du mélange réactionnel de l'étape a) et
c) purifier le produit de réaction récupéré à l'étape b).

12. Procédé selon la revendication 11, **caractérisé en ce que** le composé nucléophile dans l'étape a) est la triphénylphosphine ou le 1,2,3-triazole ou un peptide pénétrant dans les mitochondries.

13. Procédé selon la revendication 12, **caractérisé en ce que** le composé nucléophile dans l'étape a) est l'ion azide et le produit de réaction de l'étape a) ou le produit de réaction extrait de l'étape b) ou le produit de réaction purifié de l'étape c) est soumis à une réaction supplémentaire dans une étape supplémentaire d), l'étape supplémentaire d) étant insérée entre les étapes a) et b) ou entre les étapes b) et c) ou étant placée après l'étape c).

14. Procédé selon la revendication 13, **caractérisé en ce que** l'étape supplémentaire d) comprend la réaction d'un composé précurseur selon la formule (**V**), 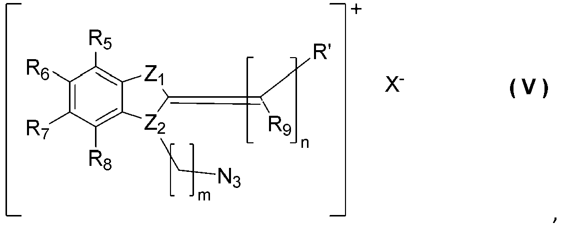 où, dans ce cas, R' est limité à un fragment selon la formule (**III**), avec de la tris((1-benzyl-4-triazolyl)méthyl)amine substituée ou non substituée, « TBTA », formant ainsi un colorant cyanine selon l'une quelconque des revendications 1 à 5 précédentes ayant une sous-structure où R' est un fragment selon la formule (**III**) et R₁ est un substituant avec une structure hétérocyclique substituée ou non substituée.

15. Procédé selon la revendication 13, **caractérisé en ce que** l'étape supplémentaire d) comprend la réaction d'un composé précurseur selon la formule (**V**), 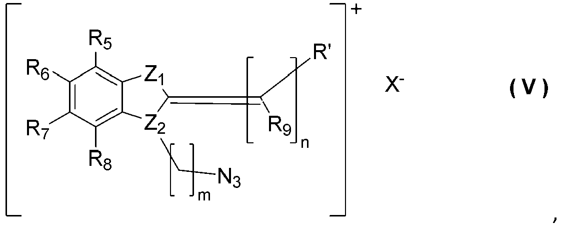 où R' est un fragment selon la formule (**II**) ou selon la formule (**III**), où n est 1 ou n est 2 ou n est 3, dans une réaction de cycloaddition azide-alcyne catalysée par le cuivre avec un alcyne substitué ou non substitué, formant ainsi un colorant cyanine selon l'une quelconque des revendications 1 à 5 précédentes, où R₁ est un substituant avec une structure hétérocyclique substituée ou non substituée.
